# EUROPEAN PATENT APPLICATION

(11) **EP 2 305 660 A1**
(43) Date of publication of application: **06.04.2011**
(21) Application number: 09382184.1
(22) Date of filing: 25.09.2009
(51) Int. Cl.: C07D 285/135, A61K 31/433, A61P 37/02

(54) **New thiadiazole derivatives**

(71) Applicant: Almirall, S.A., 08022 Barcelona (ES)
(72) Inventor: Grima Poveda, Pedro Manuel, 08028, Barcelona (ES); Aguilar Izquierdo, Nuria, 08005, Barcelona (ES); Mir Cepeda, Marta, 08024, Barcelona (ES); Carrascal Riera, Marta, 08028, Barcelona (ES); Terricabras Belart, Emma, 08173, St. Cugat del Vallés (ES); Gracia Ferrer, Jordi, 08720, Vilafranca del Penedés (ES); Casals Coll, Gaspar, 08358, Areny's de Munt (ES)
(74) Representative: Elzaburu Marquez, Alberto

(57) **Abstract**

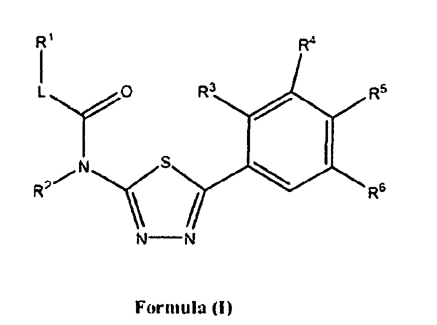

The present invention relates to thiadiazole derivatives of formula (I), to processes for their preparation and to pharmaceutical compositions containing them. These compounds are potent agonists of S1P1 receptors and thus, they are useful in the treatment, prevention or suppression of diseases and disorders known to be susceptible to improvement by sphingosine-1-phosphate receptors agonists (S1P1), such as autoimmune diseases, chronic immune and inflammatory diseases, transplant rejection, malignant neoplastic diseases, angiogenic-related disorders, pain, neurological diseases, viral and infectious diseases.

## Description

The present invention relates to thiadiazole derivatives, to processes for their preparation and to pharmaceutical compositions containing them. These compounds are potent agonists of S1P1 receptors and thus, they are useful in the treatment, prevention or suppression of diseases and disorders known to be susceptible to improvement by sphingosine-1-phosphate receptors agonists (S1P1), such as autoimmune diseases, chronic immune and inflammatory diseases, transplant rejection, malignant neoplastic diseases, angiogenic-related disorders, pain, neurological diseases, viral and infectious diseases.

Sphingosine -1 phosphate (S1P) is a pleiotropic lipid mediator that exhibits a broad spectrum of biological activities, including cell proliferation, survival, lymphocyte trafficking, cytoskeletal organization, and morphogenesis. S1P is generated from endogenous sphingosine through phosphorylation by specific kinases, named sphingosine kinases 1 and 2. The levels of S1P in biological fluids and tissues are tightly regulated by the balance between its synthesis by sphingosine kinases and its degradation by S1P lyase. This tight control is important since an excessive production of S1P has been associated to various pathological conditions, such as angiogenesis and vascular permeability changes in cancer, inflammation, myocardial infarction or transplant rejection.

Gene deletion studies and reverse pharmacology have provided evidence that most of the effects of S1P are mediated via five G-protein coupled receptor subtypes, named S1P1 to S1P5 (Brinkmann, Pharmacology & therapeutics 115:84-105, 2007). The interest on this family of receptors increased following the discovery that they were the pharmacological target of Fingolimod. This compound, a synthetic analog of a natural product derived from the fungus *Isaria sinclairii,* exhibited a peculiar immunomodulatory potential in vivo. When administered in vivo, it caused lymphopenia, due to the sequestration of lymphocytes from the blood into the lymph nodes and Peyer's patches. The close structural similarity of Fingolimod to sphingosine, together with the discovery of the formation of phosphorylated Fingolimod in vivo (FTY720P) prompted to speculate that FTY720-P could be acting as a mimetic of S1P. This proven to be the case and it was later on demonstrated that FTY-P binds 4 of the five known S1P receptors, namely S1P1, S1P3, S1P4 and S1P5.

Expression analysis identified S1P1 as the dominant S1P receptor expressed on lymphocytes. Moreover, the transfer of S1P1-deficient T cells to normal mice led to the cells being sequestered in lymph nodes, as occurred with animals treated with fingolimod. These two facts strongly pointed out at S1P1 as the main receptor involved in the lymphopenic effect of FTY-P in vivo (Baumruker et al, Exp. Opin. Invest. Drugs 2007; 16(3): 283-289). Fingolimod is currently in phase III trials for the treatment of relapsing-remitting multiple sclerosis. The drug is presumed to act by causing the retention of pathogenic lymphocytes in lymph nodes, thus preventing them to infiltrate the central nervous system (CNS).

In view of the physiological effects, several S1P1 agonists have been recently disclosed for the treatment or prevention of autoimmune diseases, such as multiple sclerosis (WO2008000419, WO2008021532), rheumatoid arthritis or Crohn's disease (WO2007091501), chronic immune and inflammatory diseases such as asthma, transplant rejection (WO199400943), cancer (WO2003097028), lymphoid malignancies (WO2007143081), angiogenic-related disorders, pain (WO2004110421, WO2007089715) neurological diseases such as neurodegeneration (WO2005025553) or dementia (WO2005058295), cardiovascular diseases (WO2004010987),.

Autoimmune diseases include but are not limited to rheumatoid arthritis, multiple sclerosis, inflammatory bowel diseases such as Crohn's diseases and ulcerative colitis, psoriatic arthritis, thyroiditis such as Hashimoto's thyroiditis, type I diabetes; systemic lupus erythematosis and Sjögrn's syndrome.

Rejection transplanted organs such as kidney, liver, heart, lung, pancreas, cornea and skin; graft-versus-hist disease brought about by stem cell transplantation.

Immune and inflammatory diseases which may be prevented or treated include but are not limited to asthma, COPD, respiratory distress syndrome, acute or chronic pancreatitis and hepatitis; chronic sarcoidosis, contact dermatitis, atopic dermatitis, allergic rhinitis, allergic conjunctivitis, Behcet syndrome, inflammatory eye conditions such as conjunctivitis and uveitis.

Malignant neoplastic diseases that may be prevented or treated include but are not limited to solid cancer, tumor metastasis and lymphoid malignancies Angiogenesis-related disorders that may be prevented or treated include but are not limited to hemangiomas, ocular neovascularization, macular degeneration or diabetic retinopathy.

Pain including neuropathic pain, that may be prevented or treated include but are not limited to prophylaxis or treatment of chronic pain, wherein chronic pain is selected from chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e. g. after amputation,trigeminal neuralgia, migraine or post herpetic neuralgia.

Cardiovascular diseases which may be prevented or treated include but are not limited to chronic heart failure, congestive heart failure, arrhythmia or tachyarrythmia, unstable angina, acute myocardial infarction or complications from cardiac surgery or for improving heart energy efficiency or cardiac output.

Neurological diseases including neurodegeneration, dementia or brain degeneration that may be prevented or treated include but are not limited to neurological disorders including Parkinson's desease, Parkinsonian disorders, Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy, Lewy body disease, stroke, cerebral infarction, multi-infarct dementia, and geriatric dementia,

Viral diseases which may be prevented or treated include but are not limited to HIV infection, hepatitis C and cytomegalovirus infection.

Infectious diseases which may be prevented or treated include but are not limited to pathogenic fungal diseases.

It has now been found that certain thiadiazoles are novel and potent agonists of S1P1 and can therefore be used in the treatment or prevention of these diseases.

Thus the present invention is directed to new thiadiazole derivatives of formula (I) or a pharmaceutically acceptable salt or N-oxide or stereoisomer thereof 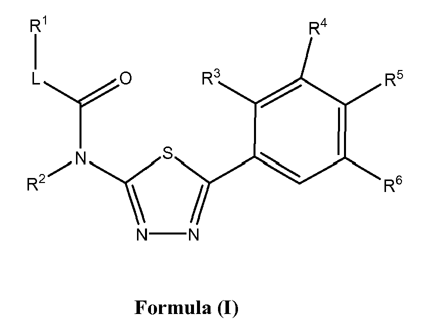 wherein:
- L represents a direct bond or a -NH- group,
- R¹ represents a phenyl, a pyridyl or a benzyl group which is optionally substituted with one or more substituents selected from an halogen atom, a C₁₋₄ alkyl group, a C₁-₄ alkoxy group, a C₃-₇ cycloalkyl group and -CN, -CONH₂ and -CF₃ groups,
- R² represents a C₁₋₆ alkyl or a C₁₋₄ alkyl-C₃₋₇ cycloalkyl group,
- R³, R⁴ and R⁶ independently represent a hydrogen atom, a halogen atom, a C₁-₄ alkyl group or a -CF₃ group,
- R⁵ represents -O-(CH₂)(₀₋₂)-R^{a}, -O-(CH₂)(₂₋₄)-NR'R", -O-(CH₂)₍₂₋₄₎-NH-(CH₂)₍₁₋₄₎-R", -(CH₂)₍₁₋₂₎-NR'R", -(CH₂)₍₁₋₄₎-NH-(CH₂)₍₁₋₄₎-R" or -C(O)-NR'R", wherein
   ○ R^{a} represents a 4 to 6-membered N-containing saturated heterocyclic group optionally substituted with one or more substituents selected from a hydroxycarbonyl group and a C₁₋₄ alkyl group which is substituted with a hydroxycarbonyl group, or a C₃₋₇ cycloalkyl group substituted with a di-(C₁₋₄ alkyl)amino group which is itself optionally substituted with a hydroxycarbonyl group,
   ○ R' represents a hydrogen atom or a C₁₋₄ alkyl group,
   ○ R" represents a methylsulfonamido group, C₂₋₄ alkanoyl group, which is optionally substituted with a hydroxy group; a C₁₋₄ alkyl group substituted with one or more substituents selected from a hydroxy group, and a hydroxycarbonyl group; a 5- to 6-membered saturated or unsaturated heterocyclic group containing one or more heteroatoms selected from nitrogen and oxygen atoms and which is optionally substituted with a hydroxy group; or a C₄₋₆ cycloalkyl group substituted with a hydroxycarbonyl group, or
   o R' and R" together with the nitrogen atom to which they are attached form a 4 to 6 membered, saturated or unsaturated heterocyclic group which contains, as heteroatoms, the N atom to which R' and R" are attached and 0, 1 or 2 further N atoms, which heterocyclic group is optionally substituted with one or more substituents selected from a cyano, a carbamoyl, a hydroxycarbonyl, a C₁₋₃ alkoxycarbonyl and a 1*H*-tetrazolyl groups and a C₁₋₃ alkyl group which is optionally substituted by a hydroxycarbonyl group.

Further objectives of the present invention are to provide a method for preparing said compounds; pharmaceutical compositions comprising an effective amount of said compounds; a compound of the invention for use in the treatment of the human or animal body by therapy; the use of the compounds in the manufacture of a medicament for the treatment of pathological conditions or diseases susceptible to improvement by sphingosine-1-phosphate receptors agonists (S1P1), wherein the pathological condition or disease is preferably selected from autoimmune diseases, chronic immune and inflammatory diseases, transplant rejection, malignant neoplastic diseases, angiogenic-related disorders, pain, neurological diseases, viral and infectious diseases, and methods of treatment of pathological conditions or diseases susceptible to amelioration by sphingosine-1-phosphate receptors agonists (S1P1), wherein the pathological condition or disease is preferably selected from autoimmune diseases, chronic immune and inflammatory diseases, transplant rejection, malignant neoplastic diseases, angiogenic-related disorders, pain, neurological diseases,viral and infectious diseases comprising the administration of the compounds of the invention to a subject in need of treatment.

As used herein the term alkyl group embraces optionally substituted, linear or branched hydrocarbon radicals having 1 to 6, preferably 1 to 4 carbon atoms. The alkyl group may be unsubstituted or substituted with one or more, for example 1, 2 or 3, halogen atoms, preferably fluorine or chlorine atoms, hydroxyl or hydroxycarbonyl groups. When an alkyl radical carries 2 or more substituents, the substituents may be the same or different. Hydroxyl and hydroxycarbonyl groups are preferred substituents. Unless otherwise specified, said alkyl group is preferably unsubstituted. Examples include methyl, ethyl, *n*-propyl, *i*-propyl, *n*-butyl, *sec*-butyl and *tert*-butyl radicals.

As used herein the term alkoxy group embraces optionally substituted, linear or branched oxy-containing radicals each having 1 to 4 carbon atoms. The alkoxy groups may be unsubstituted or substituted with one or more, for example 1, 2 or 3, halogen atoms, preferably fluorine or chlorine atoms, or hydroxyl groups. When an alkoxy radical carries 2 or more substituents, the substituents may be the same or different preferably however said alkoxy group is unsubstituted. Examples include methoxy, ethoxy, *n*-propoxy, *i*-propoxy, *n*-butoxy, *sec*-butoxy and *tert*-butoxy radicals.

As used herein, the term cycloalkyl group embraces saturated carbocyclic radicals having 3 to 7 carbon atoms, preferably from 4 to 6 and more preferably from 3 to 4 carbon atoms. Examples include cyclopropyl, cyclobutyl, cyclopentyl and cyclohexyl.

The cycloalkyl radical may be unsubstituted or substituted with one or more, for example 1 or 2, hydroxyl, hydroxycarbonyl or di(C₁₋₄alkyl)amino groups. When a cycloalkyl radical carries 2 or more substituents, the substituents may be the same or different. Unless otherwise specified, a cycloalkyl radical is preferably unsubstituted.

When R² represents a C₁₋₄ alkyl-C₃₋₇ cycloalkyl group, it is to be understood that the C₁-₄ alkyl portion is preferably bonded to the nitrogen atom of the molecule as depicted in formula (I).

As used herein, the term C₁₋₃ alkoxycarbonyl group embraces radicals of formula -C(O)O(C₁₋₃ alkyl) wherein said C₁₋₃ alkyl is a linear or branched hydrocarbon radical having 1 to 4 carbon atoms. The C₁₋₃ alkoxycarbonyl groups are unsubstituted or substituted with 1, 2 or 3, preferably 1 or 2, halogen atoms, preferably fluorine or chlorine atoms, or hydroxyl groups. Said substituents may be the same or different. Preferably alkoxycarbonyl groups are unsubstituted.

Preferred alkoxycarbonyl radicals include methoxycarbonyl, ethoxycarbonyl, n-propoxycarbonyl and i-propoxycarbonyl.

As used herein, the term C₂₋₄ alkanoyl group embraces optionally substituted, linear or branched radicals having alkyl portion of 1 to 3 carbon atoms and attached to a carbonyl radical. An alkanoyl group is typically unsubstituted or substituted with 1, 2 or 3, preferably 1 or 2, halogen atoms, preferably fluorine or chlorine atoms or hydroxyl groups. Said substituents may be the same or different. Typically an alkanoyl group is unsubstituted or substituted with one hydroxy group.

As used herein, the term di-(C₁₋₄ alkyl)amino group embraces radicals containing a trivalent nitrogen atom with two C₁₋₄ alkyl radicals attached thereto. A dialkylamino group is typically unsubstituted or substituted on one or each alkyl moiety with a hydroxycarbonyl group. Preferably a dialkylamino group is unsubstituted.

The 5- to 6-membered saturated or unsaturated heterocyclic group which R" may represent is a saturated or unsaturated non-aromatic C₅-C₆ carbocyclic ring system in which one or more, for example 1, 2, 3 or 4, preferably 1 or 2, of the carbon atoms are replaced by heteroatoms selected from N and O.

A said 5- to 6-membered saturated or unsaturated heterocyclic group is typically unsubstituted or substituted with a hydroxyl group. Preferably, said 5- to 6-membered saturated or unsaturated heterocyclic group is unsubstituted.

Examples of 5- to 6-membered saturated or unsaturated heterocyclic groups include piperidyl, pyrrolidyl, pyrrolinyl, piperazinyl, morpholinyl, pyrrolyl, pyrazolinyl, pirazolidinyl, triazolyl, pyrazolyl, tetrazolyl, imidazolidinyl and imidazolyl. Triazolyl and tetrazolyl are preferred.

The 4 to 6-membered N-containing saturated heterocyclic group which R^{a} may represent is a saturated C₄-C₆ carbocyclic ring system in which one of the carbon atoms is replaced by N and optionally in which 1, 2 or 3 further carbon atoms are replaced by heteroatoms selected from N, O and S.

A said 4 to 6-membered N-containing saturated heterocyclic group is typically unsubstituted or substituted with 1, 2 or 3 substituents which may be the same or different. Typically, the substituents on a 4 to 6-membered N-containing saturated heterocyclic group are themselves unsubstituted, unless otherwise specified.

Examples of 4 to 6-membered N-containing saturated heterocyclic groups include azetidyl, piperidyl and pyrrolidyl.

The 4 to 6-membered saturated or unsaturated heterocyclic group which R' and R" may form together with the nitrogen to which they are attached is a saturated or unsaturated, non-aromatic C₄-C₆ carbocyclic ring system in which one of the carbon atoms is replaced by N and optionally in which 1 or 2 further carbon atoms are replaced by further N atoms. Thus, said 4 to 6-membered saturated or unsaturated heterocyclic group contains, as heteroatoms, the N atom to which R' and R" are attached and 0, 1 or 2 further N atoms.

A said 4 to 6-membered saturated or unsaturated heterocyclic group is typically unsubstituted or substituted with 1, 2 or 3 substituents which may be the same or different. Typically, the substituents on a 4 to 6-membered saturated or unsaturated heterocyclic group are themselves unsubstituted, unless otherwise specified.

Examples of 4 to 6-membered saturated or unsaturated heterocyclic groups include azetidyl, piperidyl, pyrrolidyl, pyrrolinyl, piperazinyl, pyrrolyl, pyrazolinyl, pirazolidinyl, triazolyl, pyrazolyl, imidazolidinyl and imidazolyl. Preferred examples of 4 to 6-membered N-containing saturated or unsaturated heterocyclic groups are azetidyl, piperidyl, pyrrolidyl and pyrazolyl.

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms, typically fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

Typically, L represents a direct bond.

Typically, R¹ represents a phenyl or a benzyl group which is optionally substituted with one or two substituents selected from halogen atoms, a C₁₋₄ alkyl group and a C₁₋₄ alkoxy group. Preferably, the substituents are selected from a fluorine atom, a chlorine atom, a methyl group and a methoxy group.

More preferably R¹ represents a phenyl group substituted with a fluorine atom, a chlorine atom or a methyl group.

Typically R² represents an ethyl, butyl or cyclopropylmethyl group, preferably a butyl or cyclopropylmethyl group.

Typically R³, R⁴ and R⁶ independently represent a hydrogen atom, a methyl group or a -CF₃ group, preferably R³ represents a hydrogen atom.

More preferably, R⁴ represents a methyl group or a -CF₃ group, and R⁶ represents a hydrogen atom or a methyl group.

Typically, R⁵ represents -O-(CH₂)₍₀₋₁₎-R^{a}, -O-(CH₂)₍₂₋₄₎-NR'R", -O-(CH₂)₍₂₋₄₎-NH-(CH₂)₍₁₋₄₎-R", -(CH₂)₍₁₋₂₎-NR'R" or -(CH₂)₍₁₋₄₎-NH-(CH₂)₍₁₋₄₎-R", wherein
○ R^{a} represents a 5 to 6-membered N-containing saturated heterocyclic group optionally substituted with a hydroxycarbonyl group,
o R' represents a hydrogen atom or a methyl group,
o R" represents a methylsulfonamido group, a C₁₋₃ alkyl group substituted with a hydroxycarbonyl group; an unsubstituted 5- to 6 membered unsaturated heterocyclic group containing one or more nitrogen atoms as heteroatoms; or a C₄₋₆ cycloalkyl group substituted with a hydroxycarbonyl group, or
o R' and R" together with the nitrogen atom to which they are attached form a 4 to 6 membered, saturated heterocyclic group substituted with one or two substituents selected from a cyano group, a carbamoyl group, a hydroxycarbonyl group, a C₁₋₂ alkoxycarbonyl group and a 1*H*-tetrazolyl group and a C₁₋₃ alkyl group which is optionally substituted with a hydroxycarbonyl group.

Preferably, R⁵ represents -O-(CH₂)₍₂₋₄₎-NR'R", -O-(CH₂)₍₂₋₄₎-NH-(CH₂)₍₁₋₄₎-R", -(CH₂)₍₁₋₂₎-NR'R" or -(CH₂)₍₁₋₄₎-NH-(CH₂)₍₁₋₄₎-R", wherein
○ R" represents a C₁₋₃ alkyl group or a C₅₋₆ cycloalkyl group wherein both groups are substituted with a hydroxycarbonyl group, or
○ R' and R" together with the nitrogen atom to which they are attached form a 4 to 6 membered, saturated heterocyclic group substituted with one or two substituents selected from the group consisting of a carbamoyl group and hydroxycarbonyl group, and a methyl group which is optionally substituted by a hydroxycarbonyl group.

More preferably, R⁵ represents a -O-(CH₂)₍₂₋₃₎-NR'R", wherein R' and R" together with the nitrogen atom to which they are attached form a 4 to 6 membered, saturated heterocyclic ring substituted with one or two substituents selected from hydroxycarbonyl group and a methyl group which is optionally substituted with hydroxycarbonyl group.

In a preferred embodiment, L represents a direct bond, R¹ represents a phenyl group substituted with a fluorine atom, a chlorine atom or a methyl group, R² represents a butyl or cyclopropylmethyl group, R³ represents a hydrogen atom, R⁴ represents a methyl or a -CF₃ group, R⁶ represents a hydrogen atom or a methyl group and R⁵ represents -O-(CH₂)₍₂₋₃₎-NR'R", wherein R' and R" together with the nitrogen atom to which they are attached form a 4 to 6 membered, saturated heterocyclic group substituted with one or two substituents selected from a hydroxycarbonyl group and a methyl group which is optionally substituted with hydroxycarbonyl group.

In another preferred embodiment, L represents a direct bond or -NH-; R¹ represents a phenyl group substituted with one or two substituents independently selected from a fluorine atom, a chlorine atom, a methyl group or a methoxy group, a benzyl group, or pyridyl group substituted with a chlorine atom; R² represents ethyl, butyl or cyclopropylmethyl group; R³, R⁴ and R⁶ independently represent hydrogen atoms or methyl groups; R⁵ represents -O-(CH₂)₍₀₋₁₎-Ra, -O-(CH₂)₂-NR'R", -O-(CH₂)₂-NH-(CH₂)₂-R", -(CH₂)₂-NR'R", -(CH₂)₍₁₋₂₎-NH-(CH₂)₂-R" or -C(O)-NR'R", wherein: R^{a} piperidyl or pyrolidyl which is unsubstituted or substituted with a hydroxycarbonyl group; R' represents a hydrogen atom or methyl; R" represents a methylsulfonamido group, an ethanoyl or hydroxyethanoyl group; a methyl, ethyl or propyl group which is substituted with one or two substitutents independently selected from a hydroxy group and a hydroxycarbonyl group; a tetrazolyl or triazolyl group; or a cyclopentyl or cyclohexyl group substituted with a hydroxycarbonyl group; or R' and R" together with the nitrogen atom to which they are attached form an azetidyl, pyrrolidyl, pyrazolyl or piperidyl group which is substituted with one or two substituents selected from a cyano, a carbamoyl, a hydroxycarbonyl, a ethoxycarbonyl and 1*H*-tetrazolyl group and a methyl group which is optionally substituted with one hydroxycarbonyl group.

Particular individual compounds of the invention include:
1-[2-(4-{5-[butyl(2-fluorobenzoyl)amino]-1,3,4-thiadiazol-2-yl}-2,6-dimethylphenoxy)ethyl]azetidine-3-carboxylic acid,
N-[2-(4-{5-[butyl(2-fluorobenzoyl)amino]-1,3,4-thiadiazol-2-yl}-2,6-dimethylphenoxy)ethyl]-beta-alanine,
N-[2-(4-{5-[butyl(2-fluorobenzoyl)amino]-1,3,4-thiadiazol-2-yl}-2,6-dimethylphenoxy)ethyl]glycine,
N-[2-(4-{5-[butyl(2-fluorobenzoyl)amino]-1,3,4-thiadiazol-2-yl}-2,6-dimethylphenoxy)ethyl]-N-methylglycine,
1-[2-(4-{5-[butyl(2-fluorobenzoyl)amino]-1,3,4-thiadiazol-2-yl}-2,6-dimethylphenoxy)ethyl]piperidine-4-carboxylic acid,
1-[2-(4-{5-[butyl(2-fluorobenzoyl)amino]-1,3,4-thiadiazol-2-yl}-2,6-dimethylphenoxy)ethyl]piperidine-2-carboxylic acid,
(4S)-4-(4-{5-[butyl(2-fluorobenzoyl)amino]-1,3,4-thiadiazol-2-yl}-2,6-dimethylphenoxy)-L-proline,
1-[2-(4-{5-[butyl(2-fluorobenzoyl)amino]-1,3,4-thiadiazol-2-yl}-2,6-dimethylphenoxy)ethyl]piperidine-3-carboxylic acid,
1-[2-(4-{5-[butyl(2-fluorobenzoyl)amino]-1,3,4-thiadiazol-2-yl}-2,6-dimethylphenoxy)ethyl]praline,
N-butyl-2-fluoro-N-(5-{4-[2-(glycoloylamino)ethoxy]-3,5-dimethylphenyl}-1,3,4-thiadiazol-2-yl)benzamide,
N-[2-(4-{5-[butyl(2-methylbenzoyl)amino]-1,3,4-thiadiazol-2-yl}-2,6-dimethylphenoxy)ethyl]-N-methylglycine,
N-[2-(4-{5-[butyl(2-chlorobenzoyl)amino]-1,3,4-thiadiazol-2-yl}-2,6-dimethylphenoxy)ethyl]-N-methylglycine,
1-[2-(4-{5-[butyl(2-methylbenzoyl)amino]-1,3,4-thiadiazol-2-yl}-2,6-dimethylphenoxy)ethyl]praline,
1-[2-(4-{5-[butyl(2-chlorobenzoyl)amino]-1,3,4-thiadiazol-2-yl}-2,6-dimethylphenoxy)ethyl]praline,
1-[2-(4-{5-[butyl(2-fluorobenzoyl)amino]-1,3,4-thiadiazol-2-yl}-2,6-dimethylphenoxy)ethyl]pyrrolidine-3-carboxylic acid,
1-[2-(4-{5-[butyl(2-chlorobenzoyl)amino]-1,3,4-thiadiazol-2-yl}-2,6-dimethylphenoxy)ethyl]piperidine-4-carboxylic acid,
1-[2-(4-{5-[butyl(2-methylbenzoyl)amino]-1,3,4-thiadiazol-2-yl}-2,6-dimethylphenoxy)ethyl]piperidine-4-carboxylic acid,
1-[2-(4-{5-[butyl(3-chloro-2-fluorobenzoyl)amino]-1,3,4-thiadiazol-2-yl}-2,6-dimethylphenoxy)ethyl]piperidine-4-carboxylic acid,
1-[2-(4-{5-[ethyl(2-fluorobenzoyl)amino]-1,3,4-thiadiazol-2-yl}-2,6-dimethylphenoxy)ethyl]piperidine-4-carboxylic acid,
(1S,2R)-2-{[2-(4-{5-[butyl(2-fluorobenzoyl)amino]-1,3,4-thiadiazol-2-yl}-2,6-dimethylphenoxy)ethyl]amino}cyclopentanecarboxylic acid,
1-[2-(4-{5-[(2-chlorobenzoyl)(ethyl)amino]-1,3,4-thiadiazol-2-yl}-2,6-dimethylphenoxy)ethyl]piperidine-4-carboxylic acid,
{1-[2-(4-{5-[butyl(2-fluorobenzoyl)amino]-1,3,4-thiadiazol-2-yl}-2,6-dimethylphenoxy)ethyl]piperidin-4-yl}acetic acid,
N-butyl-N-{5-[3,5-dimethyl-4-(2-{[2-(2H-tetrazol-5-yl)ethyl]amin0}ethoxy)phenyl]-1,3,4-thiadiazol-2-yl}-2-fluorobenzamide,
1-[2-(4-{5-[(3-chloro-2-fluorobenzoyl)(ethyl)amino]-1,3,4-thiadiazol-2-yl}-2,6-dimethylphenoxy)ethyl]piperidine-4-carboxylic acid,
N-[2-(4-{5-[ethyl(2-fluorobenzoyl)amino]-1,3,4-thiadiazol-2-yl}-2,6-dimethylphenoxy)ethyl]-N-methylglycine,
1-[2-(4-{5-[ethyl(2-fluorobenzoyl)amino]-1,3,4-thiadiazol-2-yl}-2,6-dimethylphenoxy)ethyl]piperidine-3-carboxylic acid,
1-[2-(4-{5-[butyl(2-fluorobenzoyl)amino]-1,3,4-thiadiazol-2-yl}-2,6-dimethylphenoxy)ethyl]-4-methylpiperidine-4-carboxylic acid,
cis-4-{[2-(4-{5-[butyl(2-fluorobenzoyl)amino]-1,3,4-thiadiazol-2-yl}-2,6-dimethylphenoxy)ethyl]amino}cyclohexanecarboxylic acid,
1-[2-(4-{5-[butyl(2,4-difluorobenzoyl)amino]-1,3,4-thiadiazol-2-yl}-2,6-dimethylphenoxy)ethyl]piperidine-4-carboxylic acid,
1-[2-(4-{5-[butyl(2-fluorobenzoyl)amino]-1,3,4-thiadiazol-2-yl}phenoxy)ethyl]-azetidine-3-carboxylic acid,
1-[2-(4-{5-[butyl(2-fluorobenzoyl)amino]-1,3,4-thiadiazol-2-yl}phenoxy)ethyl]-piperidine-4-carboxylic acid,
1-[2-(4-f5-[ethyl(2-fluorobenzoyl)amino]-1,3,4-thiadiazol-2-yll-2,6-dimethylphenoxy)ethyl]azetidine-3-carboxylic acid,
1-[2-(4-{5-[butyl(2-methoxybenzoyl)amino]-1,3,4-thiadiazol-2-yl}-2,6-dimethylphenoxy)ethyl]piperidine-4-carboxylic acid,
1-[2-(4-{5-[butyl(phenylacetyl)amino]-1,3,4-thiadiazol-2-yl}-2,6-dimethylphenoxy)ethyl]piperidine-4-carboxylic acid,
1-[2-(4-{5-[butyl(2,3-dimethylbenzoyl)amino]-1,3,4-thiadiazol-2-yl}-2,6-dimethylphenoxy)ethyl]piperidine-4-carboxylic acid,
1-[2-(4-{5-[butyl(2-chloro-3-fluorobenzoyl)amino]-1,3,4-thiadiazol-2-yl}-2,6-dimethylphenoxy)ethyl]piperidine-4-carboxylic acid,
1-[2-(4-{5-[(2-chlorobenzoyl)(cyclopropylmethyl)amino]-1,3,4-thiadiazol-2-yl}-2,6-dimethylphenoxy)ethyl]piperidine-4-carboxylic acid,
N-[2-(4-{5-[(2-chlorobenzoyl)(cyclopropylmethyl)amino]-1,3,4-thiadiazol-2-yl}-2,6-dimethylphenoxy)ethyl]-N-methylglycine,
{1-[2-(4-{5-[(2-chlorobenzoyl)(cyclopropylmethyl)amino]-1,3,4-thiadiazol-2-yl}-2,6-dimethylphenoxy)ethyl]piperidin-4-yl}acetic acid,
N-butyl-2-chloro-N-{5-[3,5-dimethyl-4-(piperidin-4-ylmethoxy)phenyl]-1,3,4-thiadiazol-2-yl}benzamide,
1-[2-(4-{5-[butyl(2-chlorobenzoyl)amino]-1,3,4-thiadiazol-2-yl}-2,6-dimethylphenoxy)ethyl]pyrrolidine-3-carboxylic acid,
{1-[2-(4-{5-[butyl(2-chlorobenzoyl)amino]-1,3,4-thiadiazol-2-yl}-2,6-dimethylphenoxy)ethyl]piperidin-4-yl}acetic acid,
1-[2-(4-{5-[butyl(2-chlorobenzoyl)amino]-1,3,4-thiadiazol-2-yl}-2,6-dimethylphenoxy)ethyl]-4-methylpiperidine-4-carboxylic acid,
(1S,2R)-2-{[2-(4-{5-[butyl(2-chlorobenzoyl)amino]-1,3,4-thiadiazol-2-yl}-2,6-dimethylphenoxy)ethyl]amin0}cyclopentanecarboxylic acid,
cis-4-f[2-(4-f5-[butyl(2-chlorobenzoyl)amino]-1,3,4-thiadiazol-2-yll-2,6-dimethylphenoxy)ethyl]amino}cyclohexanecarboxylic acid,
1-[2-(4-{5-[(2-chlorobenzoyl)(cyclopropylmethyl)amino]-1,3,4-thiadiazol-2-yl}-2,6-dimethylphenoxy)ethyl]azetidine-3-carboxylic acid,
{1-[2-(4-{5-[butyl(2-chlorobenzoyl)amino]-1,3,4-thiadiazol-2-yl}-2,6-dimethylphenoxy)ethyl]pyrrolidin-3-yl}acetic acid,
N-[2-(4-{5-[butyl(2-chlorobenzoyl)amino]-1,3,4-thiadiazol-2-yl}-2,6-dimethylphenoxy)ethyl]-beta-alanine,
{1-[2-(4-{5-[butyl(2-chlorobenzoyl)amino]-1,3,4-thiadiazol-2-yl}-2,6-dimethylphenoxy)ethyl]piperidin-3-yl}acetic acid,
1-[2-(4-{5-[butyl(2-chlorobenzoyl)amino]-1,3,4-thiadiazol-2-yl}-2,6-dimethylphenoxy)ethyl]piperidine-2-carboxylic acid,
N-[2-(4-{5-[butyl(2-chlorobenzoyl)amino]-1,3,4-thiadiazol-2-yl}-2,6-dimethylphenoxy)ethyl]glycine,
1-[2-(4-{5-[butyl(2-chlorobenzoyl)amino]-1,3,4-thiadiazol-2-yl}-2,6-dimethylphenoxy)ethyl]piperidine-3-carboxylic acid,
1-{4-[5-(butyl{[(4-fluorophenyl)amino]carbonyl}amino)-1,3,4-thiadiazol-2-yl]benzyllazetidine-3-carboxylic acid,
1-(4-{5-[anilinocarbonyl)(butyl)amino]-1,3,4-thiadiazol-2-yl}benzyl)azetidine-3-carboxylic acid,
1-{4-[5-(butyl{[(2-chloropyridin-3-yl)amino]carbonyl}amino)-1,3,4-thiadiazol-2-yl]benzyl}azetidine-3-carboxylic acid,
1-(4-{5-[butyl(2-fluorobenzoyl)amino]-1,3,4-thiadiazol-2-yl}benzoyl)azetidine-3-carboxylic acid,
N-butyl-2-fluoro-N-[5-(4-{[(2-hydroxyethyl)amino]methyl}phenyl)-1,3,4-thiadiazol-2-yl]benzamide,
3-[(4-{5-[butyl(2-fluorobenzoyl)amino]-1,3,4-thiadiazol-2-yl}benzoyl)amino]-2-hydroxypropanoic acid,
1-(4-{5-[butyl(2-fluorobenzoyl)amino]-1,3,4-thiadiazol-2-yl}benzyl)azetidine-3-carboxamide,
3-[(4-f5-[butyl(phenylacetyl)amino]-1,3,4-thiadiazol-2-yllbenzyI)amino]-2-methylpropanoic acid,
(1S,2S)-2-[(4-{5-[butyl(phenylacetyl)amino]-1,3,4-thiadiazol-2-yl}benzyl)-amino]cyclohexanecarboxylic acid,
1-[4-(5-{butyl[(2-methoxyphenyl)acetyl]amino}-1,3,4-thiadiazol-2-yl)benzyl]-azetidine-3-carboxylic acid,
N-butyl-2-fluoro-N-(5-{4-[({2-[(methylsulfonyl)amino]ethyl}amino)methyl]phenyl}-1,3,4-thiadiazol-2-yl)benzamide,
N-(4-{5-[butyl(2-fluorobenzoyl)amino]-1,3,4-thiadiazol-2-yl}benzyl)-N-methylglycine, N-butyl-N-(5-{4-[(3-cyanoazetidin-1-yl)methyl]phenyl}-1,3,4-thiadiazol-2-yl)-2-fluorobenzamide,
1-[4-(5-{butyl[(2-methoxyphenyl)acetyl]amino}-1,3,4-thiadiazol-2-yl)-3-methylbenzyl]azetidine-3-carboxylic acid,
N-butyl-2-fluoro-N-[5-(4-{2-[(2-hydroxyethyl)(methyl)amlno]ethoxy}-3,5-dimethylphenyl)-1,3,4-thiadiazol-2-yl]benzamide,
N-(5-{4-[2-(acetylamino)ethoxy]-3,5-dimethylphenyl}-1,3,4-thiadiazol-2-yl)-N-butyl-2-fluorobenzamide,
1-(4-{5-[butyl(2-fluorobenzoyl)amino]-1,3,4-thiadiazol-2-yl}benzyl)-1H-pyrazole-4-carboxylic acid,
1-(4-{5-[butyl(2-fluorobenzoyl)amino]-1,3,4-thiadiazol-2-yl}benzyl)-4-(ethoxycarbonyl)piperidine-4-carboxylic acid,
N-(4-{5-[butyl(2-fluorobenzoyl)amino]-1,3,4-thiadiazol-2-yl}benzyl)-N-methyl-beta-alanine,
N-butyl-2-fluoro-N-[5-(4-{[3-(1H-tetrazol-5-yl)azetidin-1-yl]methyl}phenyl)-1,3,4-thiadiazol-2-yl]benzamide,
N-butyl-N-{5-[3,5-dimethyl-4-(2-{[2-(1H-1,2,4-triazol-1-yl)ethyl]amino}ethoxy)-phenyl]-1,3,4-thiadiazol-2-yl}-2-fluorobenzamide,
N-butyl-2-fluoro-N-{5-[4-({[2-(1H-1,2,4-triazol-1-yl)ethyl]amino}methyl)phenyl]-1,3,4-thiadiazol-2-yl}benzamide,
N-(4-{5-[butyl(2-chlorobenzoyl)amino]-1,3,4-thiadiazol-2-yl}-2,6-dimethylbenzyl)-N-methylglycine,
3-[(4-{5-[butyl(phenylacetyl)amino]-1,3,4-thiadiazol-2-yl}benzyl)amino]-3-methylpropanoic acid,

Of outstanding interest are:
N-[2-(4-{5-[butyl(2-fluorobenzoyl)amino]-1,3,4-thiadiazol-2-yl}-2,6-dimethylphenoxy)ethyl]glycine,
N-[2-(4-{5-[butyl(2-fluorobenzoyl)amino]-1,3,4-thiadiazol-2-yl}-2,6-dimethylphenoxy)ethyl]-N-methylglycine,
1-[2-(4-{5-[butyl(2-fluorobenzoyl)amino]-1,3,4-thiadiazol-2-yl}-2,6-dimethylphenoxy)ethyl]praline,
N-[2-(4-{5-[butyl(2-methylbenzoyl)amino]-1,3,4-thiadiazol-2-yl}-2,6-dimethylphenoxy)ethyl]-N-methylglycine,
N-[2-(4-{5-[butyl(2-chlorobenzoyl)amino]-1,3,4-thiadiazol-2-yl}-2,6-dimethylphenoxy)ethyl]-N-methylglycine,
N-[2-(4-{5-[ethyl(2-fluorobenzoyl)amino]-1,3,4-thiadiazol-2-yl}-2,6-dimethylphenoxy)ethyl]-N-methylglycine,
cis-4-{[2-(4-{5-[butyl(2-fluorobenzoyl)amino]-1,3,4-thiadiazol-2-yl}-2,6-dimethylphenoxy)ethyl]amino}cyclohexanecarboxylic acid,
1-[2-(4-{5-[butyl(2-fluorobenzoyl)amino]-1,3,4-thiadiazol-2-yl}phenoxy)ethyl]-azetidine-3-carboxylic acid,
N-[2-(4-{5-[butyl(2-chlorobenzoyl)amino]-1,3,4-thiadiazol-2-yl}-2,6-dimethylphenoxy)ethyl]-beta-alanine,
1-[4-(5-{butyl[(2-methoxyphenyl)acetyl]amino}-1,3,4-thiadiazol-2-yl)-3-methylbenzyl]azetidine-3-carboxylic acid,
N-butyl-2-fluoro-N-[5-(4-{[3-(1H-tetrazol-5-yl)azetidin-1-yl]methyl}phenyl)-1,3,4-thiadiazol-2-yl]benzamide,
N-butyl-2-fluoro-N-{5-[4-({[2-(1H-1,2,4-triazol-1-yl)ethyl]amino}methyl)phenyl]-1,3,4-thiadiazol-2-yl}benzamide,

Compounds of general formula (I) may be prepared following the synthetic scheme depicted in figure 1.

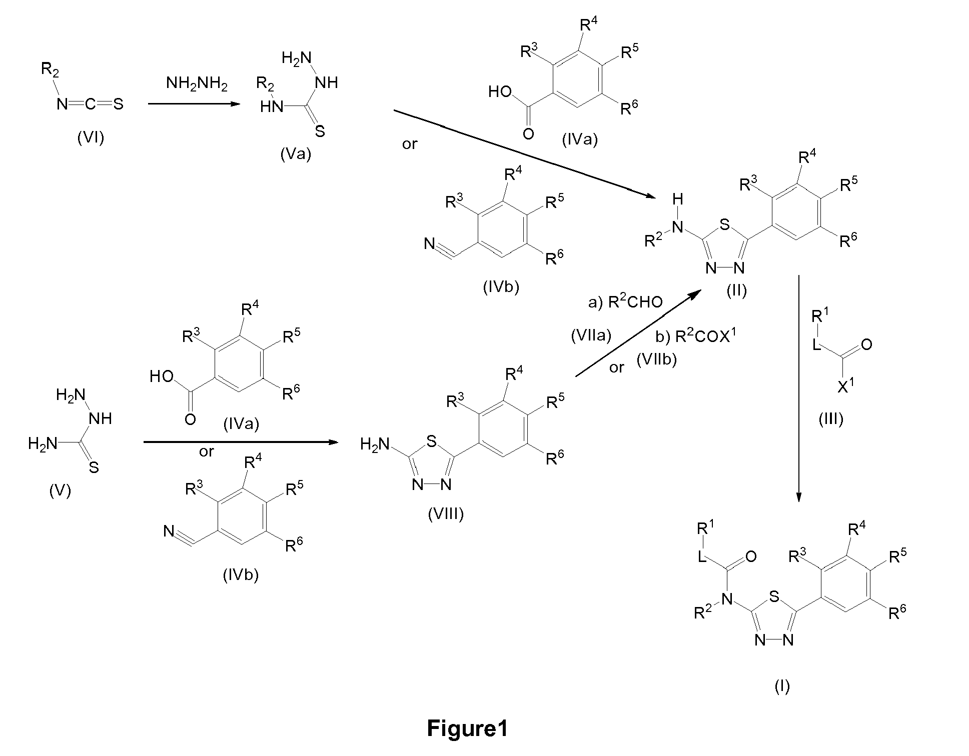

Compounds of general formula (I) may be prepared by the reaction of 1,3,4-thiadiazol-2-ylamine derivatives (II), wherein R² to R⁶ are as described above, with the corresponding acylating agent (III), wherein L is a direct bond and R¹ is as described above and X¹ represents a hydroxy group or a chlorine atom.

When X¹ is a chlorine atom, the reaction takes place in basic media such as triethylamine, pyridine or diisopropylethylamine with a solvent such as dichloromethane, dymethylformamide (DMF), tetrahydrofurane (THF) or pyridine at a temperature between 20 and 150°C and in a standard reactor or in a microwave apparatus. These reactions may be catalyzed using 4-dimethylaminopyridine.

When X¹ is a hydroxy group, a coupling agent such as 2-(1 H-7-Azabenzotriazol-1-yl)-1,1,3,3-tetramethyl uronium hexafluorophosphate (HATU) or O-Benzotriazole-N,N,N',N'-tetramethyl-uronium-hexafluoro-phosphate (HBTU) is used with a base such as triethylamine, pyridine or diisopropylethylamine, in the presence of a solvent such as dichloromethane, DMF, THF or pyridine, at a temperature between 20 and 150°C in a standard reactor or in a microwave apparatus. Other coupling agents such as 1-[3-(dimethylamino)propyl]-3-ethylcarbodiimide hydrochloride (EDC) or dicyclohexylcarbodiimide (DCC), may be used in the presence of N-hydroxybenzotriazole (HOBt) as a catalyst, in a solvent such as dichloromethane, DMF or THF at a temperature between 20 and 150°C, in a standard reactor or in a microwave apparatus.

Intermediates of general formula (II) may be prepared by condensation of the intermediates of formula (Va) with the corresponding acid derivative (IVa) using POCl₃. Alternatively they may be prepared by condensation of compounds of formula (Va) with the corresponding nitrile derivatives (IVb) in the presence of trifluoroacetic acid. Both reactions may be performed without a solvent or in a solvent such as dioxane, THF or dichloromethane at a temperature from 20 to 150°C.

Intermediates of formula (Va) may be obtained by the reaction of hydrazine hydrate with the corresponding isothiocyanate (VI) in a solvent such as THF, methanol or ethanol and at a temperature from 0 to 40°C.

Alternatively, intermediates of general formula (II) may be prepared by reductive amination of compounds of general formula (VIII) with the corresponding aldehyde (VIIa), wherein R² is as described above, in acid media such as acetic acid and in a protic solvent such as methanol or ethanol and with a reductive agent such as sodium borohydride, sodium cyanoborohydride or sodium triacetoxyborohydride at a temperature from 0°C to the boiling point of the solvent. Alternatively intermediates of general formula (II) may be prepared by acylation of intermediates of formula (VIII) with an acylating agent of formula (VIIb), wherein R² is as described above and X¹ represents a hydroxy group or a chlorine atom, using the acylating methods described above for the synthesis of compounds of general formula (I) from intermediates of formula (IV), followed by a reduction process using a reductive agent such as borane or lithium aluminium hydride in an aprotic solvent such as THF and at a temperature from 0° to the solvent boiling point.

Intermediates of general formula (VIII) may be prepared by condensation of hydrazinecarbothioamide of formula (V) with the corresponding acid derivative (IVa) using POCl₃ or with the corresponding nitrile derivative (IVb) in the presence of TFA. Both reactions may be performed without a solvent in a solvent such as dioxane or THF or dichloromethane at a temperature from 20 to 100°C.

In the particular case wherein L represents a -NH- group, the compounds of general formula (1a) may also be prepared according to the synthetic scheme depicted in figure 2.

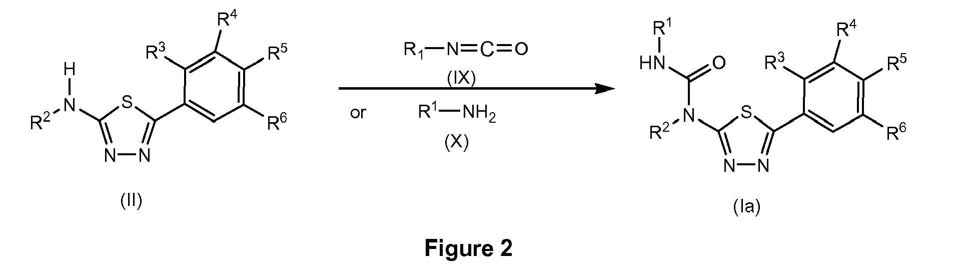

Compounds of formula (Ia) may be obtained by the reaction of 1,3,4-thiadiazol-2-ylamine derivatives (II), wherein R² to R⁶ are as described above, with fosgene or a fosgene synthetic equivalent such as trifosgene, carbonyldiimidazole (CDI), di-*para-*nitrophenyl carbonate or di-N-succinimyl carbonate in the presence of a base such as triethylamine o diisopropylethylamine in a solvent such as dichloromethane, THF or acetonitrile at a temperature from 0°C to the boiling point of the solvent, followed by the addition of the corresponding amine of formula (X) wherein R¹ is defined as described above.

Alternatively, compounds of formula (Ia) may be obtained by the reaction of 1,3,4-thiadiazol-2-ylamine derivatives (II) with the corresponding isocyanate of formula (IX) wherein R¹ is as described above in a solvent such as THF from 0°C to the boiling point of the solvent. The presence of a base such as diisopropylethylamine, pyridine or triethylamine can optionaly be required

In the particular case wherein L is a direct bond and R⁵ represents a -(CH₂)-NR'R" wherein R' and R" are as described above, the compounds of formula (Ib) may be obtained following the synthetic path shown in Figure 3.

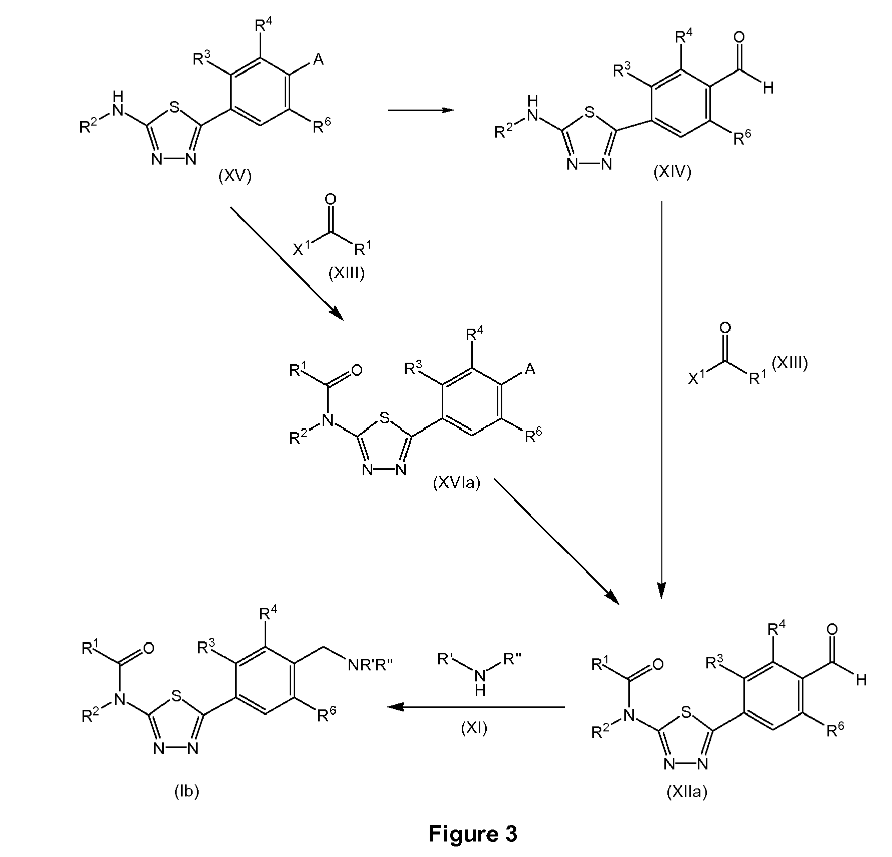

Compounds of general formula (Ib), wherein R¹, R², R³, R⁴, R⁶, R' and R" are as described above may be prepared by the reductive amination of the aldehyde derivatives of general formula (XIIa) with the corresponding amines of formula (XI) in acid media such as acetic acid and in a protic solvent such as methanol or ethanol and with a reductive agent such as sodium borohydride, sodium triacetoxyborohydride or sodium cyanoborohydride at a temperature from 0°C to the boiling point of the solvent.

Compounds of formula (XIIa) may be obtained by acylation of compounds of general formula (XIV) with the corresponding acylating agent of formula (XIII), wherein X¹ represents a chlorine atom or a hydroxy group, by standard methods as described before.

Finally, aldehyde derivatives of general formula (XIV) may be obtained by reaction of intermediates of general formula (XV), wherein A represents a -CN group, a COOR radical or a -CH=CH₂ group. In the case wherein A represents a -CN group, the reduction process may be carried out using a reductive agent such as diisobutylaluminium hydride DIBAL-H in a solvent such as dichloromethane, THF or dioxane at a low temperature from -78° to 0°C.

In the case wherein A represents a -COOR radical, the reduction process to the corresponding alcohol may be effected using LiAlH₄ as reductive agent in a solvent such as THF and the following oxidation to the corresponding aldehyde of formula (XIV) by a standard Swern oxidation or other oxidazing agents such as Dess-Martin reagent.

Finally, in the case wherein A represents a -CH=CH₂ radical, Intermediates of formula (XIV) may also be obtained by dihydroxylation and oxidative cleavage of the corresponding vinyl derivative of formula (XV) using a catalytic amount of an oxidazing agent such as osmium tetroxide and a cooxidant such as sodium periodate in a mixture of solvents such as methanol, acetonitrile and water at a temperature from 0°C to the boiling point of the mixture.

Alternatively, intermediates of formula (XIIa) may be obtained from intermediates of formula (XVIa) according to the methods described above, depending on the nature of A. Intermediates of formula (XVIa) can be obtained from intermediates of formula (XV) following the same procedures described for the obtention of intermediates of formula (XIIa) from intermediates of formula (XIV) and intermediates of formula (XIII).

In the particular case wherein L represents a -NH- group and R⁵ represents a -(CH₂)-NR'R" wherein R' and R" are as described above, compounds of formula (Ic) may be obtained following the synthetic path shown in Figure 4.

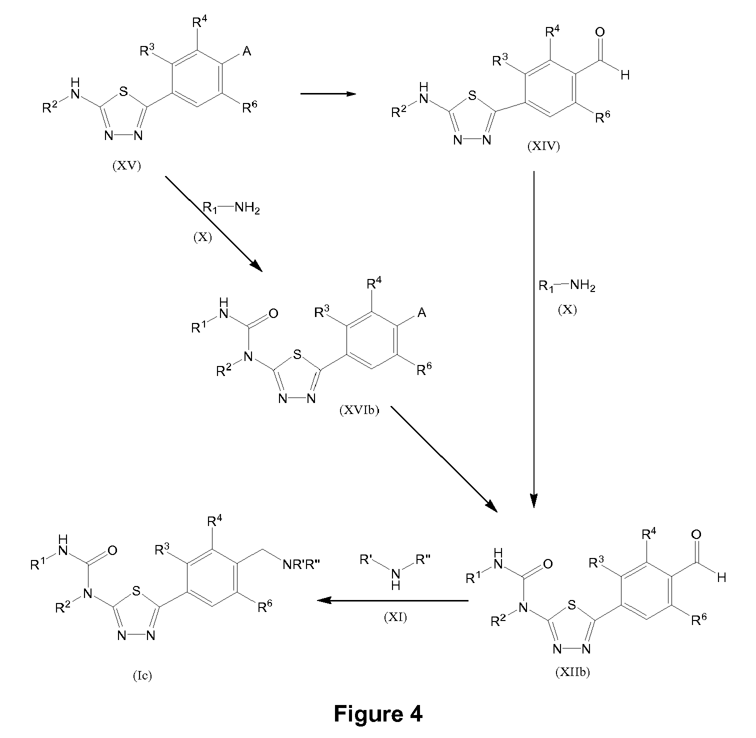

Compounds of general formula (Ic), wherein R¹, R², R³, R⁴, R⁶, R' and R" are as decribed above may be prepared by the reductive amination of the aldehyde derivatives of general formula (XIIb) with the corresponding amines of formula (XI) as described above.

Intermediates of formula (XIIb) may be obtained by the reaction of 1,3,4-thiadiazol-2-ylamine derivatives (XIV) with fosgene or a fosgene synthetic equivalent such as trifosgene, carbonyldiimidazole (CDI), di-*para*-nitrophenyl carbonate or di-N-succinimyl carbonate in the presence of a base such as triethylamine o diisopropylethylamine in a solvent such as dichloromethane, THF or acetonitrile and a temperature from 0°C to the boiling point of the solvent, followed by the addition of the corresponding amine of formula (X).

Intermediates of formula (XIV) may be obtained from intermediates of formula (XV), wherein A is as defined above, according to the methods described above, depending on the nature of A.

Alternatively, Intermediates of formula (XIIb) may be obtained from derivatives of formula (XVIb) following the same procedures described for the obtention of intermediates of formula (XIIa) from intermediates of formula (XVla) as disclosed in Figure 3. Intermediates of formula (XVlb) may be obtained from derivatives of formula (XV) following the same procedures described for the obtention of compounds of formula (1a) from intermediates of formula (II) as disclosed in Figure 2

In the particular case wherein R⁵ represents a -(CH₂)-NH-(CH₂)₍₁₋₄₎-R" wherein R" is as described above, the compounds of formula (Id) may be obtained following the synthetic path shown in Figure 5.

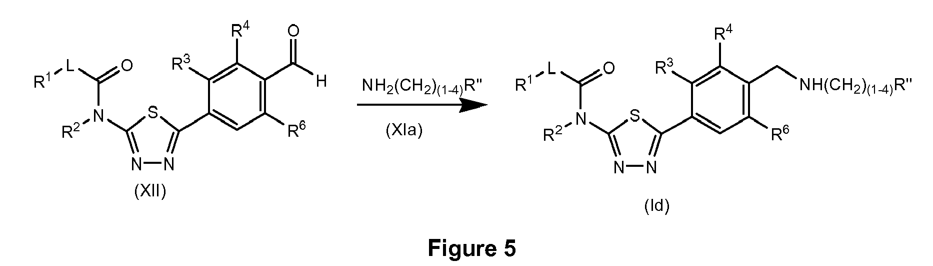

Compounds of general formula (Id), wherein R¹, R², R³, R⁴ and R⁶ are as decribed above may be prepared by the reductive amination of the aldehyde derivatives of general formula (XII) with the corresponding amines of formula (XIa) following the procedure described above, for example, for the preparation of compounds of formula (Ic) from intermediates of ofrmula (XIIb) and (XI) disclosed in figure 4.

In the particular case wherein L is a direct bond and R⁵ represents a -O-(CH₂)₂-NR'R" wherein R' and R" are as described above, the compounds of formula (Ie) may be obtained following the synthetic path shown in Figure 6.

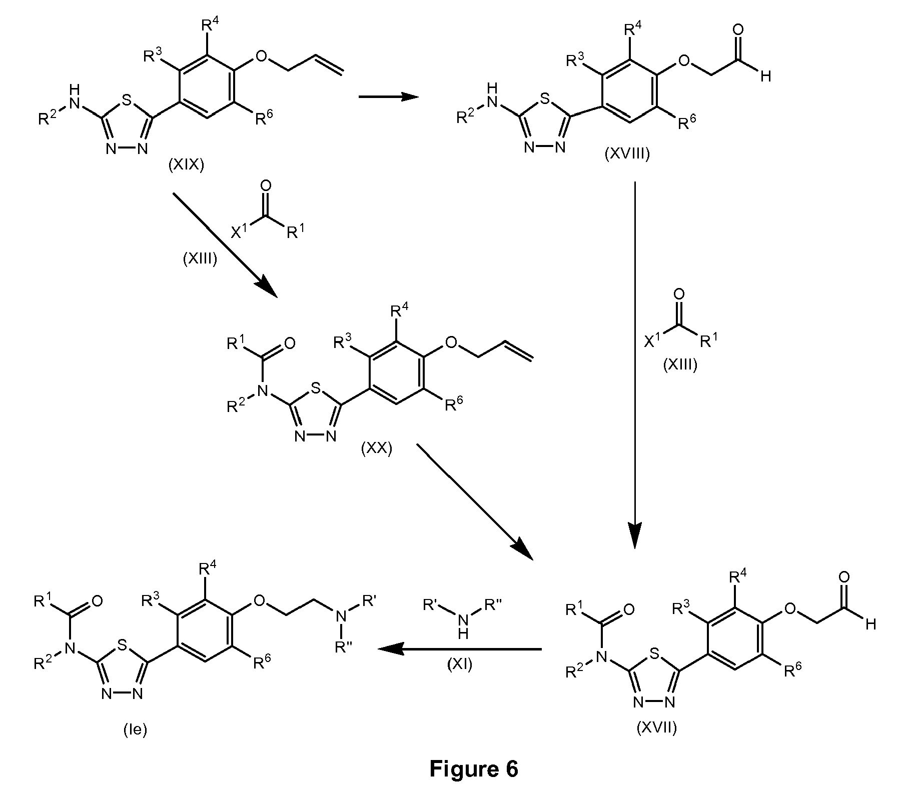

Compounds of general formula (Ie), wherein R¹, R², R³, R⁴, R⁶, R' and R" are as decribed above may be prepared by the reductive amination of the aldehyde derivatives of general formula (XVII) by the methods described above.

Intermediates of formula (XVII) may be obtained by acylation of compounds of general formula (XVIII) with the corresponding acylating agent of formula (XIII) in which X¹ represents a chlorine atom or a hydroxy group using standard methods described before.

Intermediates of general formula (XVIII) may be obtained by dihydroxylation and oxidative cleavage of the corresponding allyl derivative of formula (XIX) using a catalytic amount of an oxidazing agent such as osmium tetroxide and a cooxidant such as sodium periodate in a mixture of solvents such as methanol, acetonitrile and water at a temperature from 0°C to the boiling point of the mixture.

Alternatively, compounds of formula (XVII) may be obtained from compounds of formula (XX) by oxidative cleavage, according to the method described above. Compounds of formula (XX) may be obtained from Intermediates of formula (XIX) by acylation with compounds of general formula (XIII) following the same procedures described for the obtention of intermediates of formula (XVII) from intermediates of formula (XVIII) and intermediates of formula (XIII).

In the particular case where R⁵ represents a -O-(CH₂)₂-NH-(CH₂)₍₁₋₄₎-R" wherein R" is as described above, the compounds of formula (If) may be obtained following the synthetic path shown in Figure 7.

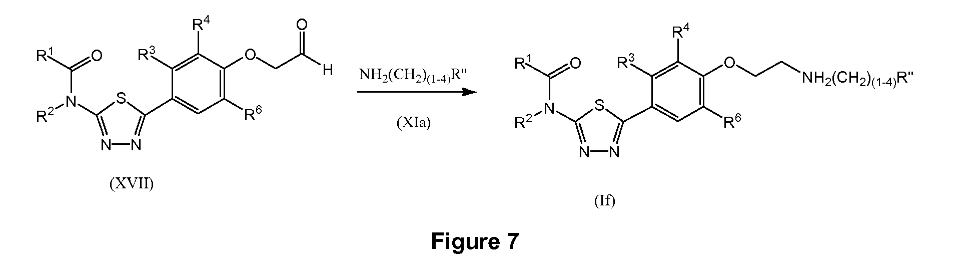

Compounds of general formula (If), wherein R¹, R², R³, R⁴, R⁶ and R" are as described above may be prepared by the reductive amination of the aldehyde derivatives of general formula (XVII) with the corresponding amines of formula (XIa) following the methods described above, for example following the synthetic step disclosed in figure 5.

In the particular case in which R⁵ represents -(CO)-NR'R", compounds of formula (1g) may be obtained following the synthetic path shown in Figure 8.

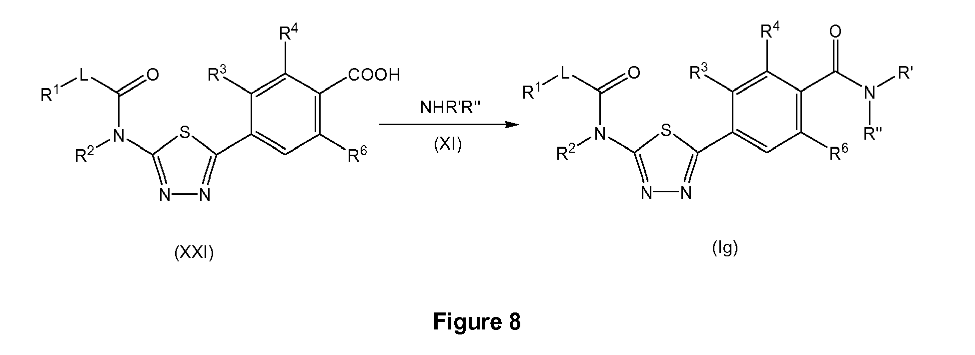

Reaction of the acid derivatives of general formula (XXI) with the corresponding amine (XI) using as a coupling agent HATU or HBTU with a base such as triethylamine, pyridine or diisopropylethylamine in a solvent such as dichloromethane, DMF, THF or pyridine, or using as a coupling agent EDC or DCC, with HOBt and in a solvent such as dichloromethane, DMFor THF, yield the final compounds of formula (Ig).

In the particular case wherein L is a direct bond and R⁵ represents an oxy radical of formula -O-(CH₂)₍₀₋₂₎-R^{a}, -O-(CH₂)₍₂₋₄₎-NR'R" or -O-(CH₂)₍₂₋₄₎-NH-(CH₂)₍₁₋₄₎-R", wherein R^{a}, R' and R" are as described above, with the exception that R^{a} is not an alkenyl group and L is a direct bond, compounds of formula (Ij) may be obtained following the synthetic path shown in Figure 9.

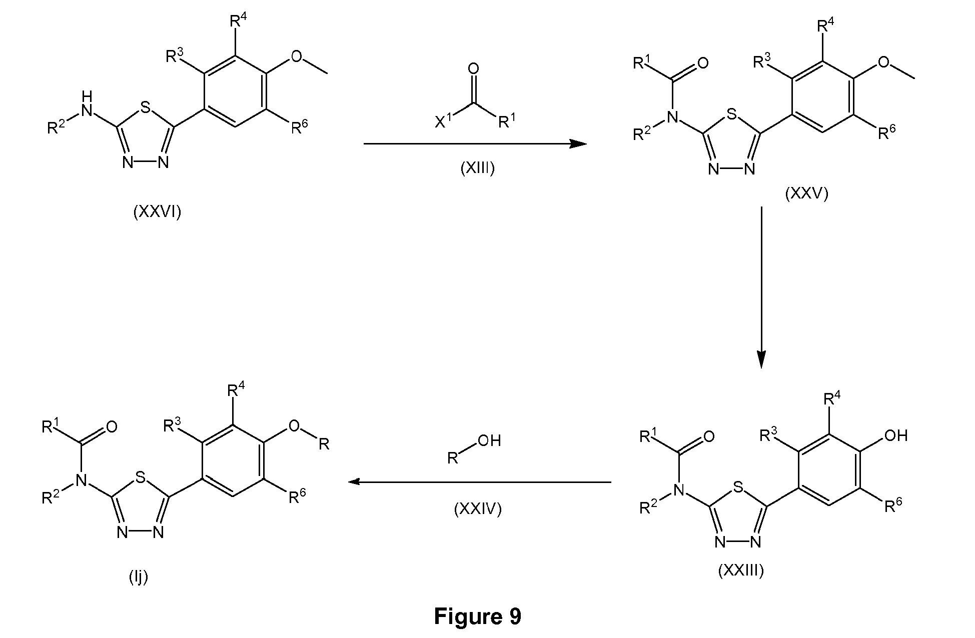

Compounds of general formula (Ij), wherein R¹, R², R³, R⁴ and R⁶ are as decribed above and -OR represents an oxy radical may be prepared by Mitsunobu reaction of the fenol derivatives of general formula (XXIII) and the alcohols of general formula (XXIV). An azodicarboxylate derivative such as diethyl azodicarboxylate (DEAD), diisoprpylazodicarboxylate (DIAD) or 1,1'-(azodicarbonyl)dipiperidine (ADDP) in the presence of a phosphine such as triphenylphosphine o tributylphosphine are used as reagents and the reaction is performed in a solvent such as THF, dioxane or diethyl ether at a temperature from 0°C to the boiling point of the solvent.

Intermediates of general formula (XXIII) may be prepared by demethylation of the corresponding derivatives of general formula (XXV) using BBr₃ or AlBr₃ or BF₃ as demethylating agent in a solvent such as dichloromethane or 1,2-dichloroethane, chloroform at a temperature between 0 and the 60°C. Alternatively compounds of general formula (XXIII) may be prepared by demethylation using HBr in acetic acid as a solvent.

Intermediates of formula (XXIII) may be obtained by acylation of compounds of general formula (XXVI) with the corresponding acylating agent of formula (XIII) in which X¹ represents a chlorine atom or a hydroxy group by standard methods as described before.

In the particular case wherein L is a direct bond and R⁵ represents a -(CH₂)₍₁₋₂₎-NR'R" wherein R' and R" are as described above and L is a direct bond, the compounds of formula (1k) may be obtained following the synthetic path shown in Figure 10. 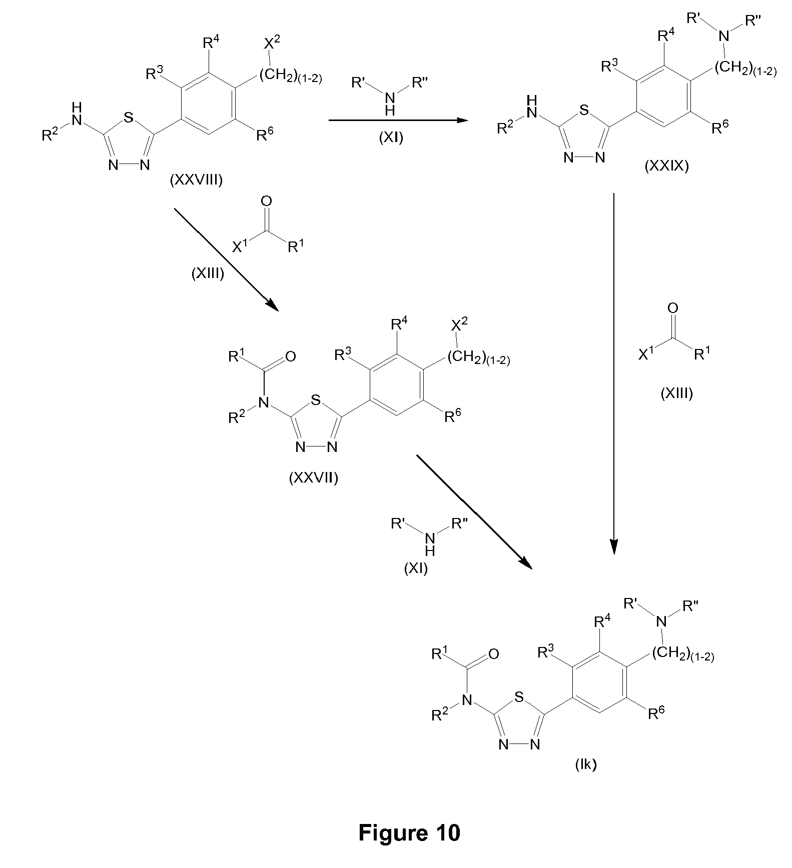 Compounds of general formula (1k), wherein R¹, R², R³, R⁴, R⁶, R' and R" are as described above may be prepared by alkylation of the haloderivatives of general formula (XXVII) in which X² represents a halogen atom selected from chlorine, bromine and iodine atoms, with the corresponding nitrogen containing compounds of formula (XI) in the presence of a base such as triethylamine, diisopropylethylamine, potassium tert-butoxide, sodium hydride in an solvent such as THF, DMF or ethanol at a temperature from 0°C to the boiling point of the solvent.

Compounds of formula (XXVII) may be obtained by acylation of compounds of general formula (XXVIII) with the corresponding acylating agent of formula (XIII) wherein X¹ is as defined above, by standard methods as described before.

Alternatively, compounds of general formula (1k) may also be obtained by acylation of compounds of general formula (XXIX) with the corresponding acylating agent of formula (XIII) by standard methods as described before. Intermediates of general formula (XXIX) may be obtained by alkylation of haloderivatives of general formula (XXVIII) with the corresponding nitrogen containing compounds of formula (XI) by the method described above.

In the particular case wherein L is a direct bond and R⁵ represents a -(CH₂)₍₁₋₂₎-NH-(CH₂)₍₁₋₄₎-R" wherein R" is as described above, the compounds of formula (Ik) may be obtained following the synthetic path shown in Figure 11.

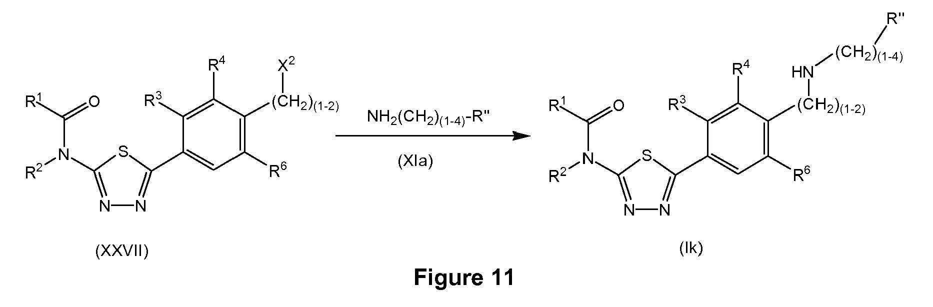

Compounds of general formula (1k), wherein R¹, R², R³, R⁴, R⁶ and R" are as described above may be prepared by the reductive alkylation of the haloderivatives of general formula (XXVII) in which X² represents a halogen atom selected from chlorine, bromine and iodine atoms,with the corresponding amines of formula (XIa) in the presence of a base such as triethylamine, diisopropylethylamine, sodium hydride in an aprotic solvent such as THF or DMF at a temperature from 0°C to the boiling point of the solvent.

When the defined groups R¹ to R⁶, R' and R" are susceptible to chemical reaction under the conditions of the hereinbefore described processes or are incompatible with said processes, conventional protecting groups may be used in accordance with standard practice, for example see T.W. Greene and P.G.M. Wuts in "protective Groups in Organic Chemistry", 3rd Edition, John Wiley&Sons (1999). It may be that deprotection will form the last step in the synthesis of compounds of formula (I).

The syntheses of the compounds of the invention and their intermediates are illustrated by the following Examples (1 to 76) including Preparation Examples (1 to 79) which do not limit the scope of the invention in any way.

Starting compounds are commercially available or may be obtained following the conventional synthetic method already known in the art.

¹H Nuclear Magnetic Resonance Spectra were recorded on a Varian Mercury 200 spectrometer. Low Resolution Mass Spectra (m/z) were recorded on a Micromass ZMD mass spectrometer using ESI ionization. The chromatographic separations were obtained using a Waters 2690 system equipped with a Symmetry C18 (2.1 x 50 mm, 3.5 µM) column for method A and B and a Symmetry C18 (2.1 x 100 mm, 3.5 µM) for method C. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A), the gradients are specified in the following table for each methode used. The reequilibration time between two injections was 1 min. The flow rate was 0.8 mL/min for method A and 0.4 mL/min for method B and C. The injection volume was 5 microliter for method A and B and 3 microliter for method C. Diode array chromatograms were collected at 210 nM.

| Method | 0% B | 0 to 95% B | 95% B |
|---|---|---|---|
| A | 0.2 min | 3min | 0.8min |
| B | 0.5min | 6.5min | 1min |
| C | 0min | 20min | 4min |

### Purification method A:

The solid was dissolved in DMSO/MeOH, injected into a Biotage C18 silica column (40M, 25M or 25S according to the crude amount) and eluted on the SP1® automated purification system from Biotage. The gradient used was H2O/Acetonitrile/MeOH (1:1) (0.1% v/v HCOONH4 both phases) from 0% to 100% acetonitrile/MeOH (1:1) in 80 column volumes. The appropriate fractions were collected and the organic solvent evaporated under reduced pressure or liofilized.

### PREPARATION EXAMPLES

### PREPARATION 1

### 4-(Allyloxy)-3,5-dimethylbenzonitrile

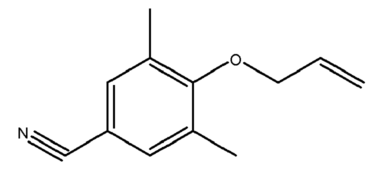

To a 0°C stirred solution of 4-hydroxy-3,5-dimethylbenzonitrile (10 g, 0.07 mol) in DMF (100ml), 60% NaH (3.0g, 0.08 mol) was added portionwise and the final mixture was stirred at that temperature for 30 min. Then a solution of 3-bromoprop-1-ene (8.6 g, 0.07 mol) in DMF (25ml) was added dropwise and the mixture was stirred at room temperature for 5h. The reaction crude was slowly poured onto ice/water to yield a solid that was filtered, washed with water and dried. 12,5g (98% yield) of the title compound were obtained.
LRMS: m/z 188 (M+1)⁺
Retention time: 6.52 min (method B)
¹H NMR (400 MHz, CHLOROFORM-*d*) δ ppm 2.29 (s, 6 H) 4.34 (d, *J*=5.86 Hz, 2 H) 5.29 (dd, *J* = 10.4, 1.1 Hz, 1 H) 5.43 (dd, *J*=17.19, 1.56 Hz, 1 H) 6.02 - 6.15 (m, 1H), 7.33 (s, 2 H)

### PREPARATION 2

### 4-(allyloxy)-3,5-dimethylbenzoic acid

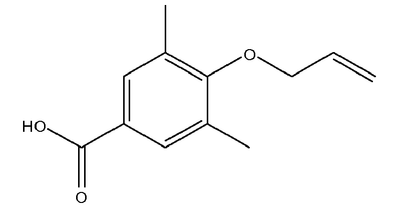

In a sealed tube the title compound of preparation 1 (1 g, 5.34 mmol) was suspended in 8 M aqueous NaOH solution (5 ml). Ethanol was added (1ml) and the mixture was stirred at 110°C for 5h and then at room temperature overnight. Dichloromethane and water were added to the mixture and it was acidified with 6 M HCI to pH 1. Layers were separated and the aqueous one was extrated with dichloromethane twice. The combined organic alyers were washed with water and brine, dried and solvent was finally removed in vaccuo. The title compound was obtained as a yellow solid (1.02g, 91% yield).
LRMS: m/z 207 (M+1)⁺
Retention time: 3.15 min (method A)

### PREPARATION 3

### N-Butylhydrazinecarbothioamide

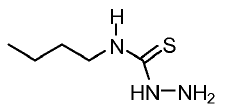

To a 10°C stirred solution of hydrazine hydrate (32 ml, 0.66 mol) in methanol (500 ml), butylisothiocyanate (25 g, 0.22 mol) was added dropwise keeping the temperature between 10-15°C. The final mixture was stirred for 1h at 10°C and the solvent was removed to yield oil that was lyophilized. A white solid was obtained. It was washed with diethyl ether. 28g (87% yield).
LRMS: m/z 148 (M+1)⁺
Retention time: 4.17min (method B)
¹H NMR (200 MHz, CDCl₃) δ ppm 1.0 (t, *J*=7.0 Hz, 3 H) 1.4 (m, 2 H) 1.6 (m, 2 H) 3.6 (m, 2 H) 3.8 (s, 2 H) 7.4 (s, 1 H) 7.8 (s, 1 H)

### PREPARATION 4

### 5-(4-(Allyloxy)-3,5-dimethylphenyl)-N-butyl-1,3,4-thiadiazol-2-amine

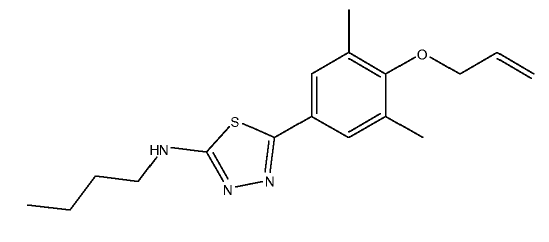

To a stirred solution of the title compound of Preparation 2 (10.43g, 50.6 mmol) in dioxane (100 ml), the title compound of Preparation 3 (8.10 g, 55.0 mmol) was added portionwise. POCl3 (8.0 ml, 87.7 mmol) was added dropwise and the final mixture was stirred at 80°C for 5h and then at room temperature overnight. Solvent was removed in vaccuo and the solid thus obtained was partiotioned between water and dichloromethane. It was basified to pH 8-9 and the organic layer was washed with brine. It was dried and solvent was removed in vaccuo to yield a solid that was washed with hexane and ethyl ether. 11.7 g (73% yield) of the title compound were obtained.
LRMS: m/z 318 (M+1)⁺
Retention time: 3.68 min (method A)

### PREPARATION 5

### N-(5-(4-(Allyloxy)-3,5-dimethylphenyl)-1,3,4-thiadiazol-2-yl)-N-butyl-2-fluorobenzamide

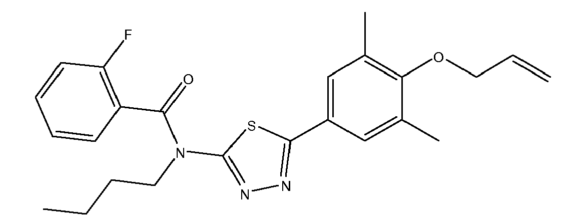

To a 0°C stirred solution of the title compound of Preparation 4 (3g, 9.45 mmol) in THF (30 ml), 60% NaH (0.8g, 20 mmol) was added and the mixture was stirred at that temperature for 30 min. Then a solution of 2-fluorobenzolyl chloride (3.3 g, 20.81 mmol) in THF (24 ml) was added dropwise and the final mixture was stirred at rt for 1.5 h. 2N HCI (50 ml) was slowly added and the final mixture was portioned between water and ethyl acetate. The aqueous layer was extracted with ethyl acetate and the combined organic layers were washed with water and brine. It was dried and solvent removed to yield a crude product that was purified on a Biotage 40S chromatography column of silica gel using hexane/ethyl acetate 10% as eluent. 3.75 g (90% yield) of the title product were obtained as a oil.
LRMS: m/z 440 (M+1)⁺
Retention time: 4.29 min (method B)

### PREPARATION 6

### N-butyl-N-(5-(3,5-dimethyl-4-(2-oxoethoxy)phenyl)-1,3,4-thiadiazol-2-yl)-2-fluorobenzamide

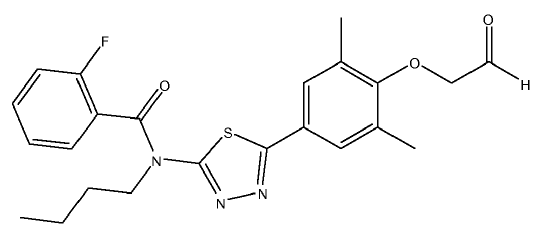

To a stirred solution of the title compound of Preparation 5 (3.75 g, 8.53 mmol) in THF (80 ml) and tert-butanol (12ml), osmium tetroxide (4% aqueous solution, 1.10 ml, 0.18 mmol) and N-methylmorpholine (2.04 g, 17.41 mmol) were added and the mixture was stirred at rt overnight. Solvent was then removed and the solid thus obtained was portioned between water and ethyl acetate. The organic layer was washed with water and brine, dried and solvent removed to yield a solid that was solved with methanol (100 ml) and water (11 ml). Sodium periodate (2.90 g, 13.56 mmol) was added and the mixture was stirred at rt for 2h. Solvent was removed and the solid thus obtained was partiotioned between water and ethyl acetate. The organic layer was washed with water and brine. Solvent was removed to yield the title compound (3.98 g, 93% yield).
LRMS: m/z 460 (M+H₂O+1)⁺ y 474 (M+MeOH+1)⁺
Retention time: 6.70, 7.08 min (method B)

### PREPARATION 7

### 5-(4-(Allyloxy)-3,5-dimethylphenyl)-N-ethyl-1,3,4-thiadiazol-2-amine

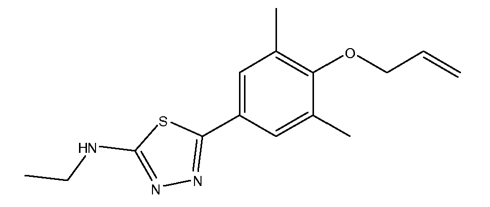

To a stirred solution of the title compound of Preparation 1 (10 g, 53.40 mmol) in TFA (22.6ml), N-Ethylhydrazinecarbothioamide (7.70 g, 64.60 mmol) was added.The final mixture was stirred at 80°C for 5h under nitrogen atmosphere. The reaction crude was slowly poured onto ice/water and basified with (4%) NaHCO₃ to pH 7-8. The aqueous layer was extracted twice with dichloromethane and the combined organic layers were washed with water and brine. The organic layer was dried and the solvent removed in vaccuo to yield an oil that was triturated with ethyl ether to yield 6.05 g of the title compound as a white-blue solid. The mother liquors were purified on a Biotage 65M chromatography column of silica gel using hexane/ethyl acetate (0-40%) as eluent. 1.9 g of the title product were obtained as a yellow solid. Global yield: 48%.
LRMS: m/z 290 (M+1)⁺
Retention time: 6.57 min (method B)

### PREPARATION 8

### N-(5-(4-(Allyloxy)-3,5-dimethylphenyl)-1,3,4-thiadiazol-2-yl)-N-ethyl-2-fluorobenzamide

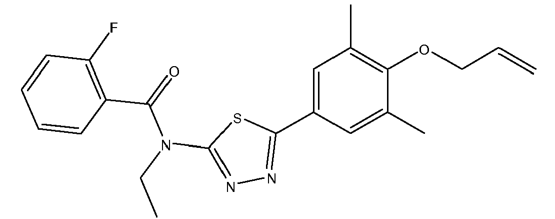

Obtained (81% yield) from the title compound of Preparation 7 and 2-fluorobenzoyl chloride following the experimental procedure of Preparation 5. The final product was triturated with ethyl ether.
LRMS: m/z 412 (M+1)⁺
Retention time: 7.52 min (method B)

### PREPARATION 9

### N-(5-(4-(Allyloxy)-3,5-dimethylphenyl)-1,3,4-thiadiazol-2-yl)-N-ethyl-2-chlorobenzamide

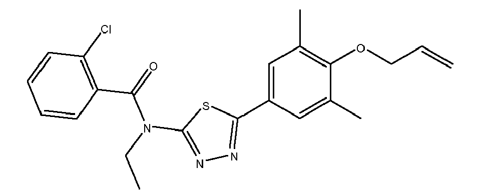

Obtained (75% yield) from the title compound of Preparation 7 and 2-chlorobenzoyl chloride following the experimental procedure of Preparation 5. The final product was triturated with ethyl ether.
LRMS: m/z 428 (M+1)⁺
Retention time: 7.63 min (method B)

### PREPARATION 10

### N-(5-(4-(Allyloxy)-3,5-dimethylphenyl)-1,3,4-thiadiazol-2-yl)-N-ethyl-3-chloro-2-fluorobenzamide

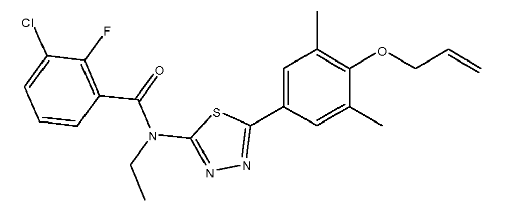

Obtained (91% yield) from the title compound of Preparation 7 and 3-chloro-2-fluorobenzoyl chloride following the experimental procedure of Preparation 5. The final product was triturated with ethyl ether.
LRMS: m/z 446 (M+1)⁺
Retention time: 7.73 min (method B)
¹H NMR (400 MHz, Chloroform-d) δ ppm 1.34 (t, *J*=6.84 Hz, 3 H) 2.36 (s, 6 H) 4.22 (d, *J*=6.25 Hz, 2 H) 4.37 (d, *J*=5.47 Hz, 2 H) 5.30 (d, *J*=10.55 Hz, 1 H) 5.46 (dd, *J*=17.19, 1.56 Hz, 1 H) 6.06 - 6.20 (m, 1 H) 7.23 - 7.31 (m, 1 H) 7.35 (t, *J*=5.86 Hz, 1 H) 7.59 (t, *J*=6.84 Hz, 1 H) 7.67 (s, 2 H)

### PREPARATION 11

### N-{5-[3,5-dimethyl-4-(2-oxoethoxy)phenyl]-1,3,4-thiadiazol-2-yl}-N-ethyl-2-fluorobenzamide

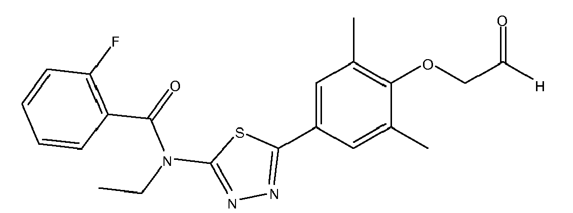

Obtained (98% yield) from the compound of Preparation 8 following the experimental procedure of Preparation 6.
LRMS: m/z 432 (M+H₂O+1)⁺ y 446 (M+MeOH+1)⁺
Retention time: 6.15 y 6.60 min (method B)

### PREPARATION 12

### N-ethyl-N-(5-(3,5-dimethyl-4-(2-oxoethoxy)phenyl)-1,3,4-thiadiazol-2-yl)-2-chlorobenzamide

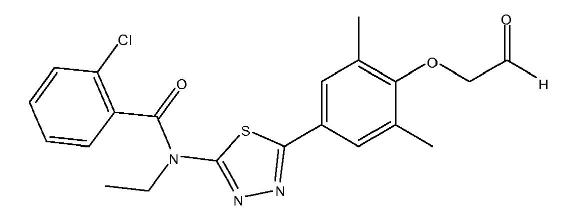

Obtained (100% yield) from the compound of Preparation 9 following the experimental procedure of Preparation 6.
LRMS: m/z 448 (M+H₂O+1)⁺ y 462 (M+MeOH+1)⁺
Retention time: 6.33 y 6.77 min (method B)

### PREPARATION 13

### 3-chloro-N-{5-[3,5-dimethyl-4-(2-oxoethoxy)phenyl]-1,3,4-thiadiazol-2-yl}-N-ethyl-2-fluorobenzamide

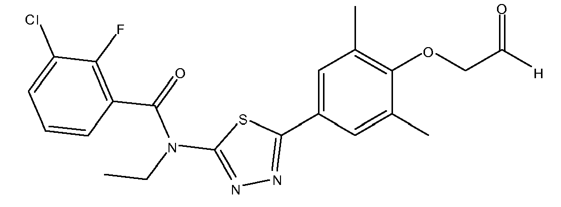

Obtained (20% yield) from the compound of Preparation 10 following the experimental procedure of Preparation 6.
LRMS: m/z 448 (M+H₂O+1)⁺ y 462 (M+MeOH+1)⁺
Retention time: 6.33 y 6.77 min (method B)

### PREPARATION 14

### N-(Cyclopropylmethyl)hydrazinecarbothioamide

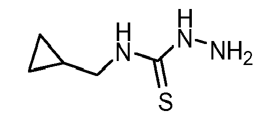

Obtained (93%) from (isothiocyanatomethyl)cyclopropane following the experimental procedure of Preparation 3.
LRMS: m/z 146 (M+1)⁺
Retention time: 3.60 min (method B)

### PREPARATION 15

### 5-(4-(allyloxy)-3,5-dimethylphenyl)-N-(cyclopropylmethyl)-1,3,4-thiadiazol-2-amine

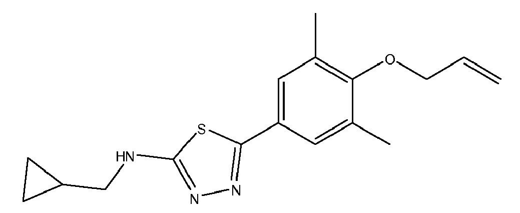

Obtained (46% yield) from the title compound of Preparation 1 and N-(Cyclopropylmethyl)hydrazinecarbothioamide (Preparation 14) following the experimental procedure of Preparation 7. The final product was triturated with ethyl ether.
LRMS: m/z 316 (M+1)⁺
Retention time: 6.88 min (method B)

### PREPARATION 16

### N-{5-[4-(allyloxy)-3,5-dimethylphenyl]-1,3,4-thiadiazol-2-yl}-2-chloro-N-(cyclopropylmethyl)benzamide

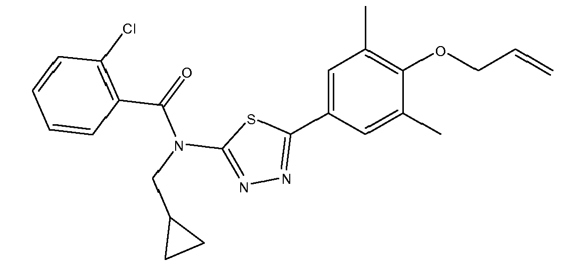

Obtained (45% yield) from the title compound of Preparation 15 and 2-chlorobenzoyl chloride following the experimental procedure of Preparation 5. The final product was triturated with ethyl ether.
LRMS: m/z 454 (M+1)⁺
Retention time: 7.75 min (method B)

### PREPARATION 17

### N-{5-[4-(allyloxy)-3,5-dimethylphenyl]-1,3,4-thiadiazol-2-yl}-2-chloro-N-(cyclopropylmethyl)-3-fluorobenzamide

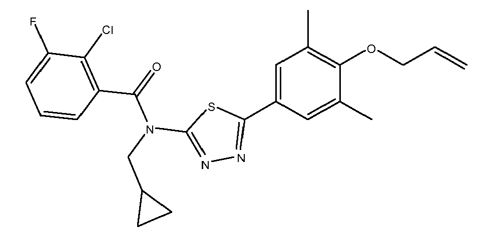

Obtained (72% yield) from the title compound of Preparation 15 and 2-chloro-3-fluorobenzoyl chloride following the experimental procedure of Preparation 5.
LRMS: m/z 472 (M+1)⁺
Retention time: 7.82 min (method B)
¹H NMR (400 MHz, CHLOROFORM-*d*) δ ppm 0.12 (br. s., 1 H) 0.29 (br. s., 1 H) 0.37 - 0.59 (m, 2 H) 1.20 - 1.32 (m, 1 H) 2.36 (s, 6 H) 3.65 - 3.80 (m, 1 H) 4.37 (d, *J*=5.47 Hz, 3 H) 5.30 (d, *J*=10.55 Hz, 1 H) 5.46 (d, *J*=17.19 Hz, 1 H) 6.05 - 6.20 (m, 1 H) 7.26 - 7.35 (m, 2 H) 7.37 - 7.46 (m, 1 H) 7.67 (s, 2 H).

### PREPARATION 18

### 2-Chloro-N-(cyclopropylmethyl)-N-{5-[3,5-dimethyl-4-(2-oxoethoxy)phenyl]-1,3,4-thiadiazol-2-yl}benzamide

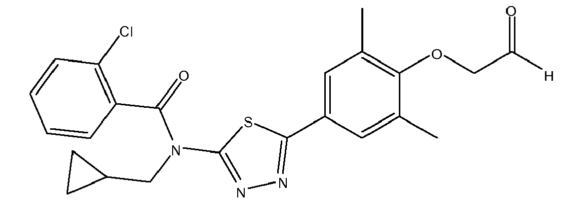

Obtained (100% yield) from the compound of Preparation 16 following the experimental procedure of Preparation 6.
LRMS: m/z 474 (M+H₂O+1)⁺ and 488 (M+MeOH+1)⁺
Retention time: 6.67 and 7.05 min (method B)

### PREPARATION 19

### 2-Chloro-N-(cyclopropylmethyl)-N-{5-[3,5-dimethyl-4-(2-oxoethoxy)phenyl]-1,3,4-thiadiazol-2-yl}-3-fluorobenzamide

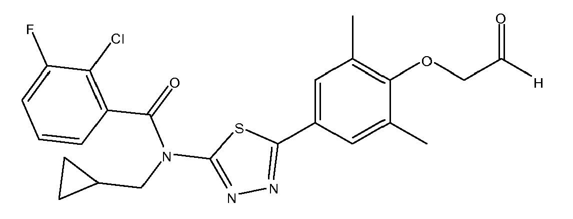

Obtained (99% yield) from the compound of Preparation 17 following the experimental procedure of Preparation 6.
LRMS: m/z 492 (M+H₂O+1)⁺ and 506 (M+MeOH+1)⁺
Retention time: 6.78 and 7.17 min (method B)

### PREPARATION 20

### N-butyl-5-(4-vinylphenyl)-1,3,4-thiadiazol-2-amine

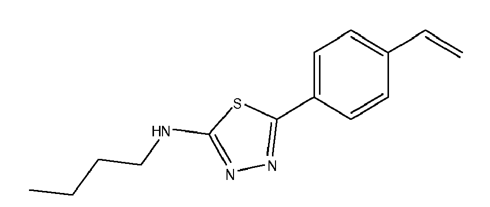

Obtained (79% yield) from the title compound of Preparation 3 and 4-vinylbenzoic acid following the experimental procedure of Preparation 4.
LRMS: m/z 260 (M+1)⁺
Retention time: 6.75 min (method B)
¹H NMR (200 MHz, DMSO-*d*₆) δ ppm 0.91 (t, *J*=7.22 Hz, 3 H) 1.23 - 1.49 (m, 2 H) 1.48 - 1.72 (m, 2 H) 3.21 - 3.40 (m, 2 H) 5.34 (d, *J*=10.93 Hz, 1 H) 5.92 (d, *J*=17.57 Hz, 1 H) 6.78 (dd, *J*=17.57, 10.93 Hz, 1 H) 7.45 - 7.87 (m, 4 H) 7.99 (t, 1 H).

### PREPARATION 21

### 4-(5-(butylamino)-1,3,4-thiadiazol-2-yl)benzaldehyde

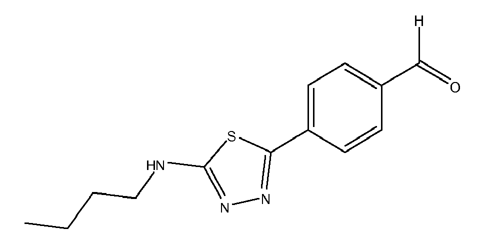

To a stirred solution of the title compound of Preparation 20 (2.28 g, 8.79 mmol) in acetone (50 ml) and water (10ml), osmium tetroxide (4% aqueous solution, 1.29 ml, 5.07 mmol) and sodium periodate ( 5.65 g, 26.4 mmol) were added and the mixture was stirred at rt for 3 hours. The suspension was filtered and the filtrate concentrated. The solid obtained was portioned between water and ethyl acetate. The organic layer was washed with water, dried and solvent removed to yield the title compound (1.76 g, 64% yield).
LRMS: m/z 262 (M+1)⁺
Retention time: 5.88 min (method B)

### PREPARATION 22

### N-butyl-N-(5-(4-formylphenyl)-1,3,4-thiadiazol-2-yl)-2-phenylacetamide

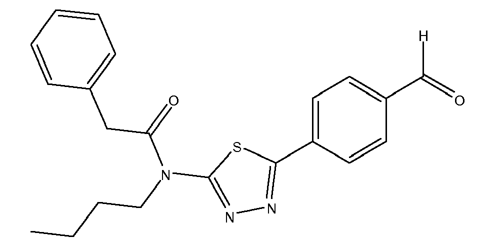

To a stirred solution of the title compound of Preparation 21 (2.6 g, 9.95 mmol) in dichloromethane (30 ml), ethyldiisopropylamine (5 ml, 28.7 mmol) and 2-phenylacetyl chloride (1.69 g, 10.9 mmol) were added and the mixture was stirred at room temperature overnight. Dichloromethane and water was added to the solution, the organic layer was washed with water, dried and solvent removed.The solid thus obtained was purified according to purification method A. 2.10g (50% yield) were obtained.
LRMS: m/z 380 (M+1)⁺
Retention time: 7.17 min (method B)

### PREPARATION 23

### N-butyl-N-(5-(4-formylphenyl)-1,3,4-thiadiazol-2-yl)-2-(2-methoxyphenyl)-acetamide

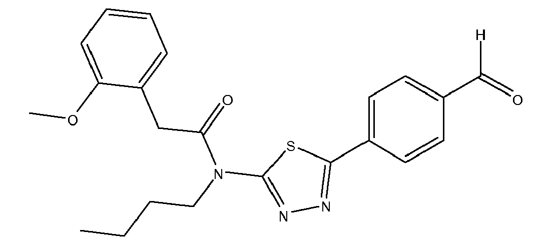

Obtained (42% yield) from the title compound of Preparation 21 and 2-(2-methoxyphenyl)acetyl chloride following the experimental procedure of Preparation 5.
LRMS: m/z 410 (M+1)⁺
Retention time: 7.20 min (method B)

### PREPARATION 24

### N-butyl-2-fluoro-N-(5-(4-formylphenyl)-1,3,4-thiadiazol-2-yl)benzamide

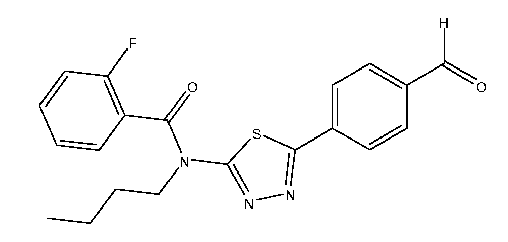

To a stirred solution of the title compound of Preparation 21 (2.0 g, 7.65 mmol) in dichloromethane (70 ml), ethyldiisopropylamine (5.3 ml, 30.6 mmol) and 4-Di(methylamino)pyridine (0.47 g, 3.83 mmols) were added and stirred at rt for 10 minutes. After that 2-fluorobenzoyl chloride (1.13 g, 9.53 mmol) was added and the mixture was stirred at 60 °C for 2 hours. The solvent was evaporated and the residue was portioned between water and ethyl acetate. The organic layer was washed with water, dried and solvent removed. The solid thus obtained was purified according to purification method A. 2.50g (85% yield) were obtained.
LRMS: m/z 384 (M+1)⁺
Retention time: 7.08 min (method B)

### PREPARATION 25

### N-butyl-5-(2-methyl-4-vinylphenyl)-1,3,4-thiadiazol-2-amine

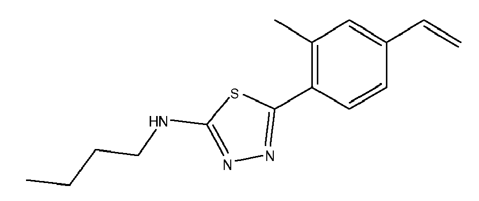

Obtained (95% yield) from the title compound of Preparation 3 and 2-methyl-4-vinylbenzoic acid following the experimental procedure of Preparation 4.
LRMS: m/z 274 (M+1)⁺
Retention time: 6.95 min (method B)

### PREPARATION 26

### 4-(5-(butylamino)-1,3,4-thiadiazol-2-yl)-3-methylbenzaldehyde

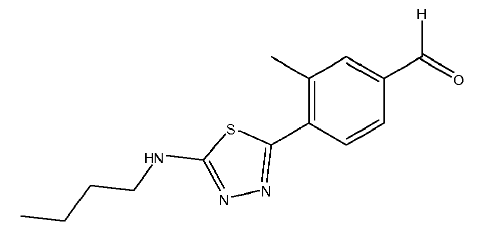

Obtained (36% yield) from the title compound of Preparation 25 following the experimental procedure of Preparation 21.
LRMS: m/z 276 (M+1)⁺
Retention time: 6.07 min (method B)

### PREPARATION 27

### N-butyl-N-(5-(4-formyl-2-methylphenyl)-1,3,4-thiadiazol-2-yl)-2-(2-methoxyphenyl) acetamide

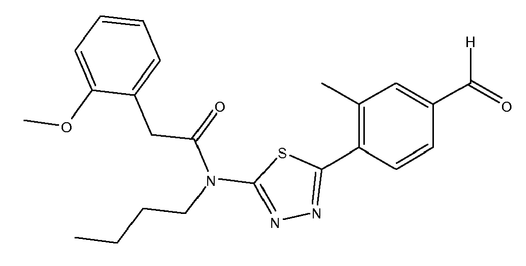

Obtained (15% yield) from the title compound of Preparation 26 and 2-(2-methoxyphenyl)acetyl chloride following the experimental procedure of Preparation 5.
LRMS: m/z 424 (M+1)⁺
Retention time: 7.25 min (method B)

### PREPARATION 28

### N-Butyl-5-(4-methoxy-3,5-dimethylphenyl)-1,3,4-thiadiazol-2-amine

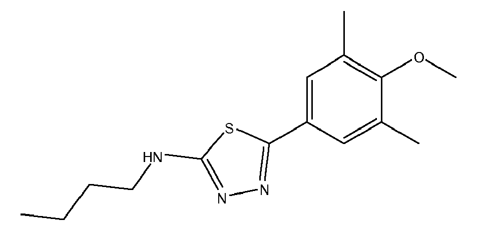

Obtained (99% yield) from the title compound of Preparation 3 and 4-methoxy-3,5-dimethylbenzoic acid following the experimental procedure of Preparation 4. The final product was recrystallized from isopropanol.
LRMS: m/z 292(M+1)⁺
Retention time: 6.67min (method B)
¹H NMR (200 MHz, DMSO-*d*₆) δ ppm 0.92 (t, *J*=7.22 Hz, 3 H) 1.39 (dq, *J*=14.54, 7.13 Hz, 2 H) 2.28 (m, 2 H) 3.38 (t, *J*=6.64 Hz, 2 H) 3.70 (s, 3 H) 4.58 - 5.60 (m, 6 H) 7.48 (s, 2 H).

### PREPARATION 29

### 4-(5-(Butylamino)-1,3,4-thiadiazol-2-yl)-2,6-dimethylphenol

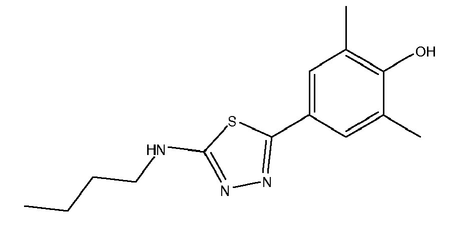

To a stirred suspension of the title product of Preparation 28 (2.0 g, 6.86 mmol) in dichloromethane (20 ml) at -78°C, a 1 M BBr3 solution in dichloromethane (10.3 ml, 10.3 mmol) was added and the mixture was stirred at that temperature for 30 min and then at room temperature overnight. The reaction mixture was poured onto ice water, diluted with dichloromethane and neutralized with saturated solution of potassium bicarbonate. The organic layer was collected and solvent was removed to yield a solid that was washed with ethyl ether. 1.64 g (87% yield) of the title product were obtained.
LRMS: m/z 278 (M+1)⁺
Retention time: 6.09min (method B)
¹H NMR (200 MHz, DMSO-D6) δ ppm 0.9 (t, *J*=7.2 Hz, 3 H) 1.4 (m, 2 H) 1.6 (m, 2 H) 2.2 (s, 6 H) 3.3 (m, 2 H) 7.3 (s, 2 H) 8.0 (s, 1 H) 8.8 (s, 1 H)

### PREPARATION 30

### N-(2-(4-(5-(butylamino)-1,3,4-thiadiazol-2-yl)-2,6-dimethylphenoxy)ethyl)acetamide

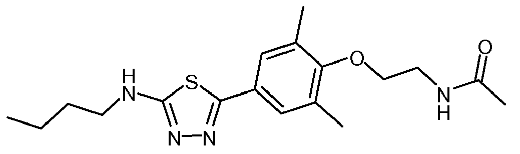

The title product of Preparation 29 (600 mg, 2.16 mmol), N-(2-hydroxyethyl)acetamide (276 mg, 2.68 mmo), triphenylphosphine (710 mg, 2.71 mmol) were mixed under Ar atmosphere followed by the addition of dry THF (5 ml). The mixture was cooled at 0° and DIAD was added drop wise. The reaction mixture was allowed to warm to rt and then stirred overnight. Solvent was removed and the residue thus obtained was partitioned between water and ethyl acetate. The organic layer was washed with water and brine. It was dried and solvent removed to yield a crude product that was purified according to purification method A to yield the title compound as a solid. 130 mg (17% yield) of the title product were obtained as a solid.
LRMS: m/z 363 (M+1)⁺
Retention time: 4.32 min (method B)
¹H NMR (200 MHz, DMSO-D6) δ ppm 0.92 (t, 3 H) 1.29 - 1.45 (m, 2 H) 1.55 (d, *J*=7.14 Hz, 2 H) 1.77 (s, 3 H) 2.20 (s, 6 H) 3.06 (t, *J*=6.59 Hz, 2 H) 3.24 - 3.45 (m, 2 H) 3.85 - 4.01 (m, 2 H) 7.23 (s, 2 H) 7.96 - 8.07 (m, 1 H) 8.85 - 8.93 (m, 1 H)

### PREPARATION 31

### 3-(4-(5-(N-butyl-2-fluorobenzamido)-1,3,4-thiadiazol-2-yl)benzylamino)propanoic acid

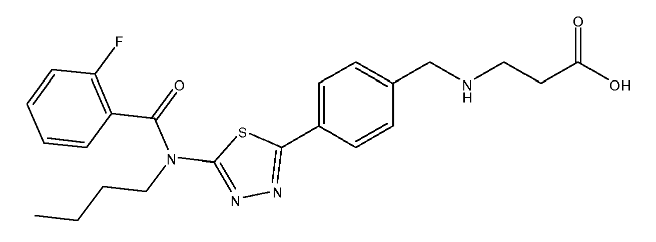

To a stirred suspension N-butyl-2-fluoro-N-(5-(4-formylphenyl)-1,3,4-thiadiazol-2-yl)benzamide (Preparation 24) (500 mg, 1.30 mmol) and 3-aminopropanoic acid (140 mg, 1.57 mmol) in methanol (12ml), AcOH (300 µL, 5.25 mmol) was added and the mixture was stirred at rt for 1h. Then, NaCNBH3 (41 mg, 0.65 mmol) was added portionwise and the final mixture was stirred for 6 hours. Solvent and acetic acid were removed in vaccuo. The crude obtained was purified according to purification method A. 391 mg (66% yield) of the title product were obtained.
LRMS: m/z 457(M+1)⁺
Retention time: 5.30 min (method B)

### PREPARATION 32

### tert-Butyl 3-(1 H-tetrazol-5-yl)azetidine-1-carboxylate

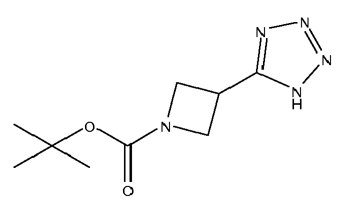

In a Schlenk tube, tert-Butyl 3-cyanoazetidine-1-carboxylate (250 mg, 1.37 mmol) and azidotrimethylstannane (452 mg, 2.20 mmol) were dissolved in toluene (6.5 ml) and stirred at 100 °C overnight. Solvent was removed and the solid obtained purified according to purification method A. 173 mg (56% yield) of the title product were obtained.
LRMS: m/z 226 (M+1)⁺
Retention time: 4.63 min (method B)

### PREPARATION 33

### 5-(azetidin-3-yl)-1H-tetrazole

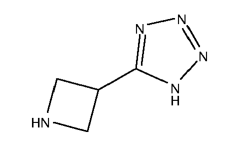

To a stirred solution of the title compound of preparation 32 (173 mg, 0.77 mmol) in methanol (6 ml), 2 ml of HCI 4M in dioxane were added and the mixture was stirred at room temperature overnight. Solvent was removed *in vaccuo* and the title compound obtained as hydrochloride.
(100% yield)
LRMS: m/z 126 (M+1)⁺
Retention time: 0.53 (method B)

### PREPARATION 34

### 4-(5-(butylamino)-1,3,4-thiadiazol-2-yl)-2,6-dimethylphenyl trifluoromethanesulfonate

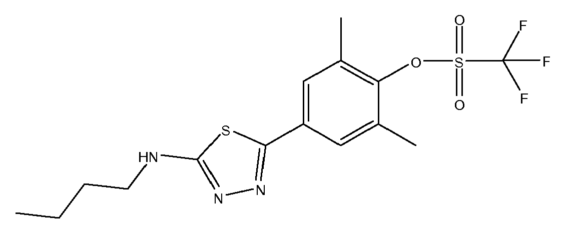

To a stirred suspension of the title product of Preparation 29 (7.20 g, 25.96 mmol) in dichloromethane (140 ml) and tetrahydrofuran (35 ml ) triethylamine (6.40 ml, 45.92 mmol) was added and the mixture was stirred at rt for 30 minutes. N-Phenytrifluoromethane sulfonimide (11.80 g, 33.03 mmol) was added and the mixture stirred at reflux for 70 hours.

The solvent was evaporated and the residue was portioned between NaOH 1 N and diethyl ether. The organic layer was washed with NaOH 1 N (twice), water, HCI 2M and neutralized with NaOH to pH 7. Then the organic layer was washed with brine, dried and solvent removed. The solid thus obtained was triturated with diisopropyl ether to give the title product. (100% yield).
LRMS: m/z 2410 (M+1)⁺
Retention time: 7.47 min (method B)
¹H NMR (200 MHz, DMSO-D6) δ ppm 0.92 (t, *J*=7.23 Hz, 3 H) 1.31 - 1.43 (m, 2 H) 1.58 (quin, *J*=7.23 Hz, 2 H) 2.39 (s, 6 H) 3.25 - 3.36 (m, 2 H) 7.68 (s, 2 H) 8.05 (t, *J*=5.28 Hz, 1 H).

### PREPARATION 35

### 4-(5-(N-butyl-2-chlorobenzamido)-1,3,4-thiadiazol-2-yl)-2,6-dimethylphenyl trifluoromethanesulfonate

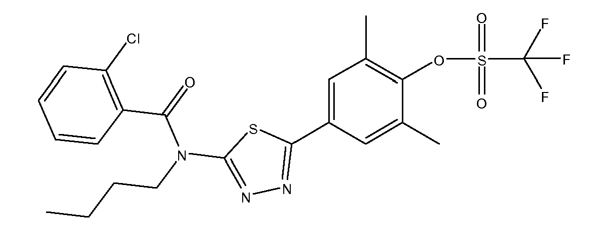

Obtained (30% yield) from the title compound of Preparation 34 and 2-chlorobenzoyl chloride following the experimental procedure of Preparation 5.
LRMS: m/z 548 (M+1)⁺
Retention time: 7.98 min (method B)

### PREPARATION 36

### N-butyl-2-chloro-N-(5-(3,5-dimethyl-4-vinylphenyl)-1,3,4-thiadiazol-2-yl)benzamide

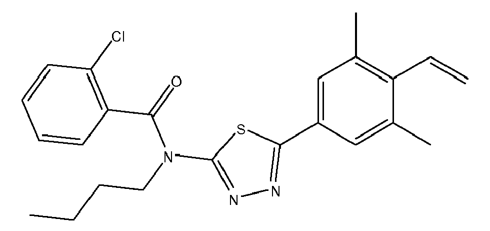

The title compound of Preparation 35 (800 mg, 1.46 mmol) and lithium chloride (185 mg, 4.36 mmol) were dissolved in DMF (15 ml). The mixture was purged (vacuum-argon three times) and tributyl (vinyl) stannane (0.643 ml, 2.19 mmol) was added and the mixture purged again (vacuum-argon three times). The mixture was stirred at 90 °C for 2 hours.

The reaction mixture was cooled to room temperature, diluted with water and extracted three times with ethyl acetate. The combined organics were washed with water and brine, and dried. The solvent was removed in vacuum and the residue was purified according to purification method A. 420 mg (67% yield) of the title product were obtained.
LRMS: m/z 426 (M+1)⁺
Retention time: 7.95 min (method B)

### PREPARATION 37

### N-butyl-2-chloro-N-(5-(4-formyl-3,5-dimethylphenyl)-1,3,4-thiadiazol-2-yl) benzamide

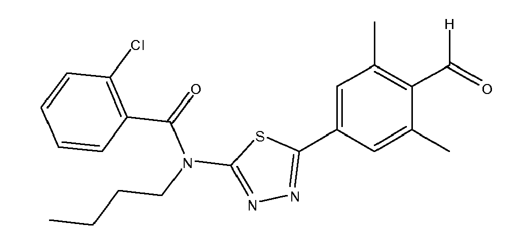

Obtained (90% yield) from the title compound of Preparation 36 following the experimental procedure of Preparation 21.
LRMS: m/z 428 (M+1)⁺
Retention time: 7.58 min (method B)

### PREPARATION 38

### 1-(tert-butoxycarbonyl)-4-(ethoxycarbonyl)piperidine-4-carboxylic acid

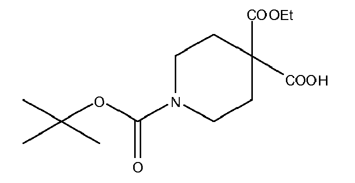

Lithium diisopropylamide (2.14 ml, 4.28mmol) was added to a solution of N,N,N',N'-tetranethylethylenediamine (1.13 g, 9.72mmol) and piperidine-1,4-dicarboxylic acid 1-tert.butyl 4-ethyl ester (1.0 g, 3.89 mmol) in dry tetrahydrofuran (30 ml) at -78°C. After stirring for 30 minutes the temperature was raised to -40°C. The reaction mixture is again cooled to -78°C and carbon dioxide gas was bubbled through it. The reaction mixture is slowly allowed to come to 0°C, and then stirred at rt for 3 hours. Saturated aqueous ammonium chloride solution (10 ml) was added and the organic layer was separated and concentrated. The residue was treated with an aqueous sodium hydrogen carbonate solution. The aqueous layer was washed with n-hexane, then acidified to pH=5 with 2N HCI and extracted with ethyl acetate, Combined organic layers were dried and concentrated to give 460 mg (31 % yield) of the title product .
¹H NMR (400 MHz, DMSO-D6) δ ppm 1.15 (t, *J*=7.03 Hz, 3 H) 1.36 (s, 9 H) 1.75 - 1.94 (m, 4 H) 3.13 - 3.25 (m, 2 H) 3.31 - 3.45 (m, 2 H) 4.11 (q, *J*=7.03 Hz, 2 H).

### PREPARATION 39

### 4-(ethoxycarbonyl)piperidine-4-carboxylic acid

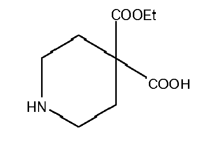

To a stirred solution of the title compound of preparation 38 (350 mg, 1.16 mmol) 5 ml of HCI 4M in dioxane were added and the mixture was stirred at rt for 4 hours. Solvent was removed *in vaccuo* and the title compound obtained (37% yield).
¹H NMR (400 MHz,DMSO-D6) δ ppm 1.16 (t, *J*=7.03 Hz, 3 H) 2.11 (t, *J*=5.28 Hz, 4 H) 2.93 - 3.12 (m, 4 H) 4.14 (q, *J*=7.03 Hz, 2 H) 8.84 (br. s., 2 H)

### PREPARATION 40

### 1-buty)-3-(2-chloropyridin-3-y)-1-(5-(4-formylpheny)-1,3,4-thiadiazol-2-yl)urea

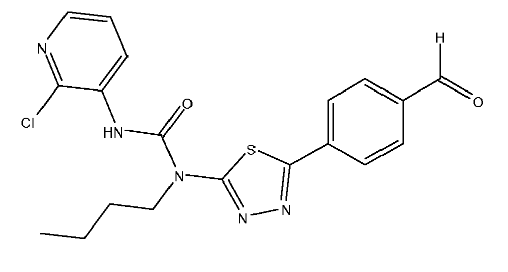

To a stirred solution of the title compound of Preparation 21 (500 mg, 1.91 mmol) in THF (15 ml), N,N-Diisopropylethyl amine ( 4.33 ml, 24.86 mmol) was added and the mixture was placed under nitrogen atmosphere, cooled at 0°C and stirred for 15 minutes. Triphosgene (283.9 mg, 0.96 mmol) was added and after 15 minutes of stirring 2-chloropyridin-3-amine (270.56 mg, 2.10 mmol) was added. The mixture was stirred at room temperature overnight. Water and dichloromethane were added and the organic layer separated, washed with water, dried and solvent removed. The crude obtained was purified according to purification method A to yield the title compound (47% yield).
LRMS: m/z 416 (M+1)⁺
Retention time: 6.85 min (method B)

### PREPARATION 41

### 1-butyl-3-(4-fluorophenyl)-1-(5-(4-formylphenyl)-1,3,4-thiadiazol-2-yl)urea

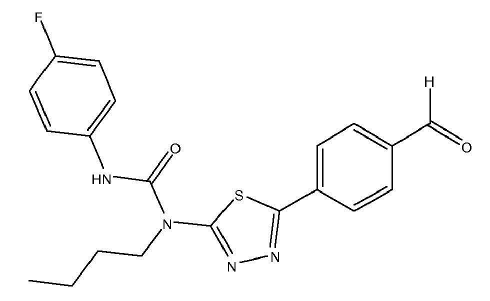

Obtained (98% yield) from the title compound of Preparation 21 and 4-fluoroaniline following the experimental procedure of Preparation 40.
LRMS: m/z 399 (M+1)⁺
Retention time: 7.13 min (method B)

### PREPARATION 42

### 1-butyl-1-(5-(4-formylphenyl)-1,3,4-thiadiazol-2-yl)-3-phenylurea

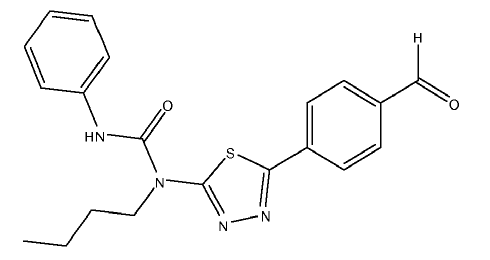

Obtained (43% yield) from the title compound of Preparation 21 and 4-fluoroaniline following the experimental procedure of Preparation 40.
LRMS: m/z 381 (M+1)⁺
Retention time: 7.12 min (method B)

### PREPARATION 43

### N-(5-(4-(allyloxy)-3,5-dimethylphenyl)-1,3,4-thiadiazol-2-yl)-N-butyl-2-methylbenzamide

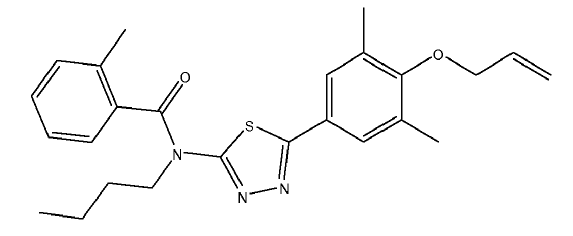

To a 0°C stirred solution of the title compound of Preparation 4 (1g, 3.15 mmol) in THF (30 ml), 60% NaH (265 mg, 6.63 mmol) was added and the mixture was stirred at that temperature for 30 min. Then a solution of 2-methybenzolyl chloride (905 µl, 6.94 mmol) in THF (24 ml) was added drop wise and the final mixture was stirred at rt for 1.5 h. 2N HCI (50 ml) was slowly added and the final mixture was portioned between water and ethyl acetate. The aqueous layer was extracted with ethyl acetate and the combined organic layers were washed with water and brine. It was dried and solvent removed to yield a crude product that was purified on a Biotage 40S chromatography column of silica gel using hexane/diethyl ether (8:2) as eluent. 1,46 g (88% purity. 94% yield) of the title product were obtained as solid.
LRMS: m/z 436 (M+1)⁺
Retention time: 4.05min (method B)

### PREPARATION 44

### N-butyl-N-(5-(3,5-dimethyl-4-(2-oxoethoxy)phenyl)-1,3,4-thiadiazol-2-yl)-2-methylbenzamide

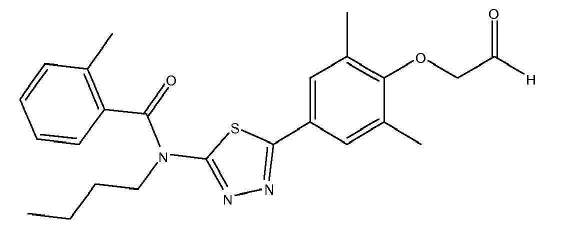

To a stirred solution of the title compound of Preparation 43 (1.46g, 89% purity, 2,97 mmol) in THF (35 ml) and tert-butanol (5ml), osmium tetroxide (4% aqueous solution, 0.37 ml, 0.06 mmol) and 4-methylmorpholine 4-oxide (0.70 g, 5.98 mmol) were added and the mixture was stirred at rt overnight. Solvent was then removed and the solid thus obtained was portioned between water and ethyl acetate. The organic layer was washed with water and brine, dried and solvent removed to yield a solid that was solved with methanol (25 ml) and water (2.6 ml). Sodium periodate (1.01 g, 4.72 mmol) was added and the mixture was stirred at room temperature for 2h. Solvent was removed and the solid thus obtained was partitioned between water and ethyl acetate. The organic layer was washed with water and brine. Solvent was removed to yield the title compound (1.16 g, 89% yield).
LRMS: m/z 470 (M+CH3OH+1)⁺
Retention time: 7.23min (method B)

### PREPARATION 45

### N-(5-(4-(allyloxy)phenyl)-1,3,4-thiadiazol-2-yl)-N-butyl-2-chlorobenzamide

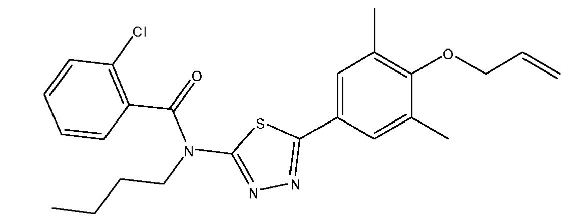

To a 0°C stirred solution of the title compound of Preparation 4 (2g, 6.30 mmol) in THF (20 ml), 60% NaH (530 mg, 12.25 mmol) was added and the mixture was stirred at that temperature for 30 min. Then a solution of 2-chlorobenzoyl chloride (2.43 g, 13.88 mmol) in THF (16 ml) was added drop wise and the final mixture was stirred at rt for 1.5 h. 2N HCI (50 ml) was slowly added and the final mixture was portioned between water and ethyl acetate. The aqueous layer was extracted with ethyl acetate and the combined organic layers were washed with water and brine. It was dried and solvent removed to yield a crude product that was purified on a Biotage 40S chromatography column of silica gel using hexane/ethyl acetate (8:2) as eluent. 2.50 g (83% purity. 72% yield) of the title product were obtained as a solid.
LRMS: m/z 456 (M+1)⁺
Retention time: 7.85 min (method B)
¹H NMR (200 MHz, DMSO-*d*₆) δ ppm 0.69 (t, *J*=7.42 Hz, 3 H) 1.10 - 1.26 (m, 2 H) 1.53 - 1.80 (m, 2 H) 2.31 (s, 6 H) 3.67 - 3.95 (m, 2 H) 4.39 (d, *J*=5.47 Hz, 2 H) 5.37 (dd, 2 H) 6.01 - 6.25 (m, 1 H) 7.50 - 7.85 (m, 6 H)

### PREPARATION 46

### N-butyl-2-chloro-N-(5-(3,5-dimethyl-4-(2-oxoethoxy)phenyl)-1,3,4-thiadiazol-2-yl)benzamide

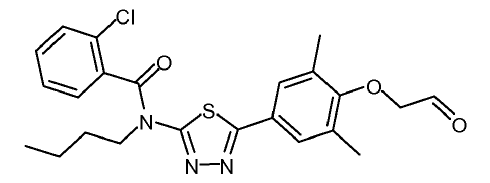

To a stirred solution of the title compound of Preparation 45 (2.50, 83% purity, 4.55 mmol) in THF (60 ml) and tert-butanol (8 ml), osmium tetroxide (4% aqueous solution, 0.56 ml, 0.09 mmol) and 4-methylmorpholine 4-oxide (1.07 g, 9.13 mmol) were added and the mixture was stirred at room temperature overnight. Solvent was then removed and the solid thus obtained was portioned between water and ethyl acetate. The organic layer was washed with water and brine, dried and solvent removed to yield a solid that was solved with methanol (70 ml) and water (8 ml). Sodium periodate (1.56 g, 7.29 mmol) was added and the mixture was stirred at room temperature for 2h. Solvent was removed and the solid thus obtained was partitioned between water and ethyl acetate. The organic layer was washed with water and brine. Solvent was removed to yield the title compound (2.28 g, 100% yield).
LRMS: m/z 490 (M+CH3OH+1)⁺
Retention time: 7.22 min (method B)
¹H NMR (300 MHz, DMSO-*d*₆) δ ppm 0.69 (t, *J*=7.28 Hz, 3 H) 1.12 - 1.23 (m, 2 H) 1.55 - 1.79 (m, 2 H) 2.32 (s, 6 H) 3.70 - 3.93 (m, 2 H) 6.41 (d, *J*=7.69 Hz, 2 H) 7.51 - 7.81 (m, 6 H) 9.73 (s, 1 H)

### PREPARATION 47

### N-(5-(4-(allyloxy)-3,5-dimethylphenyl)-1,3,4-thiadiazol-2-yl)-N-butyl-3-chloro-2-fluorobenzamide

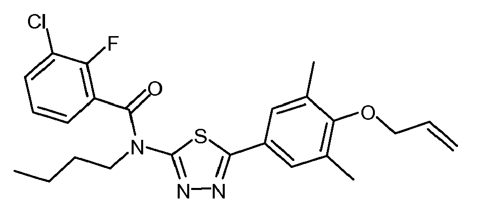

To a 0°C stirred solution of the title compound of Preparation 4 (600 mg, 1.89 mmol) in THF (5 ml), 60% NaH (170 mg, 4.25 mmol) was added and the mixture was stirred at that temperature for 30 min. Then a solution of 3-chloro-2-fluorobenzoyl chloride (550 mg, 2.85 mmol) in THF (3 ml) was added drop wise and the final mixture was stirred at room temepature for 1.5 h. 2N HCl (10 ml) was slowly added and the final mixture was portioned between water and ethyl acetate. The aqueous layer was extracted with ethyl acetate and the combined organic layers were washed with water and brine. It was dried and solvent removed to yield a crude product that was purified on a Biotage 40S chromatography column of silica gel using hexane/diethyl ether (8:2) as eluent. 780 mg (83% purity. 72% yield) of the title product were obtained as an oil.
LRMS: m/z 474 (M+1)⁺
Retention time: 7.95 min (method B)

### PREPARATION 48

### N-butyl-3-chloro-N-(5-(3,5-dimethyl-4-(2-oxoethoxy)phenyl)-1,3,4-thiadiazol-2-yl)-2-fluorobenzamide

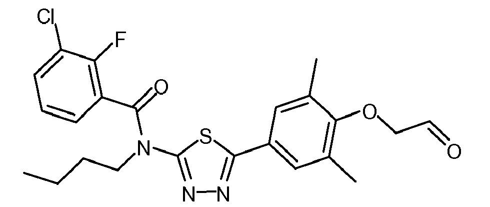

To a stirred solution of the title compound of Preparation 47 (780 mg, 83% purity, 1.36 mmol) in THF (16 ml) and tert-butanol (2.5 ml), osmium tetroxide (4% aqueous solution, 0.17 ml, 0.03 mmol) and 4-methylmorpholine 4-oxide (319 mg, 2.72 mmol) were added and the mixture was stirred at rt overnight. Solvent was then removed and the solid thus obtained was portioned between water and ethyl acetate. The organic layer was washed with water and brine, dried and solvent removed to yield a solid that was solved with methanol (22 ml) and water (2.5 ml). Sodium periodate (465 mg, 2.17 mmol) was added and the mixture was stirred at room temeprature for 2h. Solvent was removed and the solid thus obtained was partitioned between water and ethyl acetate. The organic layer was washed with water and brine. Solvent was removed to yield the title compound (611 mg, 95% yield).
LRMS: m/z 508 (M+CH30H+1)⁺
Retention time: 7.10 min (method B)

### PREPARATION 49

### 4-(Allyloxy)-benzonitrile

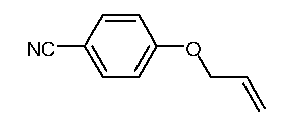

To a 0°C stirred solution of 4-hydroxybenzonitrile (3 g, 25.2 mmol) in DMF (30ml), 60% NaH (1.11 g, 27.70 mmol) was added portion wise and the final mixture was stirred at that temperature for 30 min. Then a solution of 3-bromoprop-1-ene (2.34 ml, 27.66 mmol) in DMF (25ml) was added drop wise and the mixture was stirred at room temeprature for 5h. The reaction crude was slowly poured onto ice/water to yield a solid that was filtered, washed with water and dried. 2.76 g (69% yield) of the title compound were obtained.
LRMS: m/z 160 (M+1)⁺
Retention time: 5.78 min (method B)

### PREPARATION 50

### 4-(allyloxy)-benzoic acid

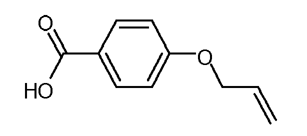

In a sealed tube the title compound of preparation 49 (2.76 g, 17.34 mmol) was suspended in 8 M aqueous NaOH solution (17 ml). Ethanol was added (1ml) and the mixture was stirred at 110°C for 5h and then at rt overnight. Dichloromethane and water were added to the mixture and it was acidified with 6 M HCl to pH 1. Layers were separated and the aqueous one was extracted with dichloromethane twice. The combined organic layers were washed with water and brine, dried and solvent was finally removed in vacuum. The title compound was obtained as a yellow solid (2.62 g, 85% yield).
LRMS: m/z 179 (M+1)⁺
Retention time: 5.42 min (method B)

### PREPARATION 51

### 5-(4-(Allyloxy)-phenyl)-N-butyl-1,3,4-thiadiazol-2-amine

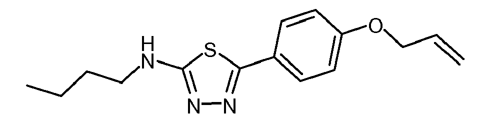

To a stirred solution of the title compound of Preparation 50 (2.62 g, 14.70 mmol) in dioxane (30 ml), the title compound of preparation 3 (1.97 g, 13.38 mmol) was added portion wise. POCl₃ (1.84 ml, 20.10 mmol) was added drop wise and the final mixture was stirred at 80°C for 5h and then at rt overnight. Solvent was removed in vacuum and the solid thus obtained was partitioned between water and dichloromethane. It was basified to pH 8-9 and the organic layer was washed with brine. It was dried and solvent was removed in vacuum to yield a solid that was washed with hexane and ethyl ether. 2.07 g (54% yield) of the title compound were obtained.
LRMS: m/z 290 (M+1)⁺
Retention time: 6.65 min (method B)

### PREPARATION 52

### N-(5-(4-(Allyloxy)-phenyl)-1,3,4-thiadiazol-2-yl)-N-butyl-2-fluorobenzamide

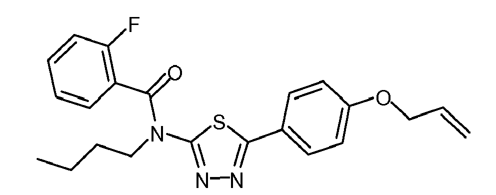

To a 0°C stirred solution of the title compound of Preparation 51 (2.07 g, 7.15 mmol) in THF (24 ml), 60% NaH (573 mg, 14.33 mmol) was added and the mixture was stirred at that temperature for 30 min. Then a solution of 2-fluorobenzolyl chloride (1.70 ml, 14.26 mmol) in THF (10 ml) was added drop wise and the final mixture was stirred at room temperature for 1.5 h. 2N HCl (50 ml) was slowly added and the final mixture was portioned between water and ethyl acetate. The aqueous layer was extracted with ethyl acetate and the combined organic layers were washed with water and brine. It was dried and solvent removed to yield a crude product that was purified according to purification method A to yield the title compound as a solid. 2.90 g (99% yield) of the title product were obtained as a pale oil.
LRMS: m/z 412 (M+1)⁺
Retention time: 750 min (method B)

### PREPARATION 53

### N-butyl-N-(5-(4-(2-oxoethoxy)phenyl)-1,3,4-thiadiazol-2-yl)-2-fluorobenzamide

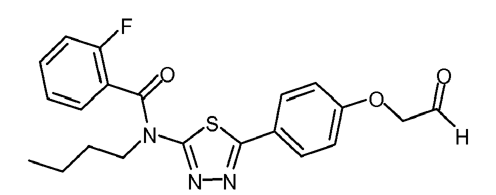

To a stirred solution of the title compound of Preparation 52 (2.90 g, 7.05 mmol) in THF (71 ml) and tert-butanol (10 ml), osmium tetroxide (4% aqueous solution, 0.10 ml, 1.5 mmol) and N-methylmorpholine-4-oxide (1.66 g, 16.39 mmol) were added and the mixture was stirred at rt overnight. Solvent was then removed and the solid thus obtained was portioned between water and ethyl acetate. The organic layer was washed with water and brine, dried and solvent removed to yield a solid that was solved with methanol (71 ml) and water (8.8 ml). Sodium periodate (2.42 g, 11.29 mmol) was added and the mixture was stirred at rt for 2h. Solvent was removed and the solid thus obtained was partitioned between water and ethyl acetate. The organic layer was washed with water and brine. Solvent was removed to yield the title compound (740 mg, 25% yield).
LRMS: m/z 446 (M+CH₃OH+1)⁺
Retention time: 4.50 min (method B)

### PREPARATION 54

### 2-(4-(5-(butylamino)-1,3,4-thiadiazol-2-yl)-2,6-dimethylphenoxy)acetaldehyde

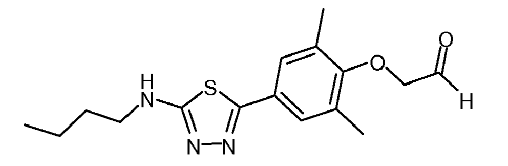

To a stirred solution of the title compound of Preparation 4 (1.00 g, 3.1 mmol) in THF (20 ml) and tert-butanol (3 ml), osmium tetroxide (4% aqueous solution, 0.40 ml, 0.07 mmol) and N-methylmorpholine-4-oxide (0.74 g, 6.32 mmol) were added and the mixture was stirred at rt overnight. Solvent was then removed and the solid thus obtained was portioned between water and ethyl acetate. The organic layer was washed with water and brine, dried and solvent removed to yield a solid that was solved with methanol (25 ml) and water (2.6 ml). Sodium periodate (1.09 g, 5.10 mmol) was added and the mixture was stirred at room temperature for 2h. Solvent was removed and the solid thus obtained was partitioned between water and ethyl acetate. The organic layer was washed with water and brine. Solvent was removed to yield the title compound (1.01 mg, 100% yield).
LRMS: m/z 352 (M+CH3OH+1)⁺
Retention time: 5.95 min (method B)

### PREPARATION 55

### 1-[2-(4-{5-[Butylamino]-1,3,4-thiadiazol-2-yl}-2,6-dimethylphenoxy)ethyl]piperidin-4-carboxylic acid

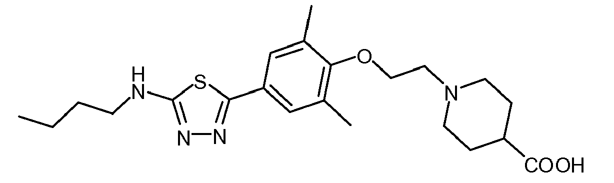

To a stirred solution of the title compound of Preparation 54 (367 g, 1149 mmol) and isonipecotic acid (1.80 g, 13.94 mmol) in methanol (200ml), AcOH (6.2 ml, 108 mmol) was added and the mixture was stirred at room temperature for 1h. Then, NaCNBH₃ (361 mg, 5.74 mmol) was added portion wise and the final mixture was stirred at rt for 2-3 h. Solvent as removed in vacuum and the crude mixture was partitioned between water and ethyl acetate. The aqueous layer was let on the fridge (-20°) and a precipitate separated. After filtering, the title compound was obtained as a solid (yield=40.8%).
LRMS: m/z 433 (M+1)⁺
Retention time: 4.57 min (method B)

### PREPARATION 56

### N-Butyl-5-(4-methoxy-3,5-dimethylphenyl)-1,3,4-thiadiazol-2-amine

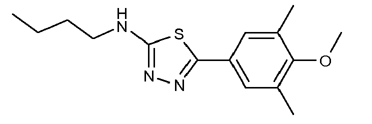

To a stirred suspension of 4-methoxy-3,5-dimethylbenzoic acid (3.0 g, 16.65 mmol) in dioxane (28 ml), POCl₃ (3.04 ml, 33.31 mmol) was added drop wise and the final mixture was stirred at 90°C for 1 h. After cooling the reaction mixture, N-butylhydrazinecarbothioamide (Preparation 3) (2.45 g, 16.65 mmol) was added and the mixture was let to react at 90° overnight. The mixture was let to cool down and was carefully poured onto ice water. The solid thus obtained was filtered and suspended in water. It was basified to pH 12 and the solution thus formed was neutralized. The solid thus formed was filtered and thoroughly washed with water and hexanes to yield 4.50 g (93%) of the title compound.
LRMS: m/z 292(M+1)⁺
Retention time: 6.67min (method B)
¹H NMR (200 MHz, DMSO-*d*₆) δ ppm 0.92 (t, *J*=7.22 Hz, 3 H) 1.39 (dq, *J*=14.54, 7.13 Hz, 2 H) 2.28 (m, 2 H) 3.38 (t, *J*=6.64 Hz, 2 H) 3.70 (s, 3 H) 4.58 - 5.60 (m, 6 H) 7.48 (s, 2 H).

### PREPARATION 57

### 4-(5-(Butylamino)-1,3,4-thiadiazol-2-yl)-2,6-dimethylphenol

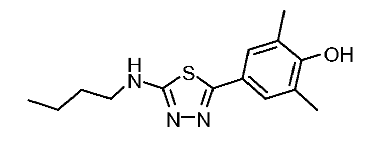

To a stirred suspension of the title product of Preparation 56 (2.0 g, 6.86 mmol) in dichloromethane (20 ml) at -78°C, a 1 M BBr₃ solution in dichloromethane (10.3 ml, 10.3 mmol) was added and the mixture was stirred at that temperature for 30 min and then at room temperature overnight. The reaction mixture was poured onto ice water, diluted with dichloromethane and neutralized with saturated solution of potassium bicarbonate. The organic layer was collected and solvent was removed to yield a solid that was washed with ethyl ether. 1.64 g (87% yield) of the title product were obtained.
LRMS: m/z 278 (M+1)⁺
Retention time: 6.09min (method B)
¹H NMR (200 MHz, DMSO-D6) δ ppm 0.9 (t, *J*=7.2 Hz, 3 H) 1.4 (m, 2 H) 1.6 (m, 2 H) 2.2 (s, 6 H) 3.3 (m, 2 H) 7.3 (s, 2 H) 8.0 (s, 1 H) 8.8 (s, 1 H)

### PREPARATION 58

### tert-butyl 4-((4-(5-(butylamino)-1,3,4-thiadiazol-2-yl)-2,6-dimethylphenoxy)-methyl)piperidine-1-carboxylate

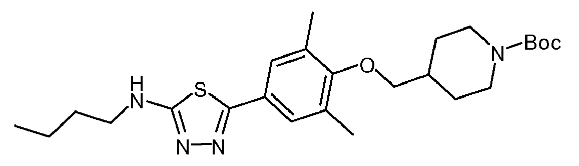

The title product of Preparation 57 ( 500 mg, 1.80 mmol), tert-butyl 4-(hydroxymethyl)piperidine-1-carboxylate (853 mg, 3.96 mmo), triphenylphosphine (1.04 g, 3.97 mmol) and DIEA (1.26 ml, 7.23 mmol) were mixed under Ar atmosphere followed by the addition of dry THF (15 ml). The mixture was cooled at 0° and Diisopropyl azodicarboxylate (DIAD) was added drop wise. The reaction mixture was allowed to warm to room temperature and then stirred overnight. Solvent was removed and the residue thus obtained was partitioned between water and ethyl acetate. The organic layer was washed with water and brine.

It was dried and solvent removed to yield a crude product that was purified according to purification method A to yield the title compound as a solid. 590 mg (69% yield) of the title product were obtained as a pale oil.
LRMS: m/z 475 (M+1)⁺
Retention time: 7.67 min (method B)
¹H NMR (200 MHz, DMSO-D6) δ ppm 0.91 (t, *J*=7.42 Hz, 3 H) 1.15 - 1.31 (m, 2 H) 1.30 - 1.49 (m+s, 11 H) 1.51 - 1.62 (m, 2 H) 1.75 - 1.87 (m, 2 H) 1.89 - 2.03 (m, 1 H) 2.25 (s, 6 H) 2.63 - 2.91 (m, 2 H) 3.23 - 3.32 (m, 2 H) 3.61 (d, *J*=6.25 Hz, 2 H) 3.91 - 4.10 (m, 2 H) 7.41 (s, 2 H) 7.87 (t, *J*=5.28 Hz, 1 H)

### PREPARATION 59

### Methyl 4-(5-(butylamino)-1,3,4-thiadiazol-2-yl)benzoate

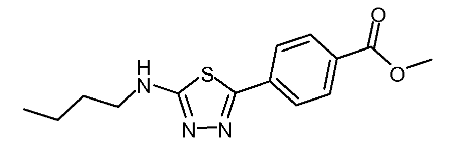

To a stirred solution of the 4-(methoxycarbonyl)benzoic acid (5 g, 27.17 mmol, Aldrich) in dioxane (40 ml), POCl₃ (5.2 ml, 55.96 mmol) was added drop wise and the mixture was stirred at rt for 30min. Then, the title compound of Preparation 3 (4 g, 27.75 mmol) was added portion wise and then let to react at 80° overnight. Solvent was removed in vacuum and the solid thus obtained was suspended in water, then it was basified to pH 8-9 and filtered. The precipitate thus obtained was dried in vacuum to yield a solid. 6.77 g (81 % yield) of the title compound were obtained.
LRMS: m/z 292 (M+1)⁺
Retention time: 3.26 min (method A)
¹H NMR (200 MHz, CDCl₃) δ ppm 0.92 (t, *J*=7.03 Hz, 3 H) 1.27 - 1.50 (m, 2 H) 1.50 - 1.73 (m, 2 H) 3.24 - 3.46 (m, 2 H) 3.88 (s, 3 H) 7.85 - 7.97 (m, 2 H) 8.03 (m, 2 H) 8.13 (t, *J*=5.08 Hz, 1 H)

### PREPARATION 60

### Methyl 4-(5-(N-butyl-2-fluorobenzamido)-1,3,4-thiadiazol-2-yl)benzoate

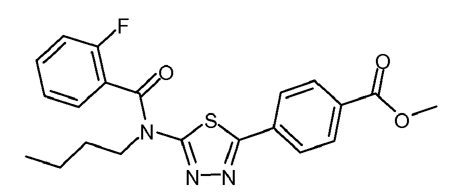

To a 0°C stirred solution of the title compound of Preparation 59 (135 g, 5.15 mmol) in THF (15 ml), 60% NaH (375 mg, 9.38 mmol) was added and the mixture was stirred at that temperature for 30 min. Then a solution of 2-fluorobenzolyl chloride (1.49 g, 9.40 mmol) in THF (12 ml) was added drop wise and the final mixture was stirred at rt for 1.5 h. 2N HCl (50 ml) was slowly added and the final mixture was portioned between water and ethyl acetate. The aqueous layer was extracted with ethyl acetate and the combined organic layers were washed with water and brine. It was dried and solvent removed to yield a crude product that was purified on a Biotage 40S chromatography column of silica gel using hexane/ethyl acetate (95:5) as eluent. 1.44 g (68% yield) of the title product were obtained as a pale oil.
LRMS: m/z 414 (M+1)⁺
Retention time: 7.30 min (method B)
¹H NMR (200 MHz, CDCl₃) δ ppm 0.78 (t, *J*=7.22 Hz, 3 H) 1.08 - 1.34 (m, 2 H) 1.63 - 1.86 (m, 2 H) 3.96 (s, 3 H) 4.09 - 4.29 (m, 2 H) 7.16 - 7.39 (m, 2 H) 7.41 - 7.63 (m, 2 H) 7.95 - 8.31 (m, *J*=8.46, 8.46, 8.46 Hz, 4 H)

### PREPARATION 61

### 4-(5-(N-butyl-2-fluorobenzamido)-1,3,4-thiadiazol-2-yl)benzoic acid

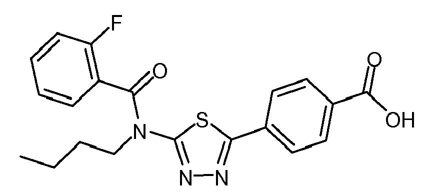

A suspension of the title compound of Preparation 60 (600 mg, 1.45 mmol) in HCl 37% (100 ml) was heated at 50° during 5h. The precipitate was filtered, washed and dried in vacuum. The title compound 0.51 g (83% yield) was obtained as a white solid.
LRMS: m/z 400 (M+1)⁺
Retention time: 6.91 min (method B)
¹H NMR (200 MHz, DMSO-d⁶) δ ppm 0.70 (t, *J*=7.22 Hz, 3 H) 1.00 - 1.33 (m, 2 H) 1.47 - 1.82 (m, 2 H) 3.96 - 4.24 (m, 2 H) 7.27 - 7.60 (m, 2 H) 7.57 - 7.92 (m, 2 H) 7.97 - 8.37 (m, 4 H)

### PREPARATION 62

### (4-(5-(butylamino)-1,3,4-thiadiazol-2-yl)phenyl)metanol

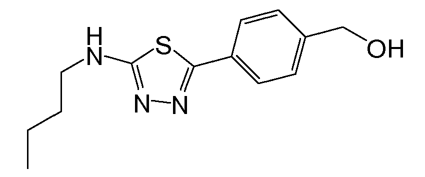

The title compound of preparation 59 (1.5g, 5.15mmol) was dissolved in THF (20ml) and LiAlH₄ (0.2g, 5.27mmol) was added portion wise and the mixture stirred overnight at room temperature. Then to the mixture was added water (2ml), NaOH 32% (4ml), water (4ml) and finally ethyl acetate. The organic layer was collected, dried and concentrated to yield the title compound as a yellow solid (75%).
LRMS: m/z 264 (M+1)⁺
Retention time: 2.75min (method A)
1H NMR (200 MHz, CDCl₃) δ ppm 0.97 (t, *J*=7.22 Hz, 3 H) 1.45 (td, *J*=14.74, 7.22 Hz, 2 H) 1.60 - 1.83 (m, 2 H) 3.37 (t, *J*=7.03 Hz, 2 H) 4.74 (s, 2 H) 5.72 (br. s., 1 H) 7.42 (d, *J*=8.20 Hz, 2 H) 7.78 (d, *J*=8.59 Hz, 2 H)

### PREPARATION 63

### 4-(5-(butylamino)-1,3,4-thiadiazol-2-yl)benzaldehyde

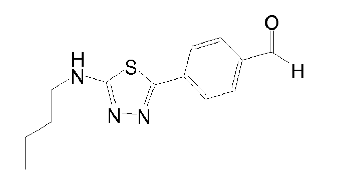

To a solution of oxalyl chloride (0.73ml, 8.35mmol) in dichloromethane (20ml) under argon at --60°C, DMSO (0.89g, 11.39mmol) was slowly added keeping the temperature at -60°C and the mixture stirred at this temperature for 15min. A suspension of the title compound of preparation 62 (1g, 3.8mmol) in 10ml of DCM was slowly added to this mixture. Finally, diisopropylethylamine was added (4.40ml, 25.3mmol) and the mixture stirred at -60°C for 1h and at room temperature overnight. Solvent was removed and the residue was solved in ethyl acetate and washed with a 4% solution of NaHCO₃. The organic layer was dried, solvent was removed in vacuum and the crude was purified according to purification method A to yield the title compound as a solid (yield=18%).
LRMS: m/z 262 (M+1)⁺
Retention time: 5.87min (method B)
¹H NMR (200 MHz, CDCl₃) δ ppm 0.98 (t, *J*=7.22 Hz, 3 H) 1.46 (dd, *J*=15.03, 7.22 Hz, 2 H) 1.72 (t, *J*=3.51 Hz, 2 H) 3.41 (t, *J*=7.03 Hz, 2 H) 7.85 - 8.05 (m, 4 H) 10.04 (s, 1H)

### PREPARATION 64

### N-butyl-2-fluoro-N-(5-(4-formylphenyl)-1,3,4-thiadiazol-2-yl)benzamide

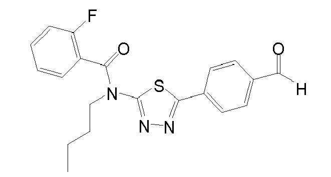

To a stirred suspension of the title compound of Preparation 63 (2.0 g, 7.65 mmol) and diisopropylethylamine (5.33 ml, 30.60 mmol) in DCM (70 ml), 2-fluorobenzoyl chloride (1.13 g, 9.53 mmol) was added and the mixture was stirred at 80°C for 4 h and then at rt overnight. Solvent was removed and the mixture was partitioned between water and ethyl acetate. The organic layer was washed with HCl 2N, with potassium carbonate and brine. Solvent was removed and the crude thus obtained was purified according to purification method A to yield the title compound as a solid (2.50 g; 85%).
LRMS: m/z 384(M+1)⁺
Retention time: 7.09min (method B)

### PREPARATION 65

### 1-(4-(5-(N-butyl-2-fluorobenzamido)-1,3,4-thiadiazol-2-yl)benzyl)azetidine-3-carboxylic acid

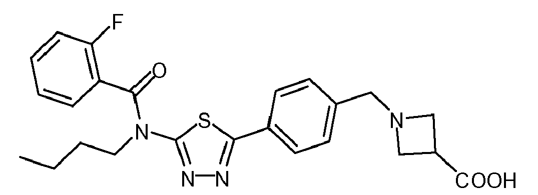

To a stirred suspension N-butyl-2-fluoro-N-(5-(4-formylphenyl)-1,3,4-thiadiazol-2-yl)benzamide (Preparation 24) (2.5 g, 6.52 mmol) and azetidine-3-carboxylic acid (726 mg, 7.18 mmol) in methanol (32ml), AcOH (1.5 ml, 26.23 mmol) was added and the mixture was stirred at rt for 1h. Then, NaCNBH3 (451 mg, 7.18 mmol) was added portion wise and the final mixture was stirred at room temperature overnight. Acetic acid was added to the mixture until pH 3-4 and solvent was removed in vacuum. The crude mixture thus obtained was purified according to purification method A to yield the title compound as a solid (yield=87%).
LRMS: m/z 469 (M+1)⁺
Retention time: 5.60 min (method C)

### PREPARATION 66

### tert-butyl 2-(methylsulfonamido)ethylcarbamate

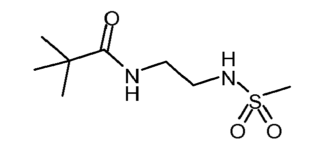

To a stirred solution of tert-butyl 2-aminoethylcarbamate (1 g, 6.24 mmol; Aldrich) and TEA (0.96 ml, 6.86 mmol) in DCM (20 ml) at 0°, was added methanesulfonyl chloride (0.48 ml, 6.24 mmol) drop wise and let to react overnight at room temeprature. The crude mixture was diluted with ethyl acetate and the organic phase was washed with water (x3) and brine (x1). The organic layer was dried, solvent was removed in vacuum and the title compound (1.22 g) was obtained as oil that slowly crystallized. (yield=82%).
LRMS: m/z 237 (M+1)⁺
Retention time: 4.47 min (method B)
¹H NMR (200 MHz, CDCl₃) δ ppm 1.44 (s, 9 H) 2.96 (s, 3 H) 3.14 - 3.42 (m, 4 H) 4.93 - 5.08 (m, 1 H) 5.09 - 5.27 (m, 1 H)

### PREPARATION 67

### N-(2-aminoethyl)methanesulfonamide

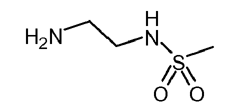

The compound from preparation 66 (1.2 g, 5.12 mmol) was dissolved in HCl-dioxane 4N (15 ml) and let to react at room temperature overight. Solvent was removed to yield the title compound 1.01 g (quantitative yield) as gum.
¹H NMR (200 MHz, DMSO-*d*₆) δ ppm 2.96 (s, 3 H) 3.13 - 3.29 (m, 2 H) 3.29 - 3.47 (m, 2 H) 7.27 - 7.57 (m, 1 H) 8.17 (br. s., 3 H)

### PREPARATION 68

### 5-(4-(bromomethyl)phenyl)-N-butyl-1,3,4-thiadiazol-2-amine

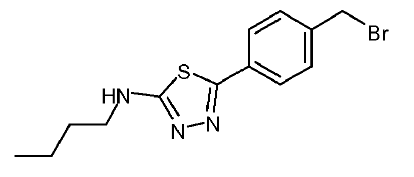

To a stirred solution of the title compound of Preparation 3 (1.00 g, 6.79 mmol) in dioxane (15 ml) and 4-(bromomethyl)benzoic acid (1.30 g; 6.05 mmol. Aldrich) was added portion wise. POCl₃ (3.20 ml, 20.87 mmol) was added drop wise and the final mixture was stirred at 80°C for 2h and then at room temperature overnight. Solvent was removed in vacuum and the solid thus obtained was partitioned between water and dichloromethane. It was basified to pH 8-9 and the organic layer was washed with brine. It was dried and solvent was removed in vacuum to yield a solid that was washed with hexane and ethyl ether. 0.7 g (23% yield) of the title compound were obtained.
LRMS: m/z 326 (M+1)⁺
Retention time: 6.54 min (method B)

### PREPARATION 69

### ethyl 1-(4-(5-(butylamino)-1,3,4-thiadiazol-2-yl)benzyl)-1H-pyrazole-4-carboxylate

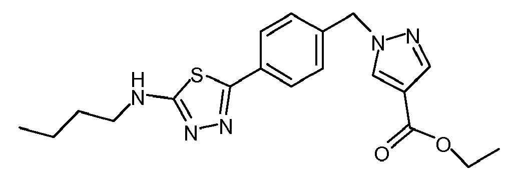

To a stirred solution of the title compound of Preparation 68 (1.00 g, 3.07 mmol) in EtOH (30 ml), ^{t}BuOK (0.52 g, 4.63 mmol) was added followed by the addition of ethyl 1H-pyrazole-4-carboxylate (0.52 g; 3.71 mmol. Aldrich) and then let to react at 60° for 72 h. Solvent was removed in vacuum and the crude mixture thus obtained was partitioned between water and ethyl acetate. The organic layer was dried and solvent was removed in vacuum to yield an oil that was purified according to purification method A to yield the title compound as a solid (280 mg, yield=23%).
LRMS: m/z 384 (M-1)⁻
Retention time: 6.28 min (method B)

### PREPARATION 70

### 1-(4-(5-(butylamino)-1,3,4-thiadiazol-2-yl)benzyl)-1H-pyrazole-4-carboxylic acid

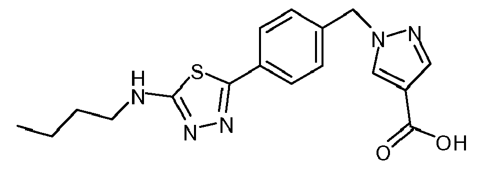

To a 0°C stirred solution of the title compound of Preparation 69 (400 mg, 1.04 mmol) in THF (10 ml), 60% NaH (29 mg, 1.21 mmol) was added and the mixture was stirred at that temperature for 30 min. Then a solution of 2-fluorobenzolyl chloride (164 mg, 1.21 mmol) in THF (1 ml) was added drop wise and the final mixture was stirred at rt for 2 h. 2N HCl (5 ml) was slowly added and the final mixture was portioned between water and ethyl acetate. The aqueous layer was extracted with ethyl acetate and the combined organic layers were washed with water and brine. Solvent was removed in vacuum to yield an oil that was purified according to purification method A to yield the title compound as a solid (60 mg, yield=11%) [LRMS: m/z 508 (M+1)⁺; retention time: 7.20 min (method B)]

The compound thus obtained was dissolved in HCl 2N (5 ml) and refluxed 72 h. The aqueous layer was extracted with ethyl acetate and the combined organic layers were washed with water and brine. Solvent was removed in vacuum and the crude thus obtained was dissolved in THF (4 ml), then LiOH (23 mg) was added and let to react at r. for 2h. It was acidified to pH 2-3 and extracted with ethyl acetate; the organic layer was washed with brine. It was dried and solvent was removed in vacuum to yield oil. 80 mg (81 % yield) of the title compound were obtained.
LRMS: m/z 358 (M+1)⁺
Retention time: 5.42 min (method B)

### PREPARATION 71

### Tert-butyl 1-(4-(5-(butylamino)-1,3,4-thiadiazol-2-yl)benzyl)-1H-pyrazole-4-carboxylate

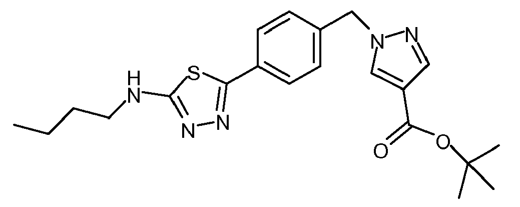

To a stirred solution of the title compound of Preparation 70 (80 mg, 0.22 mmol) in toluene (2 ml), 1,1-di-tert-butoxy-N,N-dimethylmethanamine (220 µl, 0.95 mmol) was added under N2 atmosphere and the mixture was stirred at 85° overnight. Toluene was removed in vacuum to yield oil that was partitioned between ethyl acetate and water. The organic layer was washed with water and brine and the solvent removed in vacuum to yield a solid. 60 mg (49% yield) of the title compound were obtained.
LRMS: m/z 414 (M+1)⁺
Retention time: 6.79 min (method B)

### PREPARATION 72

### tert-butyl 1-(4-(5-(N-butyl-2-fluorobenzamido)-1,3,4-thiadiazol-2-yl)benzyl)-1H-pyrazole-4-carboxylate

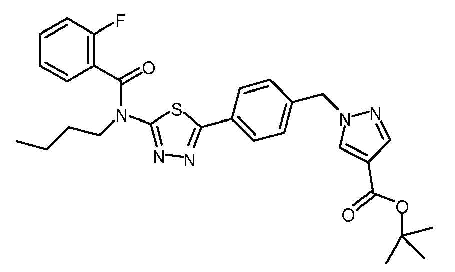

To a 0°C stirred solution of the title compound of Preparation 71 (100 mg, 0.24 mmol) in THF (3 ml), 60% NaH (5 mg, 0.210 mmol) was added and the mixture was stirred at that temperature for 30 min. Then a solution of 2-fluorobenzoyl chloride (42 mg, 0.26 mmol) in THF (2 ml) was added drop wise and the final mixture was stirred at room temperature for 5 h. AcOH (2 ml) was slowly added and the final mixture was portioned between water and ethyl acetate. The aqueous layer was extracted with ethyl acetate and the combined organic layers were washed with water and brine. It was dried and solvent removed to yield a crude product that was purified on a Biotage 40S chromatography column of silica gel using hexane/ethyl acetate (95:5) as eluent. 45 m g (40% yield) of the title product were obtained as a solid.
LRMS: m/z 536 (M+1)⁺
Retention time: 4.48 min (method B)

### PREPARATION 73

### tert-butyl 2-cyanoethylcarbamate

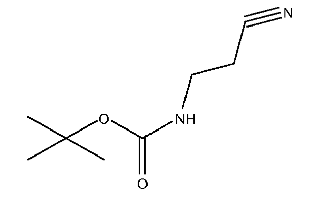

To a stirred solution of the title compound of 3-aminopropanenitrile (3 g, 42.8 mmol) in dioxane-water (27/27ml), calcium carbonate (6.03 g, 43.63 mmol) was added and the mixture was cooled at 0 °C. Di-tert-butyl dicarbonate (9.34 g, 42.79 mmol) in dioxane (22 ml) was dropped slowly and the solution stirred at room temperature overnight. Ethyl acetate was added and the organic phase was washed with sodium bicarbonate (4%) and brine. The solvent was removed in vacuum to yield oil. 7.0 g (96% yield) of the title compound were obtained.
GCMS: m/z 171 (M+1)⁺
Retention time: 6.63 min (20 min)

### PREPARATION 74

### tert-butyl 2-(1H-tetrazol-5-yl)ethylcarbamate

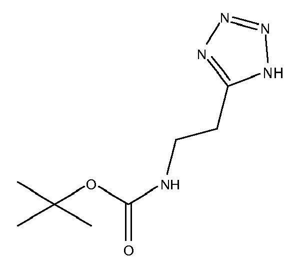

Obtained (65.5% yield) from the title compound of Preparation 73 following the experimental procedure of Preparation 32.
¹H NMR (400 MHz, CHLOROFORM-*d*) δ ppm 1.45 (s, 9 H) 3.17 - 3.27 (m, 2 H) 3.59 - 3.73 (m, 2 H) 5.17 (br. s., 1 H).

### PREPARATION 75

### 2-(1H-tetrazol-5-yl)ethanamine

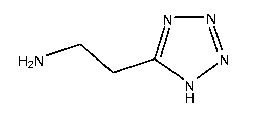

Obtained (100% yield) from the title compound of Preparation 74 following the experimental procedure of Preparation 39.
LRMS: m/z 114 (M+1)⁺
Retention time: 0.28 min (method A)

### PREPARATION 76

### (2S,4S)-1-tert-butyl 2-methyl 4-(4-(5-(butylamino)-1,3,4-thiadiazol-2-yl)-2,6-dimethylphenoxy)pyrrolidine-1,2-dicarboxylate

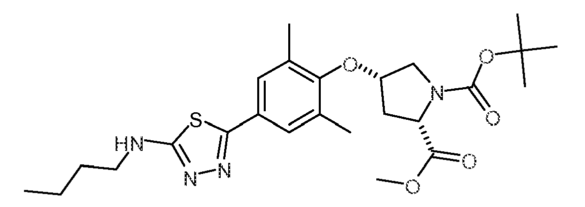

The title product of Preparation 57 (1.0 g, 3.61 mmol), (2S,4S)-1-tert-butyl 2-methyl 4-hydroxypyrrolidine-1,2-dicarboxylate (1.17 g, 4.51 mmol), triphenylphosphine (1.18 g, 4.51 mmol) were mixed under Ar atmosphere followed by the addition of dry THF (5 ml). The mixture was cooled at 0° and DIAD (885 µl, 4.51 mmol) was added drop wise. The reaction mixture was allowed to warm to rt and then stirred overnight. Solvent was removed and the residue thus obtained was partitioned between water and ethyl acetate. The organic layer was washed with water and brine. It was dried and solvent removed to yield a crude product that was purified according to purification method A to yield the title compound as a solid. 120 mg (6% yield) of the title product were obtained as a pale oil.
LRMS: m/z 505 (M+1)⁺
Retention time: 7.15 min (method B)

### PREPARATION 77

### (2S,4S)-1-tert-butyl 2-methyl 4-(4-(5-(N-butyl-2-fluorobenzamido)-1,3,4-thiadiazol-2-yl)-2,6-dimethylphenoxy)pyrrolidine-1,2-dicarboxylate

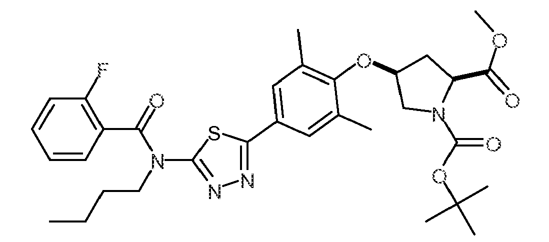

To a 0°C stirred solution of the title compound of Preparation 76 (120 mg, 0.24 mmol) in THF (3 ml), 60% NaH (20 mg, 0.52 mmol) was added and the mixture was stirred at that temperature for 30 min. Then a solution of 2-fluorobenzoyl chloride (83 mg, 0.50 mmol) in THF (1 ml) was added drop wise and the final mixture was stirred at rt for 1.5 h. Water (30 ml) was slowly added and the final mixture was portioned between water and ethyl acetate. The aqueous layer was extracted with ethyl acetate and the combined organic layers were washed with water and brine. It was dried and solvent removed to yield a crude of the title product (210 mg) used in the next step without purification.
LRMS: m/z 627 (M+1)⁺
Retention time: 7.78 min (method B)

### PREPARATION 78

### tert-butyl 2-(4-(5-(butylamino)-1,3,4-thiadiazol-2-yl)-2,6-dimethylphenoxy)-ethylcarbamate

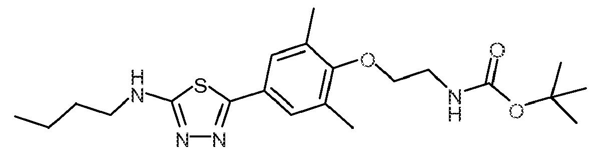

The title product of Preparation 57 (1.0 g, 3.61 mmol), tert-butyl 2-hydroxyethylcarbamate (910 mg, 5.65 mmol), triphenylphosphine (1.32 g, 5.03 mmol) was mixed under Ar atmosphere followed by the addition of dry THF (5 ml). The mixture was cooled at 0° and DIAD (1.02 µl, 5.20 mmol) was added drop wise. The reaction mixture was warm at 100° during 1h in the MW. Solvent was removed and the residue thus obtained was partitioned between water and ethyl acetate. The organic layer was washed with water and brine. It was dried and solvent removed to yield a crude product that was purified according to purification method A to yield the title compound as a white solid (540 mg, 36% yield).
LRMS: m/z 421 (M+1)⁺
Retention time: 6.97 min (method B
1H NMR (200 MHz, DMSO-*d*₆) δ ppm 0.91 (t, *J*=7.22 Hz, 3 H) 1.22 - 1.45 (m, 11 H) 1.46 - 1.66 (m, 2 H) 2.25 (s, 6 H) 3.13 - 3.40 (m, 4 H) 3.63 - 3.86 (m, 2 H) 6.97 - 7.12 (m, 1 H) 7.41 (s, 2 H) 7.76 - 7.91 (m, 1 H))

### PREPARATION 79

### N-(5-(4-(2-aminoethoxy)-3,5-dimethylphenyl)-1,3,4-thiadiazol-2-yl)-N-butyl-2-fluorobenzamide

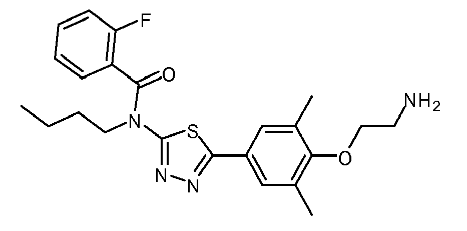

To a 0°C stirred solution of the title compound of Preparation 78 (540 mg, 1.28 mmol) in THF (6 ml), 60% NaH (108 mg, 2.70 mmol) was added and the mixture was stirred at that temperature for 30 min. Then a solution of 2-fluorobenzoyl chloride (428 mg, 2.70 mmol) in THF (5 ml) was added drop wise and the final mixture was stirred at room temperature for 2 h. Water (25 ml) was slowly added and the final mixture was portioned between water and ethyl acetate. The aqueous layer was extracted with ethyl acetate and the combined organic layers were washed with water and brine. It was dried and solvent removed to yield a crude solid that was dissolved in HCl 4N in dioxane (25 ml) and let to react during 48h. The gummy solid thus obtained was treated with diethyl ether and a white solid precipitated; after filtering, the title compound was obtained as a solid (350 mg, 59% yield).
LRMS: m/z 443M+1)⁺
Retention time: 5.60 min (method B)
1H NMR (200 MHz, DMSO-*d*₆) δ ppm 0.70 (t, *J*=7.42 Hz, 3 H) 1.04 - 1.25 (m, 2 H) 1.54 - 1.75 (m, 2 H) 2.35 (s, 6 H) 3.18 - 3.35 (m, 2 H) 3.91 - 4.15 (m, 4 H) 7.36 - 7.55 (m, 2 H) 7.60 - 7.81 (m, 4 H) 8.21 (br. s., 3 H)

### EXAMPLES

### EXAMPLE 1

### 1-[2-(4-{5-[Butyl(2-fluorobenzoyl)amino]-1,3,4-thiadiazol-2-yl}-2,6-dimethylphenoxy)ethyl]azetidine-3-carboxylic acid

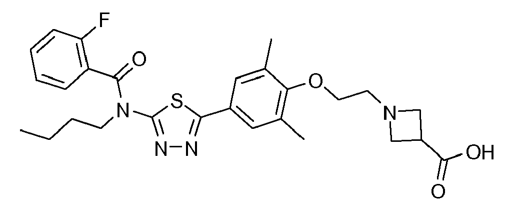

To a stirred suspension N-butyl-N-(5-(3,5-dimethyl-4-(2-oxoethoxy)phenyl)-1,3,4-thiadiazol-2-yl)-2-fluorobenzamide (Preparation 6) (150 mg, 0.34 mmol) and azetidine-3-carboxylic acid (41 mg, 0.41 mmol) in methanol (10ml), AcOH (176 µl, 3.1 mmol) was added and and the mixture was stirred at rt for 1h. Then, NaCNBH3 (11 mg, 0.18 mmol) was added portionwise and the final mixture was stirred at rt overnight. Acetic acid was added to the mixture until pH 3-4 and solvent was removed in vaccuo. The crude mixture thus obtained was purified according to purification method A to yield the title compound as a solid (yield=59%).
LRMS: m/z 527(M+1)⁺
Retention time: 14.68 min (method C)
¹H NMR (200 MHz, DMSO-d6) δ ppm 0.70 (t, 3 H) 1.03 - 1.25 (m, 2 H) 1.55 - 1.74 (m, 2 H) 2.31 (s, 6 H) 2.77 (s, 2 H) 3.28 (m, 3 H) 3.42 - 3.60 (m, 2 H) 3.75 (t, 2 H) 4.05 (t, 2 H) 7.34 - 7.54 (m, 2 H) 7.59 - 7.81 (m, 4 H)

### EXAMPLE 2

### N-[2-(4-{5-[Butyl(2-fluorobenzoyl)amino]-1,3,4-thiadiazol-2-yl}-2,6-dimethylphenoxy)ethyl]-beta-alanine

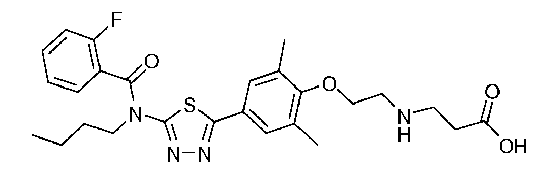

Obtained (45%) from the title compound of Preparation 6 and beta-alanine following the experimental procedure of Example 1.
LRMS: m/z 515(M+1)⁺
Retention time: 14.06 min (method C)
¹H NMR (200 MHz, DMSO-d6) δ ppm 0.70 (t, *J*=7.22 Hz, 3 H) 1.00 - 1.25 (m, 2 H) 1.55 - 1.74 (m, 2 H) 2.33 (s+m, 6 H+2H) 2.90 (t, *J*=6.44 Hz, 2 H) 2.95 - 3.09 (m, 2 H) 3.89 (t, *J*=4.69 Hz, 2 H) 4.05 (t, 2 H) 7.37 - 7.54 (m, 2 H) 7.60 - 7.80 (m, 4 H)

### EXAMPLE 3

### N-[2-(4-{5-[Butyl(2-fluorobenzoyl)amino]-1,3,4-thiadiazol-2-yl}-2,6-dimethylphenoxy)ethyl]glycine

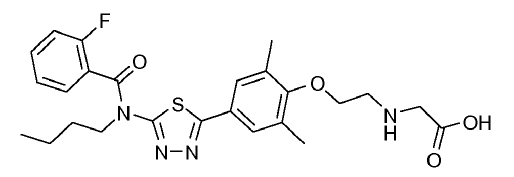

Obtained (10%) from the title compound of Preparation 6 and glycine following the experimental procedure of Example 1.
LRMS: m/z 501 (M+1)⁺
Retention time: 15.40 min (method C)
¹H NMR (200 MHz, DMSO-d6) δ ppm 0.70 (t, J=7.42 Hz, 3 H) 1.14 (m, J=8.20 Hz, 2 H) 1.65 (m, 2 H) 2.33 (s, 6 H) 3.17 (m, 2 H) 3.27 (m, 2 H) 4.00 (m, J=5.47 Hz, 4 H) 7.42 (m, J=7.42 Hz, 2 H) 7.60 - 7.81 (m, 4 H)

### EXAMPLE 4

### N-[2-(4-{5-[Butyl(2-fluorobenzoyl)amino]-1,3,4-thiadiazol-2-yl}-2,6-dimethylphenoxy)ethyl]-N-methylglycine

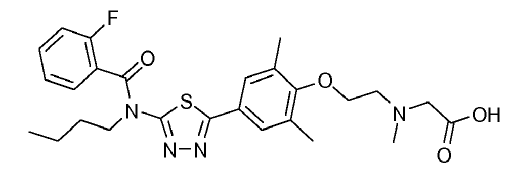

Obtained (70%) from the title compound of Preparation 6 and N-methylglycine following the experimental procedure of Example 1.
LRMS: m/z 515(M+1)⁺
Retention time: 15.25 min (method C)
¹H NMR (200 MHz, DMSO-d6) δ ppm 0.69 (t, 3 H) 1.04 - 1.25 (m, 2 H) 1.53 - 1.75 (m, 2 H) 2.32 (s, 6 H) 2.48 (s, 3 H) 3.02 (t, J=5.66 Hz, 2 H) 3.32 (s, 2 H) 3.93 (t, J=5.66 Hz, 2 H) 4.04 (t, J=1.95 Hz, 2 H) 7.35 - 7.54 (m, 2 H) 7.56 - 7.82 (m, 4 H)

### EXAMPLE 5

### 1-[2-(4-{5-[Butyl(2-fluorobenzoyl)amino]-1,3,4-thiadiazol-2-yl}-2,6-dimethylphenoxy)ethyl]piperidine-4-carboxylic acid

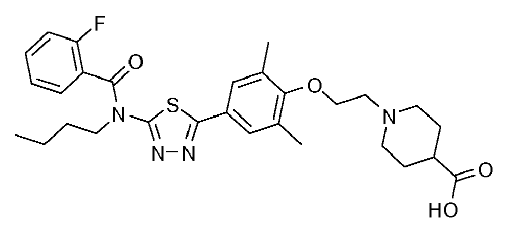

Obtained (86%) from the title compound of Preparation 6 and isonipecotic acid following the experimental procedure of Example 1.
LRMS: m/z 555(M+1)⁺
Retention time: 13.70 min (method C)
¹H NMR (200 MHz, DMSO-d6) d ppm 0.70 (t, J=7.22 Hz, 3 H) 1.03 - 1.23 (m, J=7.42 Hz, 2 H) 1.48 - 1.69 (m, 4 H) 1.70 - 1.90 (m, 2 H) 1.98 - 2.18 (m, 3 H) 2.32 (s, 6 H) 2.58 - 2.75 (m, J=5.08 Hz, 2 H) 2.77 - 3.00 (m, 2 H) 3.90 (t, J=5.27 Hz, 2 H) 4.04 (t, 2 H) 7.34 - 7.54 (m, 2 H) 7.55 - 7.81 (m, 4 H)

### EXAMPLE 6

### 1-[2-(4-{5-[butyl(2-fluorobenzoyl)amino]-1,3,4-thiadiazol-2-yl}-2,6-dimethylphenoxy)ethyl]piperidine-2-carboxylic acid

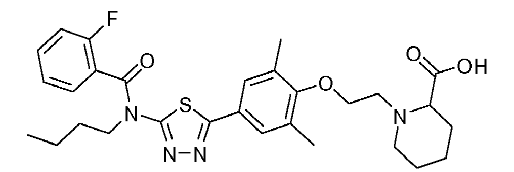

Obtained (51%) from the title compound of Preparation 6 and piperidine-2-carboxylic acid following the experimental procedure of Example 1.
LRMS: m/z 555(M+1)⁺
Retention time: 16.31 min (method C)
¹H NMR (200 MHz, DMSO-d6) δ ppm 0.70 (t, J=7.22 Hz, 3 H) 1.03 - 1.25 (m, 2 H) 1.39 - 1.59 (m, J=1.56 Hz, 2 H) 1.59 - 1.82 (m, J=9.17, 6.83 Hz, 6 H) 2.32 (s, 6 H) 2.81 - 3.20 (m, 4 H) 3.20 - 3.33 (m, 1 H) 3.92 (t, 2 H) 4.05 (t, 2 H) 7.34 - 7.54 (m, 2 H) 7.59 - 7.80 (m, 4 H)

### EXAMPLE 7

### 1-[2-(4-{5-[butyl(2-fluorobenzoyl)amino]-1,3,4-thiadiazol-2-yl}-2,6-dimethylphenoxy)ethyl]piperidine-3-carboxylic acid

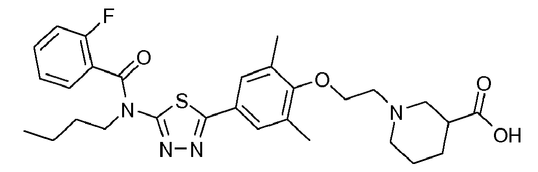

Obtained (67%) from the title compound of Preparation 6 and piperidine-3-carboxylic acid following the experimental procedure of Example 1.
LRMS: m/z 555(M+1)⁺
Retention time: 14.33 min (method C)
¹H NMR (200 MHz, DMSO-d6) δ ppm 0.70 (t, J=7.22 Hz, 3 H) 1.01 - 1.23 (m, 2 H) 1.27 - 1.93 (m, 6 H) 2.22 - 2.36 (s, 6 H) 2.40 - 2.50 (m, 2 H) 2.59 - 2.87 (m, 2 H) 2.96 (d, J=2.73 Hz, 1 H) 3.31 (m, 2 H) 3.91 (t, 2 H) 3.96 - 4.16 (m, 2 H) 7.36 - ) 7.53 (m, 2 H) 7.56-7.83 (m, 4 H)

### EXAMPLE 8

### 1-[2-(4-{5-[butyl(2-fluorobenzoyl)amino]-1,3,4-thiadiazol-2-yl}-2,6-dimethylphenoxy)ethyl]proline

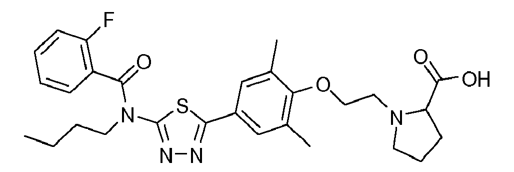

Obtained (71%) from the title compound of Preparation 6 and proline following the experimental procedure of Example 1.
LRMS: m/z 541(M+)⁺
Retention time: 15.98 min (method C)
¹H NMR (200 MHz, DMSO-d6) δ ppm 0.70 (t, 3 H) 0.95 - 1.26 (m, 2 H) 1.49 - 1.74(m, 2 H) 1.75 - 2.20 (m, 4 H) 2.32 (s, 6 H) 2.64-3.42 (m, 5 H) 3.80-4.19 (m, 4 H) 7.34 - 7.53 (m, 2 H) 7.56 - 7.80 (m, 4 H)

### EXAMPLE 9

### 1-[2-(4-{5-[butyl(2-fluorobenzoyl)amino]-1,3,4-thiadiazol-2-yl}-2,6-dimethylphenoxy)ethyl]pyrrolidine-3-carboxylic acid

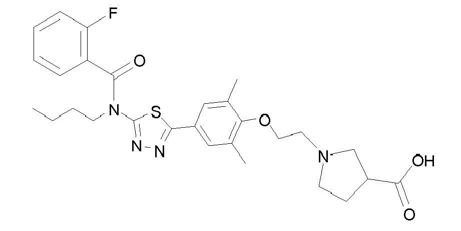

Obtained (63%) from the title compound of Preparation 6 and pyrrolidine-3-carboxylic acid following the experimental procedure of Example 1.
LRMS: m/z 541(M+1)⁺
Retention time: 14.02 min (method C)
¹H NMR (400 MHz, DMSO-d6) δ ppm 0.70 (t, 3 H) 1.10 - 1.21 (m, 2 H) 1.56 - 1.71 (m, 2 H) 2.20 - 2.32 (m, 1 H) 2.37 (s, 6 H) 3.17 - 3.34 (m, 2 H) 3.35 - 3.53 (m, 2 H) 3.60 - 3.79 (m, 3 H) 3.82 - 3.96 (m, 1 H) 3.99 - 4.11 (t, 2 H) 4.11 - 4.24 (t, 2 H) 7.40 - 7.52 (m, 2 H) 7.63 - 7.78 (s+m, 4 H)

### EXAMPLE 10

### (1SR,2RS)-2-{[2-(4-{5-[butyl(2-fluorobenzoyl)amino]-1,3,4-thiadiazol-2-yl}-2,6-dimethylphenoxy)ethyl]amino}cyclopentanecarboxylic acid

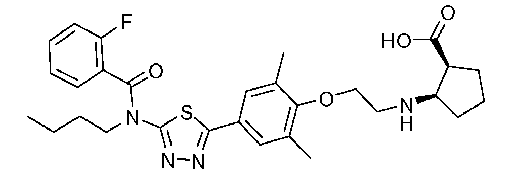

Obtained (63%) from the title compound of Preparation 6 and cis-2-aminocyclopentacarboxylic acid following the experimental procedure of Example 1.
LRMS: m/z 555(M+1)⁺
Retention time: 15.06 min (method C)
¹H NMR (400 MHz, DMSO-*d*₆) δ ppm 0.70 (t, J=7.23 Hz, 3 H) 1.13 (t, J=11.33 Hz, 2 H) 1.56 - 1.70 (m, 2 H) 1.73 - 1.84 (m, 2 H) 1.89 - 2.00 (m, 2 H) 2.36 (s, 6 H) 2.99 - 3.12 (m, 1 H) 3.59 - 3.70 (m, 1 H) 3.94 - 4.17 (m, 4 H) 7.36 - 7.53 (m, 2 H) 7.62 - 7.78 (m, 4 H)

### EXAMPLE 11

### {1-[2-(4-{5-[butyl(2-fluorobenzoyl)amino]-1,3,4-thiadiazol-2-yl}-2,6-dimethylphenoxy)ethyl]piperidin-4-yl}acetic acid

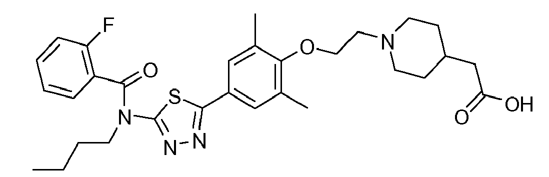

Obtained (60%) from the title compound of Preparation 6 and 2-(piperidin-4-yl)acetic acid following the experimental procedure of Example 1.
LRMS: m/z 568(M+1)⁺
Retention time: 13.36 min (method C)
¹H NMR (400 MHz, DMSO-*d*₆) δ ppm 0.70 (t, J=7.42 Hz, 3 H) 1.07 - 1.19 (m, 2 H) 1.49 - 1.59 (m, 2 H) 1.59 - 1.70 (m, 2 H) 1.65 (ddd, J=14.65, 7.62, 7.42 Hz, 1 H) 1.85 - 1.97 (m, 2 H) 2.17 - 2.27 (m, 0 H) 2.36 (s, 6 H) 3.03-3.15 (m, 2 H) 3.50 - 3.58 (m, 2 H) 3.60 - 3.72 (m, 2 H) 4.00 - 4.10 (m, 2 H) 4.15 - 4.23 (m, 2 H) 7.36 - 7.53 (m, 2 H) 7.62 - 7.78 (m, 4 H).

### EXAMPLE 12

### N-butyl-N-{5-[3,5-dimethyl-4-(2-{[2-(2H-tetrazol-5-yl)ethyl]amino}ethoxy)phenyl]-1,3,4-thiadiazol-2-yl}-2-fluorobenzamide

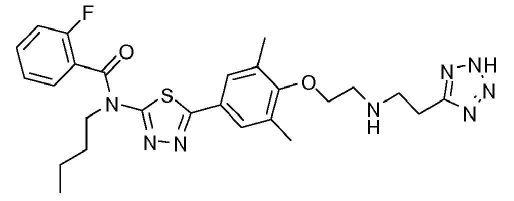

Obtained (40%) from the title compound of Preparation 6 and the title compound of Preparation 75 following the experimental procedure of Example 1.
LRMS: m/z 539(M+1)⁺
Retention time: 13.95 min (method C)
¹H NMR (400 MHz, DMSO-*d*₆) δ ppm 0.70 (t, J=7.42 Hz, 3 H), 1.14 (m, 2 H), 1.65 (dt, J=14.85, 7.42 Hz, 2 H), 2.35 (s, 6 H), 3.09 (t, J=6.84 Hz, 2 H), 3.32 (t, J=6.84 Hz, 2 H), 3.37 (t, J=5.08 Hz, 2 H), 4.03 (t, J=5.08 Hz, 4 H), 7.45 (m, 2 H), 7.69 (m, 4 H).

### EXAMPLE 13

### 1-[2-(4-{5-[butyl(2-fluorobenzoyl)amino]-1,3,4-thiadiazol-2-yl}-2,6-dimethylphenoxy)ethyl]-4-methylpiperidine-4-carboxylic acid

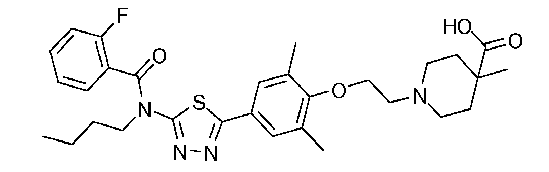

Obtained (47%) from the title compound of Preparation 6 and 4-methylpiperidine-4-carboxylic acid following the experimental procedure of Example 1.
LRMS: m/z 569(M+1)⁺
Retention time: 13.26 min (method C)
¹H NMR (400 MHz, DMSO-*d*₆) δ ppm 0.69 (t, J=7.23 Hz, 3 H) 1.04 (d, J=5.86 Hz, 3 H) 1.09 - 1.18 (m, 2 H) 1.67 - 1.88 (m, 4 H) 2.24 (d, J=12.90 Hz, 1 H) 2.37 (br. s., 6 H) 2.98 - 3.08 (m, 0 H) 3.58 - 3.67 (m, 4 H) 4.13 - 4.20 (m, 2 H) 7.53 - 7.66 (m, 2 H) 7.66 - 7.81 (m, 4 H).

### EXAMPLE 14

### cis-4-{[2-(4-{5-[butyl(2-fluorobenzoyl)amino]-1,3,4-thiadiazol-2-yl}-2,6-dimethylphenoxy)ethyl]amino}cyclohexanecarboxylic acid

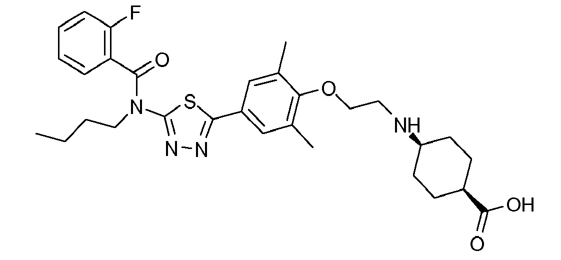

Obtained (29%) from the title compound of Preparation 6 and 4-methylpiperidine-4-carboxylic acid following the experimental procedure of Example 1.
LRMS: m/z 569(M+1)⁺
Retention time: 13.16 min (method C)
¹H NMR (400 MHz, DMSO-*d*₆) δ ppm 0.69 (t, J=7.42 Hz, 3 H) 1.05 - 1.22 (m, 2 H) 1.46 - 1.78 (m, 6 H) 1.97 - 2.16 (m, 4 H) 2.37 (s, 6 H) 2.54 - 2.63 (m, 1 H) 3.13 - 3.24 (m, 1 H) 3.74 - 3.88 (m, 1 H) 4.04 - 4.19 (m, 4 H) 7.53 - 7.66 (m, 2 H) 7.67 - 7.81 (m, 4 H) 12.32 (br. s., 1 H).

### EXAMPLE 15

### 1-[2-(4-{5-[ethyl(2-fluorobenzoyl)amino]-1,3,4-thiadiazol-2-yl}-2,6-dimethylphenoxy)ethyl]piperidine-4-carboxylic acid hydrochloride

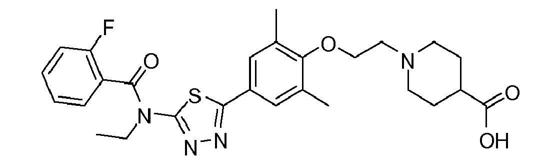

Obtained (47%) from the title compound of Preparation 11 and isonipecotic acid following the experimental procedure of Example 1. The hydrochloride salt was done in dioxane with HCl 4M in dioxane.
LRMS: m/z 527 (M+1)⁺
Retention time: 11.72 min (method C)
¹H NMR (400 MHz, DMSO-d6) δ ppm 1.23 (t, J=6.25 Hz, 3 H) 1.92 (br. s., 1 H) 2.09 (br. s., 2 H) 2.36 (br. s., 6 H) 3.09 (br. s., 2 H) 3.34 (br. s., 2 H) 3.57 (s, 2 H) 3.69 (br. s., 2 H) 4.09 (q, 2 H) 4.21 (br. s., 2 H) 7.39 - 7.52 (m, 2 H) 7.67 (q, 2 H) 7.74 (br. s., 2 H) 10.39 (br. s., 1 H) 12.57 (br. s., 1 H)

### EXAMPLE 16

### 1-[2-(4-{5-[(2-chlorobenzoyl)(ethyl)amino]-1,3,4-thiadiazol-2-yl}-2,6-dimethylphenoxy)ethyl]piperidine-4-carboxylic acid hydrochloride

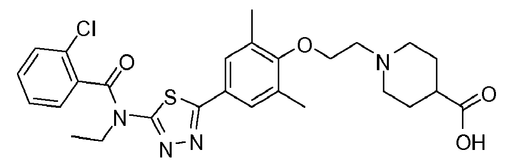

Obtained (47%) from the title compound of Preparation 12 and isonipecotic acid following the experimental procedure of Example 1. The hydrochloride salt was done in dioxane with HCl 4M in dioxane.
LRMS: m/z 543 (M+1)⁺
Retention time: 12.34 min (method C)
¹H NMR (400 MHz, DMSO-d6) δ ppm 1.23 (t, 3 H) 1.86 - 1.99 (m, 1 H) 2.08 (br. s., 2 H) 2.37 (br. s., 6 H) 3.09 (br. s., 2 H) 3.48 - 3.60 (m, 4 H) 3.67 (br. s., 2 H) 3.87 (br. s., 1 H) 4.23 (br. s., 3 H) 7.53 - 7.66 (m, 2 H) 7.66 - 7.80 (m, 4 H) 10.54 (br. s., 1 H) 12.58 (br. s., 1 H)

### EXAMPLE 17

### 1-[2-(4-{5-[(3-chloro-2-fluorobenzoyl)(ethyl)amino]-1,3,4-thiadiazol-2-yl}-2,6-dimethylphenoxy)ethyl]piperidine-4-carboxylic acid hydrochloride

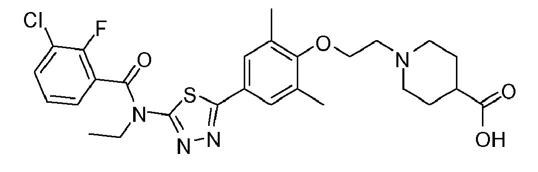

Obtained (41%) from the title compound of Preparation 13 and isonipecotic acid following the experimental procedure of Example 1. The hydrochloride salt was done in dioxane with HCl 4M in dioxane.
LRMS: m/z 561 (M+1)⁺
Retention time: 13.01 min (method C)
¹H NMR (400 MHz, DMSO-d6) δ ppm 1.21 (t, 3 H) 1.90 (br. s., 1 H) 2.06 (br. s., 2 H) 2.34 (s, 6 H) 3.06 (br. s., 2 H) 3.48 - 3.54 (m, 2 H) 3.55 (br. s., 2 H) 3.67 (br. s., 2 H) 4.07 (q, 2 H) 4.20 (br. s., 2 H) 7.44 (t, J=7.82 Hz, 1 H) 7.72 (s, 3 H) 7.84 (t, J=7.62 Hz, 1 H) 10.41 (br. s., 1 H)

### EXAMPLE 18

### N-[2-(4-{5-[ethyl(2-fluorobenzoyl)amino]-1,3,4-thiadiazol-2-yl}-2,6-dimethylphenoxy)ethyl]-N-methylglycine hydrochloride

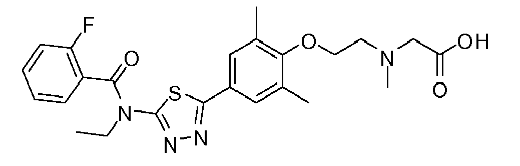

Obtained (39%) from the title compound of Preparation 11 and N-methyl-glycine following the experimental procedure of Example 1. The hydrochloride salt was done in dioxane with HCl 4M in dioxane.
LRMS: m/z 487 (M+1)⁺
Retention time: 13.45 min (method C)
¹H NMR (400 MHz, DMSO-d6) δ ppm 1.20 (t, J=6.84 Hz, 3 H) 2.32 (s, 6 H) 2.78 (br. s., 3 H) 3.38 (br. s., 2 H) 3.86 (br. s., 2 H) 4.01 - 4.13 (m, 4 H) 7.38 - 7.49 (m, 2 H) 7.61 - 7.75 (m, 4 H).

### EXAMPLE 19

### 1-[2-(4-{5-[ethyl(2-fluorobenzoyl)amino]-1,3,4-thiadiazol-2-yl}-2,6-dimethylphenoxy)ethyl]piperidine-3-carboxylic acid hydrochloride

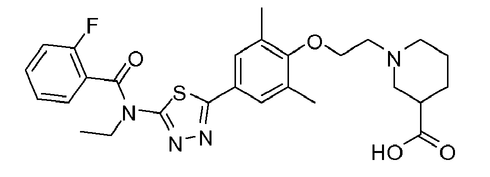

Obtained (56%) from the title compound of Preparation 11 and nipecotic acid following the experimental procedure of Example 1. The hydrochloride salt was done in dioxane with HCl 4M in dioxane.
LRMS: m/z 527 (M+1)⁺
Retention time: 12.38 min (method C)
¹H NMR (400 MHz, DMSO-d6) δ ppm 1.21 (t, J=7.03 Hz, 3 H) 1.48 (br. s., 1 H) 1.82 - 1.96 (m, 2 H) 2.05 (br. s., 1 H) 2.35 (s, 6 H) 2.96 (br. s., 2 H) 3.14 (br. s., 1 H) 3.55 (br. s., 3 H) 3.79 (br. s., 1 H) 4.06 (q, J=6.90 Hz, 2 H) 4.22 (br. s., 2 H) 7.37 - 7.50 (m, 2 H) 7.61 - 7.69 (m, 2 H) 7.72 (s, 2 H) 10.53 (br. s., 1 H) 12.89 (br. s., 1 H).

### EXAMPLE 20

### 1-[2-(4-{5-[ethyl(2-fluorobenzoyl)amino]-1,3,4-thiadiazol-2-yl}-2,6-dimethylphenoxy)ethyl]azetidine-3-carboxylic acid hydrochloride

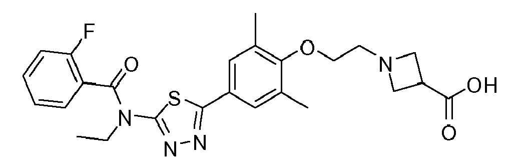

Obtained (24%) from the title compound of Preparation 11 and azetidine-3-carboxylic acid following the experimental procedure of Example 1. The hydrochloride salt was done in dioxane with HCl 4M in dioxane.
LRMS: m/z 499 (M+1)⁺
Retention time: 12.14 min (method C)
¹H NMR (400 MHz, DMSO-d6) δ ppm 1.23 (t, J=6.84 Hz, 3 H) 2.34 (s, 6 H) 3.62 - 3.75 (m, 3 H) 4.03 (br. s., 2 H) 4.09 (q, J=6.90 Hz, 2 H) 4.28 - 4.35 (m, 2 H) 4.35 - 4.45 (m, 2 H) 7.39 - 7.52 (m, 2 H) 7.63 - 7.77 (m, 4 H) 10.71 (br. s., 1 H) 10.89 (br. s., 1 H).

### EXAMPLE 21

### 1-[2-(4-{5-[(2-chlorobenzoyl)(cyclopropylmethyl)amino]-1,3,4-thiadiazol-2-yl}-2,6-dimethylphenoxy)ethyl]piperidine-4-carboxylic acid hydrochloride

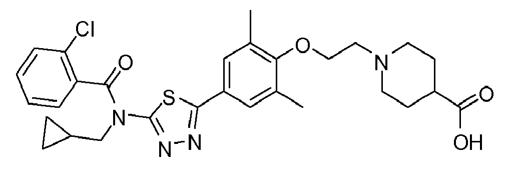

Obtained (44%) from the title compound of Preparation 18 and isonipecotic acid following the experimental procedure of Example 1. The hydrochloride salt was done in dioxane with HCl 4M in dioxane.
LRMS: m/z 569 (M+1)⁺
Retention time: 12.98 min (method C)
¹H NMR (400 MHz, DMSO-d6) δ ppm 0.05 - 0.24 (m, 2 H) 0.36 - 0.50 (m, 2 H) 1.13 - 1.23 (m, 1 H) 1.90 (q, 2 H) 2.11 (d, J=12.90 Hz, 3 H) 2.37 (s, 6 H) 3.11 (q, 2 H) 3.55 (br. s., 2 H) 3.69 (d, J=10.55 Hz, 2 H) 3.76 - 3.93 (m, 1 H) 3.98 - 4.15 (m, 1 H) 4.22 (br. s., 2 H) 7.56 (t, 1 H) 7.62 (t, J=7.03 Hz, 1 H) 7.65 - 7.72 (m, 1 H) 7.72 - 7.83 (m, 3 H) 10.27 (br. s., 1 H) 12.55 (br. s., 1 H).

### EXAMPLE 22

### N-[2-(4-{5-[(2-chlorobenzoyl)(cyclopropylmethyl)amino]-1,3,4-thiadiazol-2-yl}-2,6-dimethylphenoxy)ethyl]-N-methylglycine hydrochloride

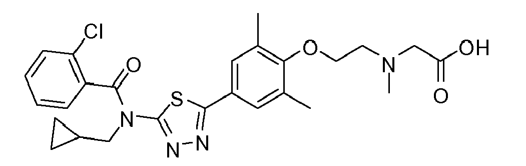

Obtained (52%) from the title compound of Preparation 18 and N-methyl-glycine following the experimental procedure of Example 1. The hydrochloride salt was done in dioxane with HCl 4M in dioxane.
LRMS: m/z 529 (M+1)⁺
Retention time: 15.29 min (method C)
¹H NMR (400 MHz, DMSO-d6) δ ppm 0.07 - 0.20 (m, 2 H) 0.37 - 0.48 (m, 2 H) 1.17 (quin, 1 H) 2.35 (s, 6 H) 2.95 (s, 3 H) 3.59 (br. s., 2 H) 3.72 - 3.93 (m, 1 H) 4.01 - 4.25 (m, 4 H) 7.56 (t, J=7.23 Hz, 1 H) 7.62 (t, J=7.23 Hz, 1 H) 7.66 - 7.71 (m, 1 H) 7.71 - 7.81 (m, 3 H).

### EXAMPLE 23

### {1-[2-(4-{5-[(2-chlorobenzoyl)(cyclopropylmethyl)amino]-1,3,4-thiadiazol-2-yl}-2,6-dimethylphenoxy)ethyl]piperidin-4-yl}acetic acid hydrochloride

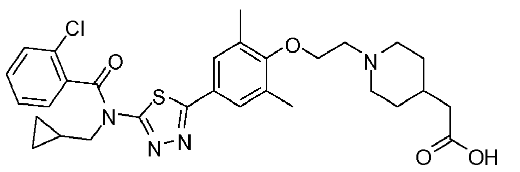

Obtained (57%) from the title compound of Preparation 18 and (piperidin-4-yl)acetic acid following the experimental procedure of Example 1. The hydrochloride salt was done in dioxane with HCl 4M in dioxane.
LRMS: m/z 583 (M+1)⁺
Retention time: 12.91 min (method C)
¹H NMR (400 MHz, DMSO-d6) δ ppm 0.06 - 0.21 (m, 2 H) 0.37 - 0.49 (m, 2 H) 1.17 (quin, 1 H) 1.59 (q, 2 H) 1.85 - 2.00 (m, 3 H) 2.23 (d, J=6.64 Hz, 2 H) 2.37 (s, 6 H) 3.09 (q, J=10.81 Hz, 2 H) 3.53 (d, J=4.30 Hz, 2 H) 3.65 (d, J=10.94 Hz, 2 H) 3.76 - 3.90 (m, 1 H) 4.01 - 4.13 (m, 1 H) 4.15 - 4.27 (m, 2 H) 7.56 (t, J=7.23 Hz, 1 H) 7.62 (t, J=7.03 Hz, 1 H) 7.65 - 7.71 (m, 1 H) 7.72 - 7.80 (m, 3 H) 10.08 (br. s., 1 H) 12.27 (s, 1 H).

### EXAMPLE 24

### 1-[2-(4-{5-[(2-chlorobenzoyl)(cyclopropylmethyl)amino]-1,3,4-thiadiazol-2-yl}-2,6-dimethylphenoxy)ethyl]azetidine-3-carboxylic acid, fumarate salt (2:1)

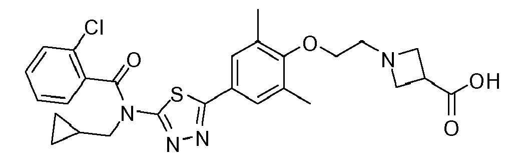

Obtained (13%) from the title compound of Preparation 18 and azetidine-3-carboxylic acid following the experimental procedure of Example 1. The fumarate was done in dioxane with fumaric acid.
LRMS: m/z 541 (M+1)⁺
Retention time: 14.03 min (method C)
¹H NMR (400 MHz, DMSO-d6) δ ppm 0.14 (br. s., 2 H) 0.42 (br. s., 2 H) 1.17 (br. s., 1 H) 2.31 (br. s., 6 H) 2.85 (br. s., 2 H) 3.05 - 3.94 (m, 9 H) 4.06 (br. s., 1 H) 6.60 (br. s., 1 H) 7.46 - 7.88 (m, 6 H).

### EXAMPLE 25

### 1-[4-(5-{butyl[(2-methoxyphenyl)acetyl]amino}-1,3,4-thiadiazol-2-yl)benzyl]-azetidine-3-carboxylic acid

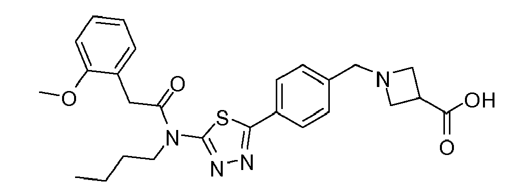

Obtained (66%) from the title compound of Preparation 23 and azetidine-3-carboxylic acid following the experimental procedure of Preparation 31
LRMS: m/z 495 (M+1)⁺
Retention time: 13.86 min (method C)
¹H NMR (400 MHz, DMSO-d6) δ ppm 0.94 (t, *J*=7.43 Hz, 3 H) 1.32 - 1.48 (m, 2 H) 1.68 1.82 (m, 2 H) 3.10 - 3.23 (m, 4 H) 3.52 - 3.62 (m, 3 H) 3.73 (s, 3 H) 4.05 (s, 2 H) 4.22 - 4.37 (m, 2 H) 6.92 (t, *J*=7.43 Hz, 1 H) 7.00 (d, *J*=8.22 Hz, 1 H) 7.21 (d, *J*=7.04 Hz, 1 H) 7.28 (t, *J*=7.83 Hz, 1 H) 7.38 (d, *J*=8.22 Hz, 2 H) 7.82 (d, *J*=7.83 Hz, 2 H)

### EXAMPLE 26

### N-(4-{5-[butyl(2-fluorobenzoyl)amino]-1,3,4-thiadiazol-2-yl}benzyl)-N-methylglycine

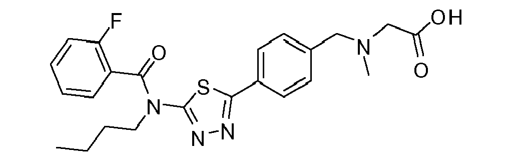

Obtained (70%) from the title compound of Preparation 24 and 2-(methylamino)acetic acid following the experimental procedure of Preparation 31
LRMS: m/z 457 (M+1)⁺
Retention time: 14.02 min (method C)
¹H NMR (400 MHz, DMSO-d6) δ ppm 0.70 (t, 3 H), 1.15 (m, 2 H), 1.66 (m, 2 H), 2.31 (s, 3 H), 3.24 (s, 2 H), 3.75 (s, 2 H), 4.08 (t, 2 H), 7.47 (m, 4 H), 7.67 (m, 1 H), 7.74 (dd, J=14.51, 1.66 Hz, 1 H), 7.97 (d, J=8.29 Hz, 2 H).

### EXAMPLE 27

### N-butyl-N-(5-{4-[(3-cyanoazetidin-1-yl)methyl]phenyl}-1,3,4-thiadiazol-2-yl)-2-fluorobenzamide

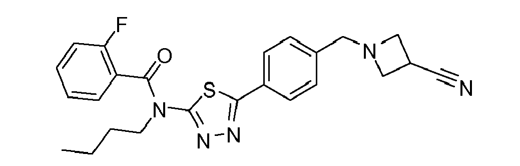

Obtained (30%) from the title compound of Preparation 24 and azetidin-3-carbonitrile hydrochloride following the experimental procedure of Preparation 31
LRMS: m/z 450 (M+1)⁺
Retention time: 13.42 min (method C)
¹H NMR (200 MHz, DMSO-d6) δ ppm 0.70 (t, 3 H), 1.14 (m, 2 H), 1.64 (m, 2 H), 3.32 (s, 3 H), 3.49 (d, J=2.73 Hz, 2 H), 3.66 (s, 2 H), 4.04 (t, 2 H), 7.45 (m, 4 H), 7.69 (m, 2 H), 7.95 (d, J=8.20 Hz, 2 H)

### EXAMPLE 28

### 1-[4-(5-{butyl[(2-methoxyphenyl)acetyl]amino}-1,3,4-thiadiazol-2-yl)-3-methylbenzyl]azetidine-3-carboxylic acid

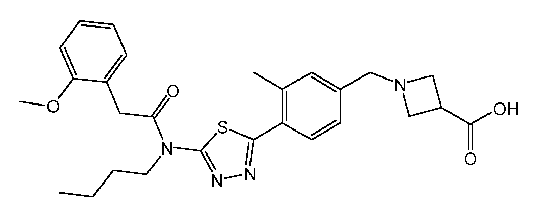

Obtained (62%) from the title compound of Preparation 27 and azetidin-3-carboxilic acid following the experimental procedure of Preparation 31
LRMS: m/z 509 (M+1)⁺
Retention time: 14.48 min (method C)
¹H NMR (400 MHz, DMSO-d6) δ ppm 0.97 (t, *J*=7.46 Hz, 3 H) 1.44 (sxt, *J*=7.46 Hz, 2 H) 1.71 - 1.86 (m, 2 H) 2.48 (s, 3 H) 3.14 - 3.26 (m, 3 H) 3.39 (br. s., 2 H) 3.56 (s, 2 H) 3.75 (s, 3 H) 4.07 (s, 2 H) 4.25 - 4.37 (m, 2 H) 6.94 (t, *J*=7.26 Hz, 1 H) 7.02 (d, *J*=7.88 Hz, 1 H) 7.19 - 7.26 (m, 2 H) 7.26 - 7.34 (m, 2 H) 7.59 (d, *J*=7.88 Hz, 1 H)

### EXAMPLE 29

### N-(4-{5-[butyl(2-fluorobenzoyl)amino]-1,3,4-thiadiazol-2-yl}benzyl)-N-methyl-beta-alanine

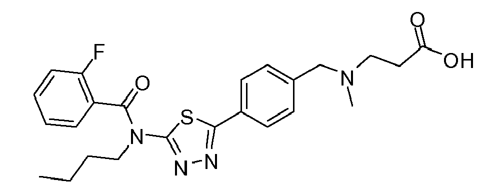

To a stirred solution 3-(4-(5-(N-butyl-2-fluorobenzamido)-1,3,4-thiadiazol-2-yl)benzylamino)propanoic acid (Preparation 31) (192 mg, 0.42 mmol) and formaldehyde (34 mg, 0.42 mmol) in methanol (5ml), AcOH (96 µl, 1.68 mmol) was added and the mixture was stirred at rt for 1 h. Then, NaCNBH₃ (13 mg, 0.21 mmol) was added portionwise and the final mixture was stirred at room temperature overnight. Solvent and acetic acid were removed in vaccuo. The crude mixture thus obtained was purified according to purification method A to yield the title compound as a solid (65% yield).
LRMS: m/z 471 (M+1)⁺
Retention time: 12.87 min (method C)
¹H NMR (400 MHz, DMSO-d6) δ ppm 0.70 (t, J=7.42 Hz, 3 H), 1.15 (m, 2 H), 1.66 (m, 2 H), 2.67 (s, 3 H), 2.87 (t, 2 H), 3.35 (s, 4 H), 4.07 (d, J=7.42 Hz, 2 H), 7.44 (t, J=7.42 Hz, 1 H), 7.49 (d, J=9.38 Hz, 1 H), 7.68 (m, 1 H), 7.73 (m, 1 H), 7.77 (d, J=8.21 Hz, 2 H), 8.10 (d, J=8.60 Hz, 2 H).

### EXAMPLE 30

### N-butyl-2-fluoro-N-[5-(4-{[3-(1H-tetrazol-5-yl)azetidin-1-yl]methyl}phenyl)-1,3,4-thiadiazol-2-yl]benzamide

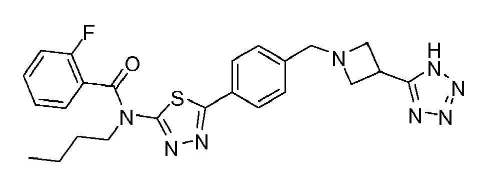

Obtained (18%) from the title compound of Preparation 24 and 5-(azetidin-3-yl)-1 H-tetrazole hydrochloride (Preparation 33) following the experimental procedure of Preparation 31
LRMS: m/z 493 (M+1)⁺
Retention time: 13.66 min (method C)
¹H NMR (400 MHz, DMSO-d6) δ ppm 0.70 (t, J=7.42 Hz, 3 H), 1.15 (m, 2 H), 1.66 (m, 2 H), 3.53 (t, 2 H), 3.82 (t, J=7.82 Hz, 2 H), 3.87 (s, 1 H), 4.00 (m, 2 H), 4.05 (t, 2 H), 7.47 (m, 4 H), 7.70 (m, 2 H), 7.97 (d, J=8.21 Hz, 2 H).

### EXAMPLE 31

### N-butyl-N-{5-[3,5-dimethyl-4-(2-{[2-(1H-1,2,4-triazol-1-yl)ethyl]amino}ethoxy)-phenyl]-1,3,4-thiadiazol-2-yl}-2-fluorobenzamide

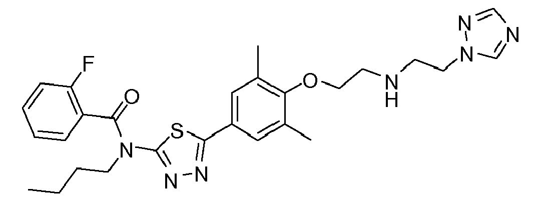

Obtained (16%) from the title compound of Preparation 6 and 2-(1H-1,2,4-triazol-1-yl)-ethanamine following the experimental procedure of Example 1.
LRMS: m/z 538 (M+1)⁺
Retention time: 12.55 min (method C)
¹H NMR (400 MHz, CHLOROFORM-d)δ ppm 0.77 (t, J=7.23 Hz, 3 H) 1.21 (sxt, 2 H) 1.74 (quin, J=7.52 Hz, 2 H) 2.32 (s, 6 H) 3.02 (t, J=5.08 Hz, 2 H) 3.21 (t, J=5.67 Hz, 2 H) 3.90 (t, J=5.08 Hz, 2 H) 4.15 (br. s., 2 H) 4.36 (t, J=5.67 Hz, 2 H) 7.22 (t, J=8.99 Hz, 1 H) 7.31 (t, J=7.62 Hz, 1 H) 7.46 (t, J=6.45 Hz, 1 H) 7.50 - 7.58 (m, 1 H) 7.66 (s, 2 H) 7.98 (s, 1 H) 8.19 (s, 1 H).

### EXAMPLE 32

### N-butyl-2-fluoro-N-{5-[4-({[2-(1H-1,2,4-triazol-1-yl)ethyl]amino}methyl)phenyl]-1,3,4-thiadiazol-2-yl}benzamide

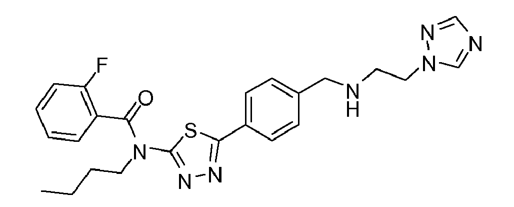

Obtained (32%) from the title compound of preparation 24 and 2-(1H-1,2,4-triazol-1-yl) ethanamine following the experimental procedure of Preparation 31
LRMS: m/z 480 (M+1)⁺
Retention time: 11.25 min (method C)
¹H NMR (400 MHz, DMSO-d6) δ ppm 0.66 (t, 3 H) 0.97 - 1.23 (m, 2 H) 1.62 (br. s., 2 H) 2.87 (br. s., 2 H) 3.73 (br. s., 2 H) 4.02 (br. s., 2 H) 4.23 (br. s., 2 H) 7.41 (br. s., 4 H) 7.57 - 7.79 (m, 2 H) 7.90 (d, J=5.86 Hz, 2 H) 7.93 (br. s., 1 H) 8.48 (br. s., 1 H).

### EXAMPLE 33

### N-(4-{5-[butyl(2-chlorobenzoyl)amino]-1,3,4-thiadiazol-2-yl}-2,6-dimethylbenzyl)-N-methylglycine hydrochloride

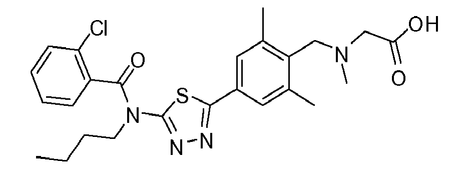

To a solution of N-butyl-2-chloro-N-(5-(4-formyl-3,5-dimethylphenyl)-1,3,4-thiadiazol-2-yl)benzamide (preparation 37) (100 mg, 0.23 mmol) and 2-(methylamino)acetic acid (21 mg, 0.24 mmol) in 4 ml of methanol was added 1050 µl of 0.3M (MeOH) solution of (ZnCl₂NaBH₃CN) at rt. The reaction mixture was stirred at room temperature overnight. Solvent was removed and the residue obtained was purified according to purification method A. The hydrochloride salt was done in dioxane with HCl 4M in dioxane. (9% yield)
LRMS: m/z 502 (M+1)⁺
Retention time: 15.42 min (method C)
¹H NMR (400 MHz, DMSO-d6) δ ppm 0.69 (t, 3 H), 1.15 (m, 2 H), 1.66 (m, 2 H), 2.53 (s, 3 H), 2.64 (s, 2 H), 3.83 (s, 2 H), 4.14 (s, 2 H), 7.61 (m, 2 H), 7.70 (m, 1 H), 7.76 (d, J=1.95 Hz, 1 H), 7.78 (s, 2 H).

### EXAMPLE 34

### 3-[(4-{5-[butyl(phenylacetyl)amino]-1,3,4-thiadiazol-2-yl}benzyl)amino]-2-methylpropanoic acid

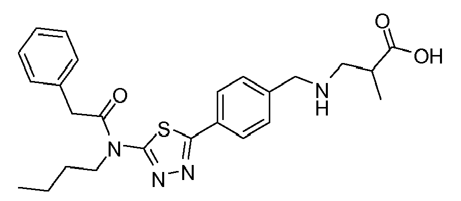

Obtained (33%) from the title compound of Preparation 22 and 3-amino-2-methylpropanoic acid following the experimental procedure of Preparation 31
LRMS: m/z 467 (M+1)⁺
Retention time: 13.31 min (method C)
¹H NMR (200 MHz, DMSO-d6) δ ppm 0.93 (t, J=7.22 Hz, 3 H) 1.03 (d, J=6.64 Hz, 2 H) 1.30 - 1.49 (m, 2 H) 1.59 - 1.79 (m, 2 H) 2.57 - 2.69 (m, 2 H) 3.83 (s, 2 H) 4.20 (s, 2 H) 4.25 (t, 2 H) 7.27 - 7.40 (m, 5 H) 7.49 (d, J=8.20 Hz, 2 H) 7.89 (d, J=7.81 Hz, 2 H).

### EXAMPLE 35

### 3-[(4-{5-[butyl(phenylacetyl)amino]-1,3,4-thiadiazol-2-yl}benzyl)amino]-3-methylpropanoic acid

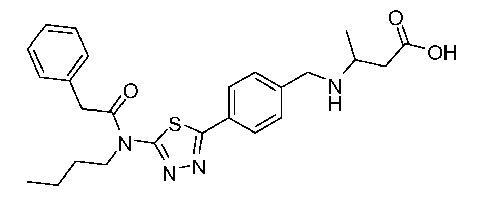

Obtained (17%) from the title compound of Preparation 22 and 3-aminobutanoic acid following the experimental procedure of Preparation 31
LRMS: m/z 467 (M+1)⁺
Retention time: 13.54 min (method C)
¹H NMR (200 MHz, DMSO-d6) δ ppm 0.94 (t, 3 H) 1.09 - 1.17 (m, 3 H) 1.32 - 1.47 (m, 2 H) 2.19 - 2.30 (m, 2 H) 3.85 - 3.94 (m, 2 H) 4.20 (s, 2 H) 4.31 (t, 3 H) 7.34 (d, J=1.17 Hz, 5 H) 7.52 (d, J=8.59 Hz, 2 H) 7.92 (d, J=8.20 Hz, 2 H).

### EXAMPLE 36

### N-butyl-2-fluoro-N-[5-(4-{[(2-hydroxyethyl)amino]methyl}phenyl)-1,3,4-thiadiazol-2-yl]benzamide, formate salt

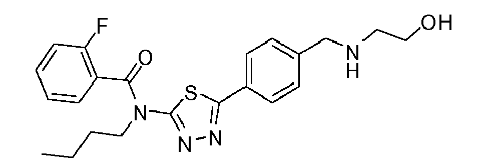

Obtained (9%) from the title compound of Preparation 24 and 2-aminoethanol following the experimental procedure of Preparation 31
LRMS: m/z 429 (M+1)⁺
Retention time: 11.36 min (method C)
¹H NMR (400 MHz, DMSO-d6) δ ppm 0.70 (t, J=7.42 Hz, 3H) 1.15 (dq, J=14.85, 7.42 Hz, 2H) 1.66 (ddd, J=14.65, 7.62, 7.42 Hz, 2H) 3.00 (br. s., 2H) 3.62-3.73 (m, 2H) 4.07 (t, J=7.42 Hz, 2H) 4.25 (br. s., 2H) 5.29 (br, s., 1 H) 7.39 - 7.52 (m, 2H) 7.64 -7.79 (m, 4H) 8.08 (d, J=8.21 Hz, 2H).

### EXAMPLE 37

### 1-(4-{5-[butyl(2-fluorobenzoyl)amino]-1,3,4-thiadiazol-2-yl}benzyl)-4-(ethoxycarbonyl)piperidine-4-carboxylic acid

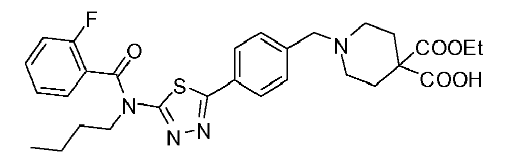

Obtained (20%) from the title compound of Preparation 24 and 4-(ethoxycarbonyl)-piperidine-4-carboxilic acid (Preparation 39) following the experimental procedure of Preparation 31
LRMS: m/z 569 (M+1)⁺
Retention time: 14.30 min (method C)
¹H NMR (400 MHz, DMSO-d6) δ ppm 0.69 (t, 3 H) 0.79 - 0.94 (m, 2 H) 1.17 (t, J=7.03 Hz, 3 H) 1.57 - 1.74 (m, 2 H) 3.50 (s, 2 H) 4.06 (t, J=6.45 Hz, 2 H) 4.09 - 4.17 (m, 3 H) 7.40 - 7.51 (m, 4 H) 7.63 - 7.77 (m, 4 H) 7.94 (d, 2 H).

### EXAMPLE 38

### N-[2-(4-{5-[butyl(2-chlorobenzoyl)amino]-1,3,4-thiadiazol-2-yl}-2,6-dimethylphenoxy)ethyl]-beta-alanine

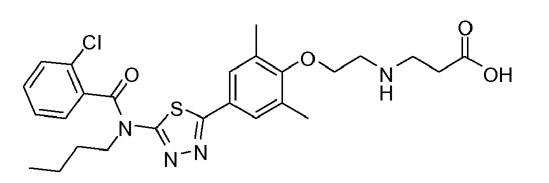

Obtained (11 %) from the title compound of Preparation 46 and 3-aminopropanoic acid following the experimental procedure of Example 1. The hydrochloride salt was done in dioxane with HCl 4M in dioxane.
LRMS: m/z 532 (M+1)⁺
Retention time: 14.20 min (method C)
¹H NMR (400 MHz, DMSO-d6) δ ppm 0.69 (t, J=7.23 Hz, 3 H) 1.09 - 1.19 (m, 2 H) 1.61 - 1.71 (m, 2 H) 2.36 (s, 6 H) 2.77 (t, J=7.23 Hz, 2 H) 3.24 - 3.33 (m, 2 H) 3.40 - 3.47 (m, 2 H) 4.07 (t, 4 H) 7.54 - 7.66 (m, 2 H) 7.66 - 7.79 (m, 4 H).

### EXAMPLE 39

### 1-{4-[5-(butyl{[(4-fluorophenyl)amino]carbonyl}amino)-1,3,4-thiadiazol-2-yl]-benzyl}azetidine-3-carboxylic acid

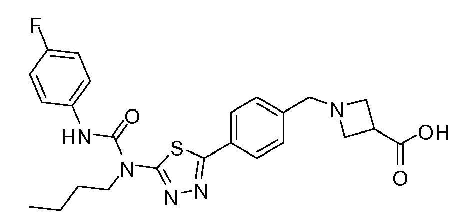

Obtained (2%) from the title compound of Preparation 41 and azetidine-3-carboxylic acid following the experimental procedure of Preparation 31
LRMS: m/z 484 (M+1)⁺
Retention time: 12.95 min (method C)
¹H NMR (200 MHz, DMSO-d6) δ 0.95 (t, J=7.22 Hz, 3 H) 1.40 (q, 2 H) 1.71 (q, 2 H) 3.14 - 3.23 (m, 5 H) 3.59 (s, 2 H) 4.35 (t, 2 H) 7.21 (t, J=8.78 Hz, 2 H) 7.40 (d, J=8.20 Hz, 2 H) 7.46 - 7.61 (m, 2 H) 7.84 (d, J=7.81 Hz, 2 H) 9.47 (s, 1 H).

### EXAMPLE 40

### 1-(4-{5-[(anilinocarbonyl)(butyl)amino]-1,3,4-thiadiazol-2-yl}benzyl)azetidine-3-carboxylic acid

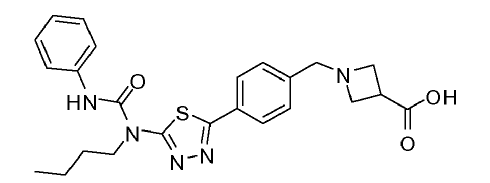

Obtained (15%) from the title compound of Preparation 42 and azetidine-3-carboxylic acid following the experimental procedure of Preparation 31
LRMS: m/z 466 (M+1)⁺
Retention time: 12.84 min (method C)
¹H NMR (200 MHz, DMSO-d6) δ ppm 0.95 (t, J=7.22 Hz, 3 H) 1.41 (q, 2 H) 1.62 - 1.82 (m, 2 H) 3.14 - 3.30 (m, 3 H) 3.32 - 3.49 (m, 2 H) 3.53 - 3.65 (m, 2 H) 4.36 (t, 2 H) 7.17 (t, 1 H) 7.28 - 7.47 (m, 4 H) 7.54 (d, 2 H) 7.85 (d, J=7.42 Hz, 2 H) 9.44 (s, 1H).

### EXAMPLE 41

### 1-{4-[5-(butyl{[(2-chloropyridin-3-yl)amino]carbonyl}amino)-1,3,4-thiadiazol-2-yl]benzyl}azetidine-3-carboxylic acid

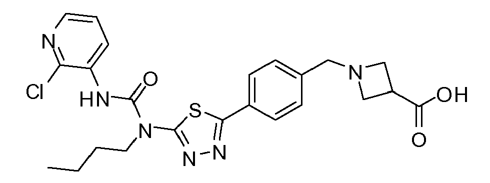

Obtained (41%) from the title compound of Preparation 40 and azetidine-3-carboxylic acid following the experimental procedure of Preparation 31
LRMS: m/z 502 (M+1)⁺
Retention time: 12.24 min (method C)
¹H NMR (400 MHz, DMSO-d6) δ ppm 0.97 (t, J=7.46 Hz, 3 H) 1.37 - 1.50 (m, J=14.88, 7.39, 7.39, 7.26 Hz, 2 H) 1.81 (ddd, J=15.14, 7.46, 7.26 Hz, 2 H) 3.18 - 3.25 (m, 3 H) 3.56 - 3.62 (m, 3 H) 4.33 (t, 2 H) 7.40 (d, J=8.29 Hz, 2 H) 7.47 - 7.54 (m, 1 H) 7.83 (d, J=8.29 Hz, 2 H) 8.06 (dd, J=7.88, 1.66 Hz, 1 H) 8.29 - 8.34 (m, 1 H).

### EXAMPLE 42

### N-[2-(4-{5-[butyl(2-methylbenzoy)amino]-1,3,4-thiadiazol-2-yl}-2,6-dimethylphenoxy)ethyl]-N-methylglycine

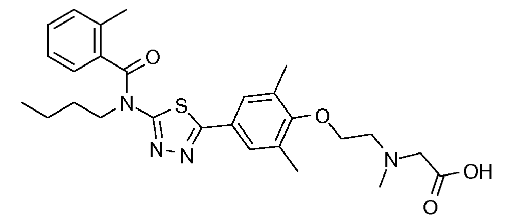

Obtained (60%) from the title compound of Preparation 44 and 2-(methylamino)acetic acid following the experimental procedure of Example 1.
LRMS: m/z 511 (M+1)⁺
Retention time: 16.13 min (method C)
¹H NMR (400 MHz, DMSO-d6) δ ppm 0.69 (t, 3 H) 0.96 - 1.29 (m, 2 H) 1.51 - 1.80 (m, 2 H) 2.26 (s, 3 H) 2.36 (s, 6 H) 3.02 (s, 3 H) 3.61 - 3.75 (m, 2 H) 3.61 - 4,02 (m, 2 H) 4.17 - 4.33 (m, 4 H) 7.18 - 7.62 (m, 4 H) 7.73 (s, 2 H)

### EXAMPLE 43

### 1-[2-(4-{5-[butyl(2-methylbenzoyl)amino]-1,3,4-thiadiazol-2-yl}-2,6-dimethylphenoxy)ethyl]proline

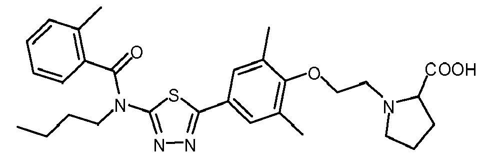

Obtained (94%) from the title compound of Preparation 44 and R,S-proline following the experimental procedure of Example 1.
LRMS: m/z 537 (M+1)⁺
Retention time: 16.28 min (method C)
¹H NMR (400 MHz, DMSO-d6) δ ppm 0.69 (t, 3 H) 1.03 - 1.21 (m, 2 H) 1.55 - 1.71 (m, 2 H) 1.90 - 2.03 (m, 1 H) 2.02 - 2.18 (m, 2 H) 2.26 (s, 3 H) 2.35 (s, 6 H) 3.25 - 3.48 (m, 3 H) 3.73 - 3.90 (m, 3 H) 4.11 - 4.27 (m, 3 H) 4.41 - 4.55 (m, 1 H) 7.34 - 7.44 (m, 2 H) 7.49 (dd, 2 H) 7.72 (s, 2 H)

### EXAMPLE 44

### 1-[2-(4-{5-[butyl(2-methylbenzoyl)amino]-1,3,4-thiadiazol-2-yl}-2,6-dimethylphenoxy)ethyl]piperidine-4-carboxylic acid

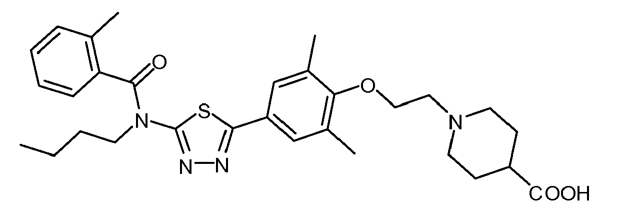

Obtained (50%) from the title compound of Preparation 44 and isonipecotic acid following the experimental procedure of Example 1.
LRMS: m/z 551 (M+1)⁺
Retention time: 14.22 min (method C)
¹H NMR (400 MHz, DMSO-d6) δ ppm 0.69 (t, 3 H) 1.04 - 1.21 (m, 2 H) 1.56 - 1.71 (m, 2 H) 1.86 - 2.02 (m, 2 H) 2.03 - 2.17 (m, 3 H) 2.26 (s, 3 H) 2.37 (s, 6 H) 3.03 - 3.17 (m, 2 H) 3.45 - 3.64 (m, 4 H) 3.63 - 3.74 (m, 2 H) 4.18 - 4.31 (m, 2 H) 7.34 - 7.43 (m, 2 H) 7.44 - 7.54 (m, 2 H) 7.72 (s, 2 H) 10.63 (s, 1 H)

### EXAMPLE 45

### N-[2-(4-{5-[butyl(2-chlorobenzoyl)amino]-1,3,4-thiadiazol-2-yl}-2,6-dimethylphenoxy)ethyl]-N-methylglycine

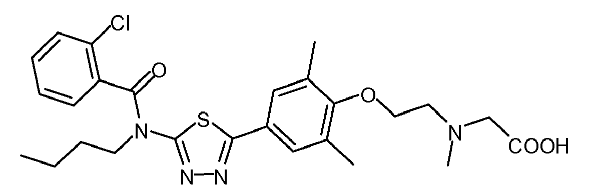

Obtained (34%) from the title compound of Preparation 46 and 2-(methylamino)acetic acid following the experimental procedure of Example 1.
LRMS: m/z 532 (M+1)⁺
Retention time: 15.97 min (method C)
¹H NMR (400 MHz, DMSO-d6) δ ppm 0.69 (t, J=7.24 Hz, 3 H) 1.05 - 1.22 (m, 2 H) 1.56 - 1.78 (m, 2 H) 2.35 (s, 6 H) 2.98 (s, 3 H) 3.59 - 3.70 (m, 2 H) 3.73 - 4.18 (m, 2 H) 4.16 - 4.29 (m, 4 H) 7.52 - 7.66 (m, 2 H) 7.66 - 7.83 (m, 4 H)

### EXAMPLE 46

### 1-[2-(4-{5-[butyl(2-chlorobenzoyl)amino]-1,3,4-thiadiazol-2-yl}-2,6-dimethylphenoxy)ethyl]proline

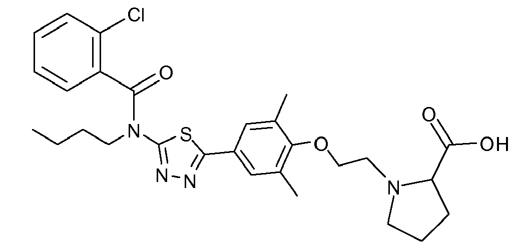

Obtained (82%) from the title compound of Preparation 46 and *R*,S-proline following the experimental procedure of Example 1.
LRMS: m/z 558 (M+1)⁺
Retention time: 16.16 min (method C)
¹H NMR (400 MHz, DMSO-d6) δ ppm 0.69 (t, 3 H) 1.09 - 1.21 (m, 2 H) 1.56 - 1.78 (m, 2 H) 1.88 - 2.02 (m, 1 H) 2.03 - 2.17 (m, 2 H) 2.35 (s, 6 H) 3.27 - 3.46 (m, 3 H) 3.72 - 3.90 (m, 3 H) 4.06 - 4.24 (m, 3 H) 4.38 - 4.50 (m, 1 H) 7.53 - 7.66 (m, 2 H) 7.67 - 7.79 (m, 4 H)

### EXAMPLE 47

### 1-[2-(4-{5-[butyl(2-chlorobenzoyl)amino]-1,3,4-thiadiazol-2-yl}-2,6-dimethylphenoxy)ethyl]piperidine-4-carboxylic acid

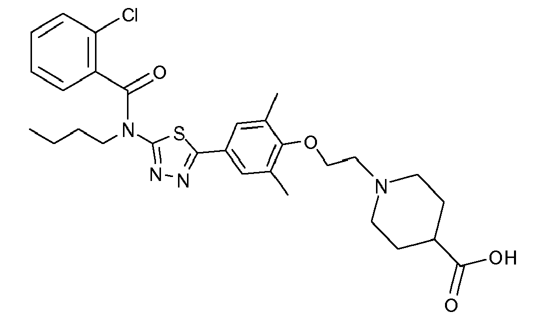

Obtained (51%) from the title compound of Preparation 46 and isonipecotic acid following the experimental procedure of Example 1.
LRMS: m/z 572 (M+1)⁺
Retention time: 14.17 min (method C)
¹H NMR (400 MHz, DMSO-d6) δ ppm 0.69 (t, 3 H) 1.09 - 1.21 (m, 2 H) 1.55 - 1.76 (m, 2 H) 1.82 - 2.01 (m, 2 H) 2.03 - 2.19 (m, 3 H) 2.37 (s, 6 H) 3.04 - 3.18 (m, 2 H) 3.47 - 3.64 (m, 4 H) 3.64 - 3.74 (m, 2 H) 4.17 - 4.29 (m, 2 H) 7.53 - 7.65 (m, 2 H) 7.66 - 7.80 (m+s, 4 H) 10.37 (s, 1 H)

### EXAMPLE 48

### 1-[2-(4-{5-[butyl(3-chloro-2-fluorobenzoyl)amino]-1,3,4-thiadiazol-2-yl}-2,6-dimethylphenoxy)ethyl]piperidine-4-carboxylic acid

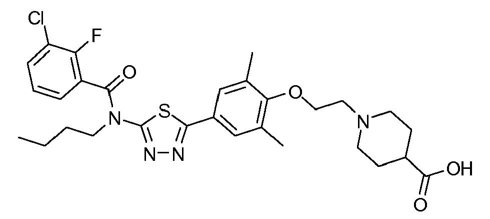

Obtained (48%) from the title compound of Preparation 48 and isonipecotic acid following the experimental procedure of Example 1.
LRMS: m/z 590 (M+1)⁺
Retention time: 14.53 min (method C)
¹H NMR (400 MHz, DMSO-d6) δ ppm 0.72 (t, 3 H) 1.11 - 1.23 (m, 2 H) 1.58 - 1.72 (m, 2 H) 1.81 - 1.97 (m, 2 H) 2.05 - 2.19 (m, 3 H) 2.30 - 2.40 (m, 6 H) 3.03 - 3.20 (m, 2 H) 3.43 - 3.55 (m, 2 H) 3.68 - 3.75 (m, 2 H) 3.98 - 4.10 (t, 2 H) 4.15 - 4.26 (m, 2 H) 7.41 - 7.50 (m, 1 H) 7.69 - 7.79 (m, 3 H) 7.82 - 7.91 (m, 1 H) 10.11 (s, 1 H)

### EXAMPLE 49

### 1-[2-(4-{5-[butyl(2-fluorobenzoyl)amino]-1,3,4-thiadiazol-2-yl}phenoxy)ethyl]-azetidine-3-carboxylic acid

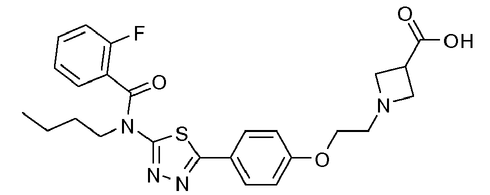

Obtained (6%) from the title compound of Preparation 53 and azetidinecarboxylic acid following the experimental procedure of Example 1.
LRMS: m/z 499 (M+1)⁺
Retention time: 13.14 min (method C)
¹H NMR (400 MHz, DMSO-d6) δ ppm 0.70 (s, 3H), 1.09 (m, 2H), 1.65 (m, 2H), 3.61 (m, 4H), 4.02 (m, 3H), 4.23 (m, 2H), 7.14 (t, 2H), 7.45 (m, 2H), 7.71 (d, J = 18.3 Hz, 2H), 7.98 (d, J = 6.9 Hz, 2H).

### EXAMPLE 50

### 1-[2-(4-{5-[butyl(2-fluorobenzoyl)amino]-1,3,4-thiadiazol-2-yl}phenoxy)ethyl]-piperidine-4-carboxylic acid

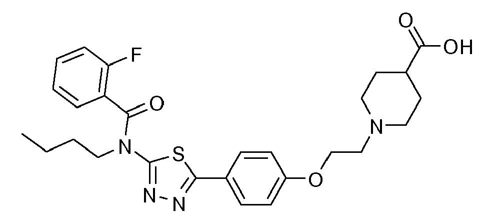

Obtained (17%) from the title compound of Preparation 53 and isonipecotic acid following the experimental procedure of Example 1.
LRMS: m/z 527 (M+1)⁺
Retention time: 12.00 min (method C)
¹H NMR (400 MHz, DMSO-d6) δ ppm 0.70 (t, *J*=7.23 Hz, 3 H) 1.05 - 1.22 (m, 2 H) 1.56 - 1.71 (m, 2 H) 1.74 - 1.90 (m, 2 H) 2.07 (s, 3 H) 3.00 - 3.20 (m, 2 H) 3.24 - 3.38 (m, 2 H) 3.57 - 3.68 (m, 2 H) 3.98 - 4.11 (m, 2 H) 4.38 - 4.53 (m, 2 H) 7.18 (d, *J*=7.82 Hz, 2 H) 7.38 - 7.52 (m, 2 H) 7.62 - 7.78 (m, 2 H) 7.99 (d, *J*=8.60 Hz, 2 H)

### EXAMPLE 51

### 1-[2-(4-{5-[butyl(2,4-difluorobenzoyl)amino]-1,3,4-thiadiazol-2-yl}-2,6-dimethylphenoxy)ethyl]piperidine-4-carboxylic acid

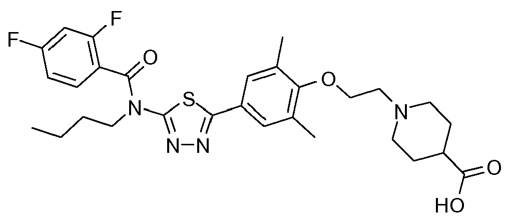

To a 50°C stirred solution of the title compound of Preparation 55 (120 mg, 0.28 mmol) in DMF (10 ml), 60% NaH (25 mg, 0.63 mmol) was added and the mixture was stirred at that temperature for 30 min. Then a solution of 2,4-difluorobenzolyl chloride (75 µl, 0.61 mmol) in THF (1 ml) was added drop wise and the final mixture was stirred at rt for 1.5 h. 2N HCl (10 ml) was slowly added and the final mixture was portioned between water and ethyl acetate. The aqueous layer was extracted with ethyl acetate and the combined organic layers were washed with water and brine. It was dried and solvent removed to yield a crude product that was purified according to purification method A to yield the title compound as a solid. 28 mg (18% yield) of the title product were obtained as a solid.
LRMS: m/z 573 (M+1)⁺
Retention time: 13.37 min (method C)
¹H NMR (400 MHz, DMSO-d6) δ ppm 0.89 (t, 3 H) 1.27 - 1.41 (m, 2 H) 1.45 - 1.60 (m, 4 H) 1.76 (dd, 2 H) 1.99 - 2.11 (m, 2 H) 2.10 - 2.21 (m, 1 H) 2.25 (s, 6 H) 2.64 (t, 2 H) 2.87 (d, 2 H) 3.22 - 3.32 (m, 2 H) 3.83 (t, 2 H) 7.39 (s, 2 H) 7.86 (t, 1 H) 8.29 (s, 1 H)

### EXAMPLE 52

### 1-[2-(4-{5-[butyl(2-methoxybenzoyl)amino]-1,3,4-thiadiazol-2-yl}-2,6-dimethylphenoxy)ethyl]piperidine-4-carboxylic acid

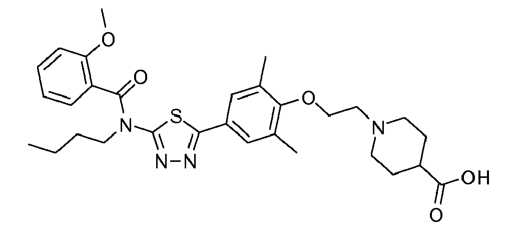

Obtained (20%) from the title compound of Preparation 55 and 2-methoxybenzoyl chloride following the experimental procedure of Example 51.
LRMS: m/z 567 (M+1)⁺
Retention time: 13.08 min (method C)
¹H NMR (400 MHz, DMSO-d6) δ 0.68 (t, 3 H) 1.03 - 1.19 (m, 2 H) 1.52 - 1.69 (m, 2 H) 1.91 (s, 1 H) 2.04 - 2.18 (m, 2 H) 2.37 (s, 6 H) 3.10 (d, 2 H) 3.47 - 3.75 (m, 8 H) 3.84 (s, 3 H) 4.17 - 4.26 (m, 2 H) 7.12 (t, 1 H) 7.23 (d, 1 H) 7.47 (dd, 1 H) 7.52 - 7.61 (m, 1 H) 7.72 (s, 2 H) 10.12 10.34 (m, 1 H)

### EXAMPLE 53

### 1-[2-(4-{5-[butyl(phenylacetyl)amino]-1,3,4-thiadiazol-2-yl}-2,6-dimethylphenoxy)-ethyl]piperidine-4-carboxylic acid

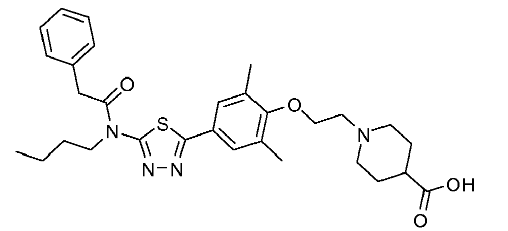

Obtained (10%) from the title compound of Preparation 55 and 2-phenylacetyl chloride following the experimental procedure of Example 51
LRMS: m/z 551 (M+1)⁺
Retention time: 13.49 min (method C)
¹H NMR (400 MHz, DMSO-d6) δ ppm 0.93 (t, 3 H) 1.32 - 1.46 (m, 2 H) 1.62 - 1.75 (m, 2 H) 1.82 - 1.97 (m, 2 H) 2.02 - 2.17 (m, 3 H) 2.29 - 2.39 (s, 6 H) 3.09 (d, 2 H) 3.45 - 3.56 (m, 2 H) 3.63 - 3.74 (m, 2 H) 4.12 - 4.23 (m+s, 4 H) 4.23 - 4.33 (m, 2 H) 7.24 - 7.41 (m, 5 H) 7.64 (s, 2 H) 10.31 (s, 1 H)

### EXAMPLE 54

### 1-[2-(4-{5-[butyl(2,3-dimethylbenzoyl)amino]-1,3,4-thiadiazol-2-yl}-2,6-dimethylphenoxy)ethyl]piperidine-4-carboxylic acid

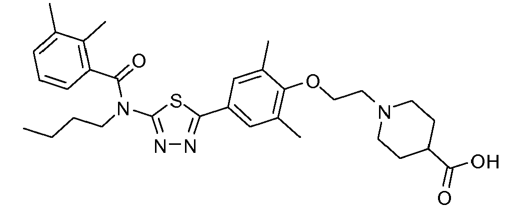

Obtained (24%) from the title compound of Preparation 55 and 2,3-dimethylbenzoyl chloride following the experimental procedure of Example 51.
LRMS: m/z 565 (M+1)⁺
Retention time: 14.33 min (method C)
¹H NMR (400 MHz, DMSO-d6) δ ppm 0.66 (t, 3 H) 1.02 - 1.18 (m, 2 H) 1.44 - 1.70 (m, 5 H) 1.76 (m, 2 H) 1.99 - 2.21 (m+s, 4+3 H) 2.29 (s, 3 H) 2.30 (s, 6 H) 2.66 (t, 2 H) 2.88 (d, 2 H) 3.83 - 3.93 (m, 2 H) 7.19 - 7.36 (m, 3 H) 7.65 (s, 2 H)

### EXAMPLE 55

### 1-[2-(4-{5-[butyl(2-chloro-3-fluorobenzoyl)amino]-1,3,4-thiadiazol-2-yl}-2,6-dimethylphenoxy)ethyl]piperidine-4-carboxylic acid

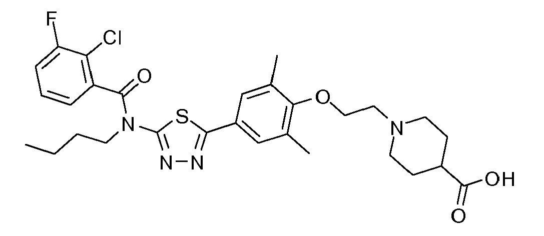

Obtained (1%) from the title compound of Preparation 55 and 2-chloro-3-fluorobenzoyl chloride following the experimental procedure of Example 51.
LRMS: m/z 590 (M+1)⁺
Retention time: 13.93 min (method C)
¹H NMR (400 MHz, DMSO-d6) δ ppm 0.71 (t, 3 H) 1.10 - 1.25 (m, 2 H) 1.57 - 1.76 (m, 2 H) 1.84 - 2.00 (m, 2 H) 2.04 - 2.17 (m, 3 H) 2.32 - 2.42 (s, 6 H) 3.03 - 3.16 (m, 2 H) 3.32 - 3.43 (m, 2 H) 3.49 - 3.59 (m, 2 H) 3.64 - 3.75 (m, 2 H) 4.16 - 4.28 (m, 2 H) 7.60 - 7.71 (m, 3 H) 7.75 (s, 2 H)

### EXAMPLE 56

### N-butyl-2-chloro-N-{5-[3,5-dimethyl-4-(piperidin-4-ylmethoxy)phenyl]-1,3,4-thiadiazol-2-yl}benzamide

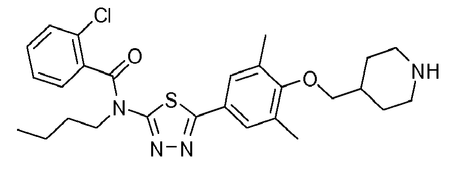

To a stirred suspension tert-butyl 4-((4-(5-(butylamino)-1,3,4-thiadiazol-2-yl)-2,6-dimethylphenoxy)methyl)piperidine-1-carboxylate (Preparation 58) (100 mg, 0.21 mmol) in THF (2 ml), 60% NaH (18 mg, 0.45 mmol) was added and the mixture was stirred at that temperature for 30 min. Then a solution of 2-chlorobenzoyl chloride (81 mg, 0.46 mmol) in THF (1 ml) was added drop wise and the final mixture was stirred at rt for 1.5 h. 2N HCl (50 ml) was slowly added and the final mixture was portioned between water and ethyl acetate. The aqueous layer was extracted with ethyl acetate and the combined organic layers were washed with water and brine. The residue thus obtained was dissolved in HCl-dioxane 4N (1 ml) and let to react at room temperature during 1 h. Solvent was removed to yield a crude product that was purified according to purification method A to yield the title compound 79 mg (72% yield) as a solid.
LRMS: m/z 514 (M+1)⁺
Retention time: 14.01 min (method C)
¹H NMR (200 MHz, DMSO-d⁶) δ ppm 0.69 (t, *J*=7.23 Hz, 3 H) 1.07 - 1.20 (m, 2 H) 1.39 - 1.53 (m, 2 H) 1.56 - 1.76 (m, 2 H) 1.84 - 1.94 (m, 2 H) 1.94 - 2.06 (m, 1 H) 2.23 - 2.35 (m, 6 H) 2.75 (t, 2 H) 3.19 (d, *J*=12.11 Hz, 2 H) 3.25 - 3.46 (m, 2 H) 3.67 (d, *J*=5.67 Hz, 2 H) 7.56 (t, *J*=7.23 Hz, 1 H) 7.62 (t, 1 H) 7.66 - 7.72 (m+s, 3 H) 7.76 (d, *J*=6.25 Hz, 1 H) 8.38 (s, 1 H)

### EXAMPLE 57

### N-butyl-2-fluoro-N-[5-(4-{2-[(2-hydroxyethyl)(methyl)amino]ethoxy}-3,5-dimethylphenyl)-1,3,4-thiadiazol-2-yl]benzamide

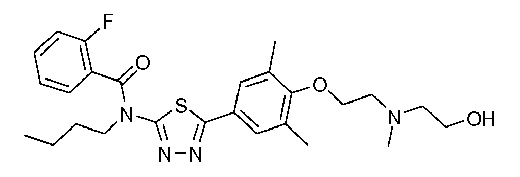

To a stirred suspension N-butyl-N-(5-(3,5-dimethyl-4-(2-oxoethoxy)phenyl)-1,3,4-thiadiazol-2-yl)-2-fluorobenzamide (Preparation 6) (300 mg, 0.68 mmol) and 2-methylamino)ethanol (62 mg, 0.83 mmol) in methanol (30ml), AcOH (350 µL, 6.12 mmol) was added and the mixture was stirred at rt for 1h. Then, NaCNBH3 (25 mg, 0.40 mmol) was added portion wise and the final mixture was stirred at room temperature overnight. The solvent was removed in vacuum and the crude mixture thus obtained was purified according to purification method A. The pure compound thus obtained was dissolved in dioxane (3 ml) and HCl-dioxane 4N (2 ml) was added to precipitate the hydrochloride; then filtered and washed with diethyl ether rendering the title compound as a white solid (133 mg, yield=36%).
LRMS: m/z 501 (M+1)⁺
Retention time: 13.12 min (method C)
¹H NMR (200 MHz, DMSO-d6) δ ppm 0.70 (t, 3 H) 0.99 - 1.25 (m, 2 H) 1.50 - 1.77 (m, 2 H) 2.36 (s, 6 H) 2.94 (s, 3 H) 3.25 - 3.35 (m, 2 H) 3.48 - 3.70 (m, 2 H) 3.71 - 3.91 (m, 2 H) 3.92 - 4.12 (m, 2 H) 4.12 - 4.32 (m, 2 H) 5.38 (s, 1 H) 7.34 - 7.56 (m, 2 H) 7.58 - 7.82 (m, 4 H)

### EXAMPLE 58

### N-(5-{4-[2-(acetylamino)ethoxy]-3,5-dimethylphenyl}-1,3,4-thiadiazol-2-yl)-N-butyl-2-fluorobenzamide

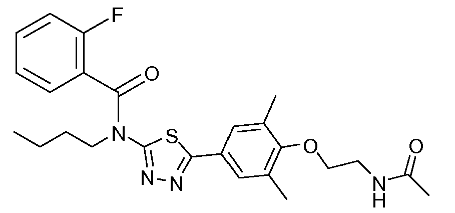

To a stirred suspension N-(2-(4-(5-(butylamino)-1,3,4-thiadiazol-2-yl)-2,6-dimethylphenoxy)ethyl)acetamide (Preparation 30) (130 mg, 0.36 mmol) in DCM (5 ml) were added DIEA (240 µl, 1.38 mmol) and DMAP (22 mg, 0.18 mmol). After 5 min, 2-fluorobenzoylchloride (68 mg, 0.43 mmol) solved in DCM (2 ml) was added to the reaction mixture and let react overnight. Water was added to the reaction mixture and the aqueous layer was extracted with ethyl acetate and the combined organic layers were washed with water and brine. The crude mixture thus obtained was purified according to purification method A to yield the title compound as a solid (43 mg, yield=25%).
LRMS: m/z 485 (M+1)⁺
Retention time: 13.07 min (method C)
¹H NMR (200 MHz, DMSO-d6) δ ppm 0.86 - 0.98 (m, 3 H) 1.31 - 1.46 (m, 2 H) 1.52 - 1.65 (m, 2 H) 1.74 - 1.82 (s, 3 H) 2.20 (s, 6 H) 3.03 - 3.14 (t, 2 H) 3.40 (m, 2 H) 3.99 (t, 2 H) 7.42 - 7.54 (m+s, 4 H) 7.78 - 7.87 (m, 1 H) 8.04 (t, 1 H) 8.16 (t, 1 H)

### EXAMPLE 59

### 3-[(4-{5-[butyl(2-fluorobenzoyl)amino]-1,3,4-thiadiazol-2-yl}benzoyl)amino]-2-hydroxypropanoic acid

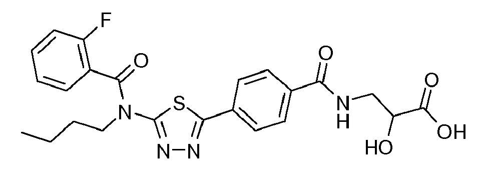

To a stirred solution of 4-(5-(N-butyl-2-fluorobenzamido)-1,3,4-thiadiazol-2-yl)benzoic acid (Preparation 61) (150 mg, 0.38 mmol) in DMF (4 ml) were added HOBt (51 mg, 0.38 mmol) and EDC (72 mg, 0.38 mmol). After 30 min, 3-amino-2-hydroxypropanoic acid (40 mg, 0.38 mmol) solved in DMF (2 ml) was added to the reaction mixture and let react overnight. The crude was added over water and the aqueous layer was extracted with ethyl acetate and the combined organic layers were washed with water and brine. The crude mixture thus obtained was purified according to purification method A to yield the title compound as a solid (3.6 mg, yield=2%).
LRMS: m/z 487(M+1)⁺
Retention time: 15.91 min (method C)
¹H NMR (200 MHz, DMSO-d6) δ ppm 0.70 (t, 3 H) 1.07 - 1.27 (m, 2 H) 1.54 - 1.77 (m, 2 H) 3.34 (br. s., 3 H) 3.68 (br. s., 1 H) 4.00 - 4.17 (m, 2 H) 7.37 - 7.55 (m, 2 H) 7.61 - 7.81 (m, 2 H) 7.91 - 8.17 (m, 4 H) 8.76 (br. s., 1 H)

### EXAMPLE 60

### 1-(4-{5-[butyl(2-fluorobenzoyl)amino]-1,3,4-thiadiazol-2-yl}benzoyl)azetidine-3-carboxylic acid

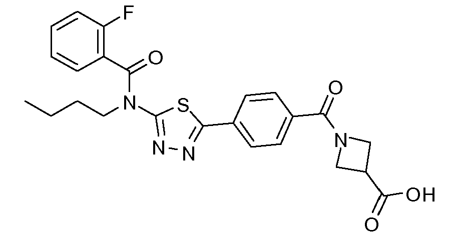

To a stirred solution of 4-(5-(N-butyl-2-fluorobenzamido)-1,3,4-thiadiazol-2-yl)benzoic acid (Preparation 61) (100 mg, 0.25 mmol) in DMF (2 ml) were added HOBt (34 mg, 0.25 mmol) and EDC (48 mg, 0.25 mmol). After 30 min, azetidine-3-carboxylic acid (25 mg, 0.25 mmol) solved in DMF (2 ml) at 80°, was added to the reaction mixture and let react overnight at 80°. The crude was added over water and after adjusted to pH 3, and then extracted with DCM and the combined organic layers were washed with water and brine. The crude mixture thus obtained was purified according to purification method A to yield the title compound as a solid (20 mg, yield=15%).
LRMS: m/z 483 (M+1)⁺
Retention time: 15.96 min (method C)
¹H NMR (200 MHz, DMSO-d6) δ ppm 0.59 - 0.87 (m, 3 H) 1.19 (m, 2 H) 1.56 - 1.85 (m, 2 H) 3.34 - 3.68 (m, 1 H) 4.01 - 4.25 (m, 2 H) 4.27 - 4.68 (m, 4 H) 7.13 - 7.38 (m, 2 H) 7.39 - 7.62 (m, 2 H) 7.65 - 7.84 (m, 2 H) 7.93 - 8.13 (m, 2 H)

### EXAMPLE 61

### 1-(4-{5-[butyl(2-fluorobenzoyl)amino]-1,3,4-thiadiazol-2-yl}benzyl)azetidine-3-carboxamide

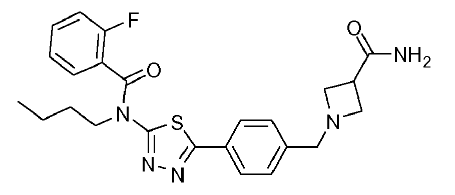

To a stirred solution of 4-(5-(N-butyl-2-fluorobenzamido)-1,3,4-thiadiazol-2-yl)benzoic acid (Preparation 65) (733 mg, 1.56 mmol) in DMF (15 ml) were added HOBt (324 mg, 2.40 mmol) and EDC (460 mg, 240 mmol). After 30 min, ammonium hydroxide (300 µl, 7.62 mmol) solved in DMF (2 ml) was added to the reaction mixture and let react overnight. Solvent was removed to yield a crude mixture that was purified according to purification method A to yield the title compound 36 mg (yield=5%).as a solid.
LRMS: m/z 468 (M+1)⁺
Retention time: 11.17 min (method C)
¹H NMR (200 MHz, DMSO-d6) δ ppm 0.70 (t, J = 7.3 Hz, 3H), 1.26 - 1.03 (m, 2H), 1.76 - 1.55 (m, 2H), 3.16 (q, J = 7.4 Hz, 1 H), 3.36 (d, J = 5.5 Hz, 2H), 3.57 (s, 2H), 3.59 (d, J = 5.2 Hz, 1 H), 4.15 - 3.97 (m, 2H), 7.55 - 7.36 (m, 4H), 7.82 - 7.60 (m, 2H).

### EXAMPLE 62

### (1S,2S)-2-[(4-{5-[butyl(phenylacetyl)amino]-1,3,4-thiadiazol-2-yl}benzyl)amino]-cyclohexanecarboxylic acid

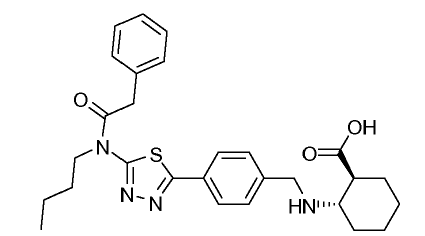

Obtained (1%) from the title compound of Preparation 22 and (1S, 2S)-2-aminocyclohexanecarboxylic acid following the experimental procedure of Example 1.
LRMS: m/z 507 (M+1)⁺
Retention time: 14.74 min (method C)

### EXAMPLE 63

### N-butyl-2-fluoro-N-(5-{4-[({2-[(methylsulfonyl)amino]ethyl}amino)methyl]phenyl}-1,3,4-thiadiazol-2-yl)benzamide

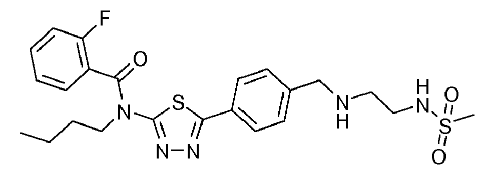

Obtained (19%) from the title compound of Preparation 64 and N-(2-aminoethyl)-methanesulfonamide hydrochloride (Preparation 67) following the experimental procedure of Example 1.
LRMS: m/z 506 (M+1)⁺
Retention time: 11.66 min (method C)
¹H NMR (200 MHz, DMSO-d6) δ ppm 0.70 (t, *J*=7.24 Hz, 3 H) 1.10 - 1.21 (m, 2 H) 1.59 - 1.72 (m, 2 H) 2.66 (t, *J*=6.65 Hz, 2 H) 2.91 (s, 3 H) 3.07 (t, *J*=5.87 Hz, 2 H) 3.80 (s, 2 H) 4.01 - 4.12 (m, 2 H) 6.98 (br. s., 1 H) 7.39 - 7.50 (m, 2 H) 7.53 (d, *J*=8.61 Hz, 2 H) 7.63 - 7.78 (m, 2 H) 7.95 (d, *J*=8.22 Hz, 2 H) 8.17 (s, 1 H)

### EXAMPLE 64

### 1-(4-{5-[butyl(2-fluorobenzoyl)amino]-1,3,4-thiadiazol-2-yl}benzyl)-1H-pyrazole-4-carboxylic acid

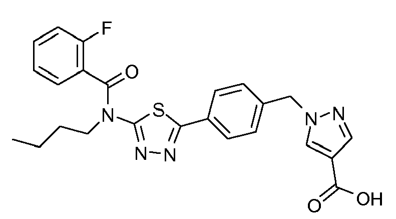

The tert-butyl 1-(4-(5-(N-butyl-2-fluorobenzamido)-1,3,4-thiadiazol-2-yl)benzyl)-1H-pyrazole-4-carboxylate (Preparation 72) was suspended in HCl 4N (2 ml) and let to react at rt overnight. Solvent was removed to yield a crude product that was purified according to purification method A to yield the title compound 13 mg (32% yield) as a solid.
LRMS: m/z 480 (M+1)⁺
Retention time: 16.51 min (method C)
¹H NMR (200 MHz, DMSO-d6) δ ppm 0.70 (t, J=7.42 Hz, 3 H) 1.04 - 1.21 (m, 2 H) 1.58 - 1.72 (m, 2 H) 3.97 - 4.11 (m, 2 H) 5.46 (s, 2 H) 7.36 - 7.53 (m, 4 H) 7.60 - 7.70 (m, 2 H) 7.84 (s, 1 H) 7.99 (d, J=7.82 Hz, 2 H) 8.42 (s, 1 H)

### EXAMPLE 65

### 1-[2-(4-{5-[butyl(2-chlorobenzoyl)amino]-1,3,4-thiadiazol-2-yl}-2,6-dimethylphenoxy)ethyl]pyrrolidine-3-carboxylic acid

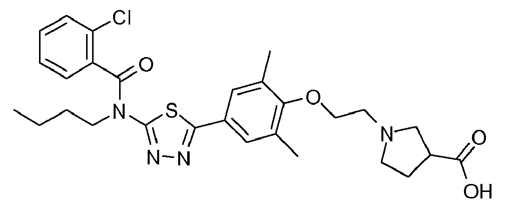

Obtained (34%) from the title compound of Preparation 46 and pyrrolidine-3-carboxylic acid following the experimental procedure of Example 1.
LRMS: m/z 558 (M+1)⁺
Retention time: 14.18 min (method C)
¹H NMR (200 MHz, DMSO-d6) δ ppm 0.70 (t, 3 H) 1.10 - 1.21 (m, 2 H) 1.56 - 1.71 (m, 2 H) 2.20 - 2.32 (m, 1 H) 2.37 (s, 6 H) 3.17 - 3.34 (m, 2 H) 3.35 - 3.53 (m, 2 H) 3.60 - 3.79 (m, 3 H) 3.82 - 3.96 (m, 1 H) 3.99 - 4.11 (t, 2 H) 4.11 - 4.24 (t, 2 H) 7.40 - 7.52 (m, 2 H) 7.63 - 7.78 (s+m, 4 H)

### EXAMPLE 66

### {1-[2-(4-{5-[butyl(2-chlorobenzoyl)amino]-1,3,4-thiadiazol-2-yl}-2,6-dimethylphenoxy)ethyl]piperidin-4-yl}acetic acid

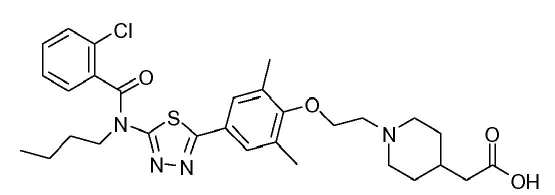

Obtained (31%) from the title compound of Preparation 46 and 2-(piperidin-4-yl)acetic acid following the experimental procedure of Example 1.
LRMS: m/z 586 (M+1)⁺
Retention time: 13.62 min (method C)
¹H NMR (200 MHz, DMSO-d6) δ ppm 0.70 (t, J=7.42 Hz, 3 H) 1.07 - 1.19 (m, 2 H) 1.49 - 1.59 (m, 2 H) 1.59 - 1.70 (m, 2 H) 1.65 (m, J=14.65, 7.62, 7.42 Hz, 1 H) 1.85 - 1.97 (m, 2 H) 2.17 - 2.27 (m, 0 H) 2.36 (s, 6 H) 3.03 - 3.15 (m, 2 H) 3.50 - 3.58 (m, 2 H) 3.60 - 3.72 (m, 2 H) 4.00 - 4.10 (m, 2 H) 4.15 - 4.23 (m, 2 H) 7.36 - 7.53 (m, 2 H) 7.62 - 7.78 (m, 4 H)

### EXAMPLE 67

### 1-[2-(4-{5-[butyl(2-chlorobenzoyl)amino]-1,3,4-thiadiazol-2-yl}-2,6-dimethylphenoxy)ethyl]-4-methylpiperidine-4-carboxylic acid

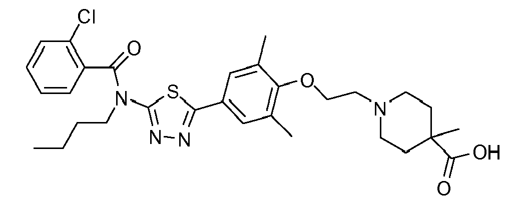

Obtained (35%) from the title compound of Preparation 46 and 4-methylpiperidine-4-carboxylic acid following the experimental procedure of Example 1.
LRMS: m/z 586 (M+1)⁺
Retention time: 13.93 min (method C)
¹H NMR (200 MHz, DMSO-d6) δ ppm 0.69 (t, J=7.23 Hz, 3 H) 1.04 (d, J=5.86 Hz, 3 H) 1.09 - 1.18 (m, 2 H) 1.67 - 1.88 (m, 4 H) 2.24 (d, J=12.90 Hz, 1 H) 2.37 (br. s., 6 H) 2.98 - 3.08 (m, 0 H) 3.58 - 3.67 (m, 4 H) 4.13 - 4.20 (m, 2 H) 7.53 - 7.66 (m, 2 H) 7.66 - 7.81 (m, 4 H)

### EXAMPLE 68

### (1S,2R)-2-{[2-(4-{5-[butyl(2-chlorobenzoyl)amino]-1,3,4-thiadiazol-2-yl}-2,6-dimethylphenoxy)ethyl]amino}cyclopentanecarboxylic acid

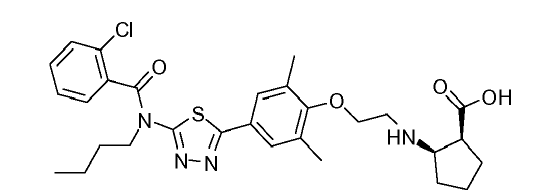

Obtained (44%) from the title compound of Preparation 46 and (1S,2R)-2-aminocyclopentanecarboxylic acid following the experimental procedure of Example 1.
LRMS: m/z 572 (M+1)⁺
Retention time: 15.58 min (method C)
¹H NMR (200 MHz, DMSO-d6) δ ppm 0.69 (t, J=7.42 Hz, 3 H) 1.09 - 1.19 (m, 2 H) 1.57 - 1.73 (m, 3 H) 1.83 (dd, J=17.58, 12.50 Hz, 2 H) 1.93 - 2.03 (m, 2 H) 2.07 - 2.18 (m, 1 H) 2.36 (s, 6 H) 3.11 - 3.19 (m, 1 H) 3.41 (br. s., 2 H) 3.54 - 3.58 (m, 1 H) 3.67 - 3.75 (m, 1 H) 3.77 - 3.87 (m, 1 H) 4.12 (dt, J=11.63, 5.72 Hz, 3 H) 7.57 (t, J=7.42 Hz, 1 H) 7.63 (td, J=7.72, 1.76 Hz, 1 H) 7.67 - 7.79 (m, 4 H)

### EXAMPLE 69

### cis-4-{[2-(4-{5-[butyl(2-chlorobenzoyl)amino]-1,3,4-thiadiazol-2-yl}-2,6-dimethylphenoxy)ethyl]amino}cyclohexanecarboxylic acid

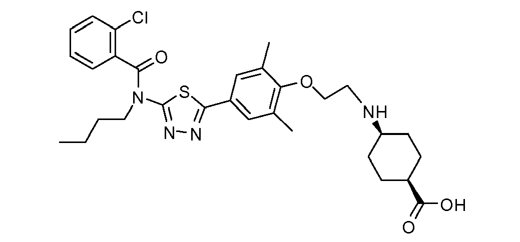

Obtained (8%) from the title compound of Preparation 46 and (1S, 4S)-4-aminocyclohenanecarboxylic acid following the experimental procedure of Example 1.
LRMS: m/z 586 (M+1)⁺
Retention time: 13.87 min (method C)
¹H NMR (200 MHz, DMSO-d6) δ ppm 0.69 (t, J=7.42 Hz, 3 H) 1.05 - 1.22 (m, 2 H) 1.46 - 1.78 (m, 6 H) 1.97 - 2.16 (m, 4 H) 2.37 (s, 6 H) 2.54 - 2.63 (m, 1 H) 3.13 - 3.24 (m, 1 H) 3.74 - 3.88 (m, 1 H) 4.04 - 4.19 (m, 4 H) 7.53 - 7.66 (m, 2 H) 7.67 - 7.81 (m, 4 H) 12.32 (br. s., 1 H)

### EXAMPLE 70

### {1-[2-(4-{5-[butyl(2-chlorobenzoyl)amino]-1,3,4-thiadiazol-2-yl}-2,6-dimethylphenoxy)ethyl]pyrrolidin-3-yl}acetic acid

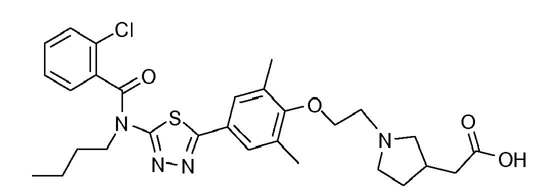

Obtained (21 %) from the title compound of Preparation 46 and 2-(pyrrolidin-3-yl)acetic acid following the experimental procedure of Example 1.
LRMS: m/z 572 (M+1)⁺
Retention time: 13.49 min (method C)
¹H NMR (200 MHz, DMSO-d6) δ ppm 0.69 (t, J=7.42 Hz, 3 H) 1.10 - 1.20 (m, 2 H) 1.56 - 1.76 (m, 4 H) 2.14 - 2.28 (m, 2 H) 2.37 (s, 6 H) 2.62 - 2.73 (m, 1 H) 3.62 (t, J=4.30 Hz, 3 H) 3.81 (br. s., 1 H) 4.07 - 4.21 (m, 4 H) 7.53 - 7.66 (m, 2 H) 7.66 - 7.82 (m, 4 H).

### EXAMPLE 71

### {1-[2-(4-{5-[butyl(2-chlorobenzoyl)amino]-1,3,4-thiadiazol-2-yl}-2,6-dimethylphenoxy)ethyl]piperidin-3-yl}acetic acid

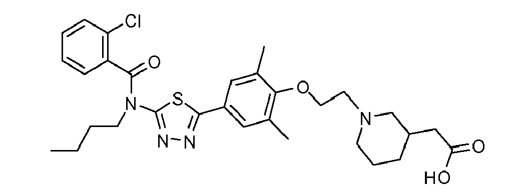

Obtained (13%) from the title compound of Preparation 46 and 2-(piperidin-3-yl)acetic acid following the experimental procedure of Example 1.
LRMS: m/z 586 (M+1)⁺
Retention time: 13.68 min (method C)
¹H NMR (200 MHz, DMSO-d6) δ ppm 0.69 (t, J=7.42 Hz, 3 H) 1.08 - 1.20 (m, 2 H) 1.56 - 1.74 (m, 2 H) 1.75 - 1.93 (m, 3 H) 2.28 (br. s., 2 H) 2.33 (br. s., 1 H) 2.37 (s, 6 H) 2.81 (br. s., 0 H) 2.95 (br. s., 1 H) 3.59 - 3.72 (m, 2 H) 3.73 - 3.86 (m, 1 H) 4.09 - 4.25 (m, 3 H) 7.52 - 7.66 (m, 2 H) 7.67 - 7.81 (m, 4 H)

### EXAMPLE 72

### 1-[2-(4-{5-[butyl(2-chlorobenzoyl)amino]-1,3,4-thiadiazol-2-yl}-2,6-dimethylphenoxy)ethyl]piperidine-2-carboxylic acid

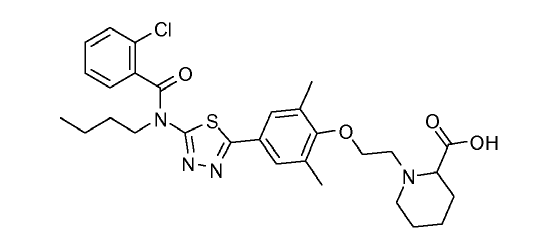

Obtained (52%) from the title compound of Preparation 46 and piperidine-2-carboxylic acid following the experimental procedure of Example 1.
LRMS: m/z 572 (M+1)⁺
Retention time: 16.88 min (method C)
¹H NMR (200 MHz, DMSO-d6) δ ppm 0.70 (t, J=7.22 Hz, 3 H) 1.03 - 1.25 (m, 2 H) 1.39 - 1.59 (m, J=1.56 Hz, 2 H) 1.59 - 1.82 (m, J=9.17, 6.83 Hz, 6 H) 2.32 (s, 6 H) 2.81 - 3.20 (m, 4 H) 3.20 - 3.33 (m, 1 H) 3.92 (t, 2 H) 4.05 (t, 2 H) 7.34 - 7.54 (m, 2 H) 7.59 - 7.80 (m, 4 H)

### EXAMPLE 73

### N-[2-(4-{5-[butyl(2-chlorobenzoyl)amino]-1,3,4-thiadiazol-2-yl}-2,6-dimethylphenoxy)ethyl]glycine

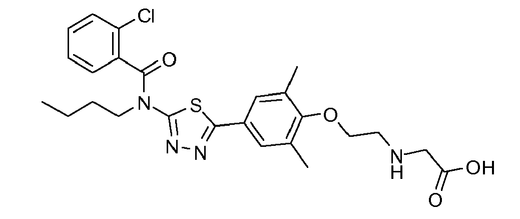

Obtained (4%) from the title compound of Preparation 46 and gycine following the experimental procedure of Example 1.
LRMS: m/z 518 (M+1)⁺
Retention time: 15.88 min (method C)
¹H NMR (200 MHz, DMSO-d6) δ ppm 0.70 (t, J=7.42 Hz, 3 H) 1.14 (m, J=8.20 Hz, 2 H) 1.65 (m, 2 H) 2.33 (s, 6 H) 3.17 (m, 2 H) 3.27 (m, 2 H) 4.00 (m, J=5.47 Hz, 4 H) 7.42 (m, J=7.42 Hz, 2 H) 7.60 - 7.81 (m, 4 H)

### EXAMPLE 74

### 1-[2-(4-{5-[butyl(2-chlorobenzoyl)amino]-1,3,4-thiadiazol-2-yl}-2,6-dimethylphenoxy)ethyl]piperidine-3-carboxylic acid

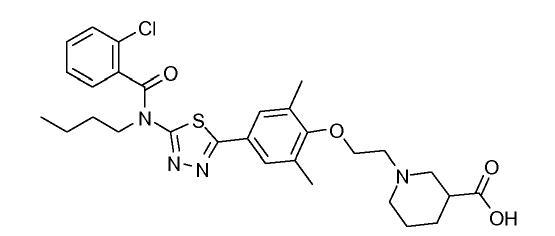

Obtained (55%) from the title compound of Preparation 46 and piperidine-3-carboxylic acid following the experimental procedure of Example 1.
LRMS: m/z 572 (M+1)⁺
Retention time: 16.71 min (method C)
¹H NMR (200 MHz, DMSO-d6) δ ppm 0.69 (t, J=7.42 Hz, 3 H) 1.05 - 1.22 (m, 2 H) 1.51 (br. s., 1 H) 1.68 (br. s., 2 H) 1.91 (br. s., 2 H) 2.06 (br. s., 1 H) 2.37 (s, 6 H) 2.90 - 3.22 (m, 2 H) 3.56 - 3.69 (m, 2 H) 3.82 (br. s., 2 H) 4.06 - 4.33 (m, 3 H) 7.51 - 7.66 (m, 2 H) 7.65 - 7.84 (m, 4 H)

### EXAMPLE 75

### (2S,4S)-4-(4-(5-(N-butyl-2-fluorobenzamido)-1,3,4-thiadiazol-2-yl)-2,6-dimethylphenoxy)pyrrolidine-2-carboxylic acid

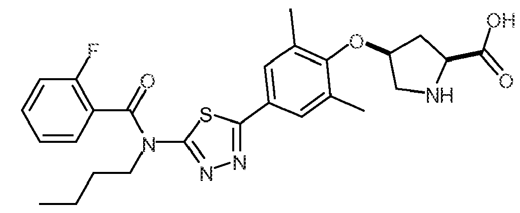

The (2S,4S)-1-tert-butyl 2-methyl 4-(4-(5-(N-butyl-2-fluorobenzamido)-1,3,4-thiadiazol-2-yl)-2,6-dimethylphenoxy)pyrrolidine-1,2-dicarboxylate (Preparation 77) was suspended in HCl 4N (2 ml) and let to react at rt overnight. Solvent was removed to yield a crude product that was purified according to purification method A to yield the title compound 18 mg (10% yield) as a solid.
LRMS: m/z 513 (M+1)⁺
Retention time: 15.24 min (method C)

### EXAMPLE 76

### N-butyl-2-fluoro-N-(5-{4-[2-(glycoloylamino)ethoxy]-3,5-dimethylphenyl}-1,3,4-thiadiazol-2-yl)benzamide

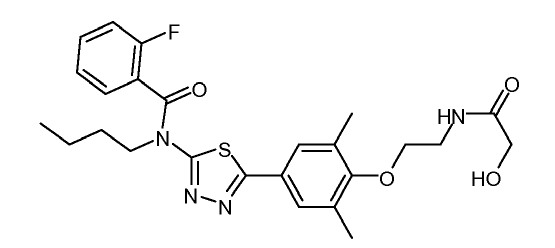

To a stirred solution of N-(5-(4-(2-aminoethoxy)-3,5-dimethylphenyl)-1,3,4-thiadiazol-2-yl)-N-butyl-2-fluorobenzamide (Preparation 79) (60 mg, 0.14 mmol) in DMF (2 ml) were added HOBt (20 mg, 0.15 mmol) DIEA (26 µl, 0.15 mmol) and EDC (29 mg, 0.15 mmol). After 30 min, 2-hydroxyacetic acid (11 mg, 0.15 mmol) solved in DMF (1 ml) was added to the reaction mixture and let react overnight. The crude was added over water and the aqueous layer was extracted with ethyl acetate and the combined organic layers were washed with water and brine. The crude mixture thus obtained was purified according to purification method A to yield the title compound as a solid (49 mg, yield=72%).
LRMS: m/z 501(M+1)⁺
Retention time: 17.10 min (method C)
¹H NMR (200 MHz, DMSO-d6) δ ppm 0.70 (t, J = 7.4 Hz, 3H), 1.14 (dd, J = 14.8, 7.4 Hz, 2H), 1.65 (m, 2H), 7.94 (s, 2H), 2.31 (s, 6H), 3.53 (q, J = 6.1 Hz, 2H), 3.86 (dd, J = 11.7, 5.6 Hz, 4H), 4.05 (m, 2H), 5.56 (s, 1H), 7.45 (m, 4H), 7.70 (m, 2H).

### PHARMACOLOGICAL ACTIVITY

### 35S-GTP-g binding assay:

The effect of the compounds was measured using a 35S-GTPyS binding assay. Briefly, membranes were incubated in a buffer containing 20 mM HEPES pH 7.4, 100 mM NaCl, 10 mM MgCl2, 10µM GDP, 50µg/ml saponin and 0.2% fatty acid-free BSA at various concentrations (0.1nM-10µM) and 0.1nM 35S-GTPyS. 10µM S1P was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffer. After drying the filter plates scintillant liquid were added to each well and 35S-GTPyS binding was measured on a Trilux Scintillation Counter.

The results are shown in Table 1.

**Table 1**

| **EXAMPLES** | **EC₅₀ (nM)** |
|---|---|
| 1 | 2.5 |
| 12 | 0.4 |
| 23 | 4.1 |
| 30 | 2.3 |
| 31 | 4.1 |
| 36 | 40 |
| 40 | 70 |
| 45 | 2.8 |
| 57 | 8.5 |
| 68 | 10.1 |

The thiadiazole derivatives of the invention may also be combined with other active compounds in the treatment of diseases known to be susceptible to improvement by treatment with a sphingosine-1-phosphate receptor agonist (S1P1).

The combinations of the invention can optionally comprise one or more additional active substances which are known to be useful in the treatment of autoimmune diseases, chronic immune and inflammatory diseases, transplant rejection, malignant neoplastic diseases, angiogenic-related disorders, pain, neurological diseases,viral and infectious diseases, such as (a) Interferons comprising Interferon beta 1a such as Avonex from Biogen Idec, CinnoVex from CinnaGen and Rebif from Merck Serono, and Interferon beta 1b such as Betaferon from Schering and Betaseron from Berlex, (b), immunomodulators such as glatiramer acetate, (c) inhibitors of DNA synthesis and repair, such as Mitoxantrone, (d) anti-alpha 4 integrin antibodies, such as Natalizumab (Tysabri), (e) alpha 4 integrin antagonists such as R-1295 , TBC-4746, CDP-323, ELND-002, Firategrast and TMC-2003, (f), dyhydrofolate reductase inhibitors, such as Methotrexate or CH-1504, (g) glucocorticoids such as prednisone or methylprednisolone, (h), DHODH inhibitors such as Teriflunomide, (i) fumaric acid esters, such as *BG-12,* (j) immunomodulators such as Laquinimod, (k) anti-CD20 monoclonal antibodies such as Rituximab, Ocrelizumab Ofatumumab or TRU-015, (I) anti-CD52 such as alemtuzumab, (m) anti-CD25 such as daclizumab, (n) anti-CD88, such as eculizumab or pexilizumab, (o) calcineurin inhibitors such as cyclosporine A or tacrolimus, (p) IMPDH inhibitors, such as mycophenolate mophetyl, (q) cannabinoid receptor agonists such as Sativex, (r) chemokine CCR1 antagonists such as MLN-3897 or PS-031291, (s) chemokine CCR2 antagonists such as INCB-8696, (t) interferon alpha such as Sumiferon MP, (u) NF-kappaB activation inhibitors such as FAE and MLN-0415, (v) JAK inhibitors such as CP-690550 or INCB018424, (W) Syk inhibitors, such as R-112, (x) PKC inhibitors, such as NVP-AEB071, (y) phosphosdiesterase IV inhibitors such as GRC-4039, (z) P38 Inhibitors such as ARRY-797, and (aa) MEK inhibitors, such as ARRY-142886 or ARRY-438162 and (ab) Adenosine aminohydrolase inhibitors such as Cladribine from Merck Serono.

The combinations of the invention may be used in the treatment of disorders which are susceptible to amelioration by sphingosine-1-phosphate receptors agonists (S1P1). Thus, the present application encompasses methods of treatment of these disorders, as well as the use of the combinations of the invention in the manufacture of a medicament for the treatment of these disorders.

Preferred examples of such disorders are multiple sclerosis, transplant rejection, systemic lupus erythematosus, asthma, psoriasis, rheumatoid arthritis, psoriatic arthritis and Crohn's disease, more preferably multiple sclerosis, transplant rejection, asthma and rheumatoid arthritis, and most preferably multiple sclerosis.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the sphingosine-1-phosphate agonist of the invention, and the other optional active compounds may be administered together in the same pharmaceutical composition or in different compositions intended for separate, simultaneous, concomitant or sequential administration by the same or a different route.

One execution of the present invention consists of a kit of parts comprising a sphingosine-1-phosphate agonist of the invention together with instructions for simultaneous, concurrent, separate or sequential use in combination with another active compound useful in the treatment of multiple sclerosis, transplant rejection, systemic lupus erythematosus, asthma, psoriasis, rheumatoid arthritis, psoriatic arthritis and Crohn's disease,

Another execution of the present invention consists of a package comprising a sphingosine-1-phosphate agonist of formula (I) and another active compound useful in the treatment of multiple sclerosis, transplant rejection, systemic lupus erythematosus, asthma, psoriasis, rheumatoid arthritis, psoriatic arthritis and Crohn's disease,

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as the Novolizer® SD2FL or Genuair® which are described in the following patent applications: WO 97/000703, WO 03/000325 and WO 03/061742.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Effective doses are normally in the range of 2-2000 mg of active ingredient per day. Daily dosage may be administered in one or more treatments, preferably from 1 to 4 treatments, per day. Preferably, the active ingredients are administered once or twice a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of N-butyl-2-fluoro-N-[5-(4-{[3-(1H-tetrazol-5-yl)azetidin-1-yl]methyl}phenyl)-1,3,4-thiadiazol-2-yl]benzamide (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of N-butyl-2-fluoro-N-[5-(4-{[3-(1H-tetrazol-5-yl)azetidin-1-yl]methyl}phenyl)-1,3,4-thiadiazol-2-yl]benzamide (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I) or a pharmaceutically acceptable salt or N-oxide or stereoisomer thereof wherein: 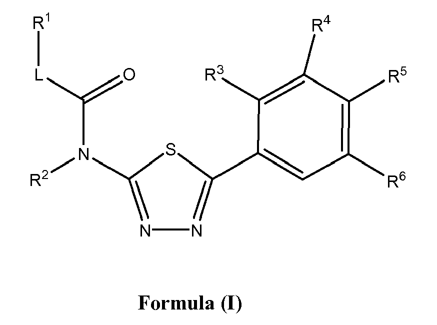 wherein:
• L represents a direct bond or a -NH- group,
• R¹ represents a phenyl, a pyridyl or a benzyl group which is optionally substituted with one or more substituents selected from halogen atoms, a C₁₋₄ alkyl group, a C₁₋₄ alkoxy group, a C₃₋₇ cycloalkyl group, and -CN, -CONH₂ and -CF₃ groups,
• R² represents a C₁₋₆ alkyl or a C₁₋₄ alkyl-C₃₋₇ cycloalkyl group,
• R³, R⁴ and R⁶ independently represent a hydrogen atom, a halogen atom, a C₁₋₄ alkyl group or a -CF₃ group,
• R⁵ represents -O-(CH₂)₍₀₋₂₎-R^{a}, -O-(CH₂)₍₂₋₄₎-NR'R", -O-(CH₂)₍₂₋₄₎-NH-(CH₂)₍₁₋₄₎-R", -(CH₂)₍₁₋₂₎-NR'R", -(CH₂)₍₁₋₄₎-NH-(CH₂)₍₁₋₄₎-R" or -(CO)-NR'R", wherein
○ R^{a} represents a 4 to 6-membered N-containing saturated heterocyclic group optionally substituted with one or more substituents selected from a hydroxycarbonyl group and a C₁₋₄ alkyl group substituted with a hydroxycarbonyl group, or a C₃₋₇ cycloalkyl group substituted with a di-(C₁₋₄ alkyl)amino group which is itself optionally substituted with a hydroxycarbonyl group,
○ R' represents a hydrogen atom or a C₁₋₄ alkyl group,
○ R" represents a methylsulfonamido group, a C₂₋₄ alkanoyl group which is optionally substituted with a hydroxy group; a C₁₋₄ alkyl group substituted with one or more substituents selected from a hydroxy group and a hydroxycarbonyl group; a 5- to 6-membered saturated or unsaturated heterocyclic group containing one or more heteroatoms selected from nitrogen and oxygen atoms and which is optionally substituted with a hydroxy group; or a C₄₋₆ cycloalkyl group substituted with a hydroxycarbonyl group, or
○ R' and R" together with the nitrogen atom to which they are attached form a 4 to 6 membered, saturated or unsaturated heterocyclic group which contains, as heteroatoms, the N atom to which R' and R" are attached and 0, 1 or 2 further N atoms, which heterocyclic group is optionally substituted with one or more substituents selected from a cyano, a carbamoyl, hydroxycarbonyl, (C₁₋₃alkoxy)carbonyl and 1*H*-tetrazolyl groups and C₁₋₃ alkyl group which is optionally substituted by a hydroxycarbonyl group.

2. A compound according to claim 1, wherein L represents a direct bond.

3. A compound according to claim 1 or 2, wherein R¹ represents a phenyl or a benzyl group which is optionally substituted with one or two substituents selected from halogen atoms, a C₁₋₄ alkyl group and a C₁₋₄ alkoxy group.

4. A compound according to claim 3 wherein R¹ is optionally substituted with one or two substituents selected from a fluorine atom, a chlorine atom, a methyl group and a methoxy group, and preferably wherein R¹ represents a phenyl group substituted with a fluorine atom, a chlorine atom or a methyl group.

5. A compound according to any one of the preceding claims, wherein R² represents an ethyl, butyl or cyclopropylmethyl group, preferably a butyl or cyclopropylmethyl group.

6. A compound according to any one of the preceding claims wherein R³, R⁴ and R⁶ independently represent a hydrogen atom, a methyl group or a -CF₃ group, and preferably wherein R³ represents a hydrogen atom, being more preferably R⁴ represents a methyl group or -CF₃ group and R⁶ represent a hydrogen atom or a methyl groups.

7. A compound according to any one of the preceding claims wherein R⁵ represents a -O-(CH₂)₍₀₋₁₎-R^{a}, -O-(CH₂)₍₂₋₄₎-NR'R", -O-(CH₂)₍₂₋₄₎-NH-(CH₂)₍₁₋₄₎-R", -(CH₂)₍₁₋₂₎-NR'R" or -(CH₂)₍₁₋₄₎-NH-(CH₂)₍₁₋₄₎-R", wherein
○ R^{a} represents a 5 to 6-membered N-containing saturated heterocyclic group optionally substituted with a hydroxycarbonyl group
○ R' represents a hydrogen atom or a methyl group,
o R" represents a methylsulfonamido group, a C₁₋₃ alkyl group substituted with a hydroxycarbonyl group; an unsubstituted 5- to 6 membered unsaturated heterocyclic group containing one or more nitrogen atoms as heteroatoms or a C₄₋₆ cycloalkyl group substituted with a hydroxycarbonyl group, or
○ R' and R" together with the nitrogen atom to which they are attached form a 4 to 6 membered, saturated heterocyclic group substituted with one or two substituents selected from cyano, carbamoyl, hydroxycarbonyl, C₁₋₂ alkoxycarbonyl and 1*H*-tetrazolyl groups and a C₁₋₃ alkyl group which is optionally substituted by a hydroxycarbonyl group.

8. A compound according to claim 7 wherein R⁵ represents -O-(CH₂)₍₂₋₄₎-NR'R", -O-(CH₂)₍₂₋₄₎-NH-(CH₂)₍₁₋₄₎-R", -(CH₂)₍₁₋₂₎-NR'R" or -(CH₂)₍₁₋₄₎-NH-(CH₂)₍₁₋₄₎-R", wherein
○ R" represents a C₁₋₃ alkyl group substituted with a hydroxycarbonyl group or a C₅₋₆ cycloalkyl group substituted with a hydroxycarbonyl group, or
○ R' and R" together with the nitrogen atom to which they are attached form a 4 to 6 membered, saturated heterocyclic group substituted with one or two substituents selected from carbamoyl and hydroxycarbonyl groups and a methyl group which is optionally substituted by a hydroxycarbonyl group.

9. A compound according to claim 8 wherein R⁵ represents a -O-(CH₂)₍₂₋₃₎-NR'R", wherein R' and R" together with the nitrogen atom to which they are attached form a 4 to 6 membered, saturated heterocyclic group substituted with one or two substituents selected from hydroxycarbonyl group and a methyl group which is optionally substituted by a hydroxycarbonyl group.

10. A compound according to any one of the preceding claims wherein, L represents a direct bond, R¹ represents a phenyl group substituted with a fluorine atom, a chlorine atom or a methyl group, R² represents a butyl or cyclopropylmethyl group, R³ represents a hydrogen atom, R⁴ represents a methyl group or -CF₃ group, R⁶ represents a hydrogen atom or a methyl group and R⁵ represents -O-(CH₂)₍₂₋₃₎-NR'R", wherein R' and R" together with the nitrogen atom to which they are attached form a 4 to 6 membered, saturated heterocyclic group substituted with one or two substituents selected from a hydroxycarbonyl group and a methyl group which is optionally substituted by a hydroxycarbonyl group.

11. A compound according to claim 1 wherein:
• L represents a direct bond or-NH-;
• R¹ represents a phenyl group substituted with one or two substituents independently selected from a fluorine atom, a chlorine atom, a methyl group or a methoxy group, a benzyl group, or pyridyl group substituted with a chlorine atom;
• R² represents ethyl, butyl or cyclopropylmethyl group;
• R³, R⁴ and R⁶ independently represent hydrogen atoms or methyl groups;
• R⁵ represents -O-(CH₂)₍₀₋₁₎-R^{a}, -O-(CH₂)₂-NR'R", -O-(CH₂)₂-NH-(CH₂)₂-R", - (CH₂)₂-NR'R", -(CH₂)₍₁₋₂₎-NH-(CH₂)₂-R" or -C(O)-NR'R", wherein:
○ R^{a} piperidyl or pyrolidyl which is unsubstituted or substituted with a hydroxycarbonyl group;
○ R' represents a hydrogen atom or methyl;
○ R" represents a methylsulfonamido group, an ethanoyl or hydroxyethanoyl group; a methyl, ethyl or propyl group which is substituted with one or two substitutents independently selected from a hydroxy group and a hydroxycarbonyl group; a tetrazolyl or triazolyl group; or a cyclopentyl or cyclohexyl group substituted with a hydroxycarbonyl group; or
○ R' and R" together with the nitrogen atom to which they are attached form an azetidyl, pyrrolidyl, pyrazolyl or piperidyl group which is substituted with one or two substituents selected from a cyano, a carbamoyl, a hydroxycarbonyl, a ethoxycarbonyl and 1*H*-tetrazolyl group and a methyl group which is optionally substituted with one hydroxycarbonyl group

12. A compound according to claim 1 which is one of:
1-[2-(4-{5-[butyl(2-fluorobenzoyl)amino]-1,3,4-thiadiazol-2-yl}-2,6-dimethylphenoxy)ethyl]azetidine-3-carboxylic acid,
N-[2-(4-{5-[butyl(2-fluorobenzoyl)amino]-1,3,4-thiadiazol-2-yl}-2,6-dimethylphenoxy)ethyl]-beta-alanine,
N-[2-(4-{5-[butyl(2-fluorobenzoyl)amino]-1,3,4-thiadiazol-2-yl}-2,6-dimethylphenoxy)ethyl]glycine,
N-[2-(4-f5-[butyl(2-fluorobenzoyl)amino]-1,3,4-thiadiazol-2-yll-2,6-dimethylphenoxy)ethyl]-N-methylglycine,
1-[2-(4-{5-[butyl(2-fluorobenzoyl)amino]-1,3,4-thiadiazol-2-yl}-2,6-dimethylphenoxy)ethyl]piperidine-4-carboxylic acid,
1-[2-(4-{5-[butyl(2-fluorobenzoyl)amino]-1,3,4-thiadiazol-2-yl}-2,6-dimethylphenoxy)ethyl]piperidine-2-carboxylic acid,
(4S)-4-(4-{5-[butyl(2-fluorobenzoyl)amino]-1,3,4-thiadiazol-2-yl}-2,6-dimethylphenoxy)-L-proline,
1-[2-(4-{5-[butyl(2-fluorobenzoyl)amino]-1,3,4-thiadiazol-2-yl}-2,6-dimethylphenoxy)ethyl]piperidine-3-carboxylic acid,
1-[2-(4-{5-[butyl(2-fluorobenzoyl)amino]-1,3,4-thiadiazol-2-yl}-2,6-dimethylphenoxy)ethyl]praline,
N-butyl-2-fluoro-N-(5-{4-[2-(glycoloylamino)ethoxy]-3,5-dimethylphenyl}-1,3,4-thiadiazol-2-yl)benzamide,
N-[2-(4-{5-[butyl(2-methylbenzoyl)amino]-1,3,4-thiadiazol-2-yl}-2,6-dimethylphenoxy)ethyl]-N-methylglycine,
N-[2-(4-{5-[butyl(2-chlorobenzoyl)amino]-1,3,4-thiadiazol-2-yl}-2,6-dimethylphenoxy)ethyl]-N-methylglycine,
1-[2-(4-{5-[butyl(2-methylbenzoyl)amino]-1,3,4-thiadiazol-2-yl}-2,6-dimethylphenoxy)ethyl]praline,
1-[2-(4-{5-[butyl(2-chlorobenzoyl)amino]-1,3,4-thiadiazol-2-yl}-2,6-dimethylphenoxy)ethyl]praline,
1-[2-(4-{5-[butyl(2-fluorobenzoyl)amino]-1,3,4-thiadiazol-2-yl}-2,6-dimethylphenoxy)ethyl]pyrrolidine-3-carboxylic acid,
1-[2-(4-{5-[butyl(2-chlorobenzoyl)amino]-1,3,4-thiadiazol-2-yl}-2,6-dimethylphenoxy)ethyl]piperidine-4-carboxylic acid,
1-[2-(4-{5-[butyl(2-methylbenzoyl)amino]-1,3,4-thiadiazol-2-yl}-2,6-dimethylphenoxy)ethyl]piperidine-4-carboxylic acid,
1-[2-(4-{5-[butyl(3-chloro-2-fluorobenzoyl)amino]-1,3,4-thiadiazol-2-yl}-2,6-dimethylphenoxy)ethyl]piperidine-4-carboxylic acid,
1-[2-(4-{5-[ethyl(2-fluorobenzoyl)amino]-1,3,4-thiadiazol-2-yl}-2,6-dimethylphenoxy)ethyl]piperidine-4-carboxylic acid,
(1S,2R)-2-{[2-(4-{5-[butyl(2-fluorobenzoyl)amino]-1,3,4-thiadiazol-2-yl}-2,6-dimethylphenoxy)ethyl]amino}cyclopentanecarboxylic acid,
1-[2-(4-{5-[(2-chlorobenzoyl)(ethyl)amino]-1,3,4-thiadiazol-2-yl}-2,6-dimethylphenoxy)ethyl]piperidine-4-carboxylic acid,
{1-[2-(4-{5-[butyl(2-fluorobenzoyl)amino]-1,3,4-thiadiazol-2-yl}-2,6-dimethylphenoxy)ethyl]piperidin-4-yl}acetic acid,
N-butyl-N-{5-[3,5-dimethyl-4-(2-{[2-(2H-tetrazol-5-yl)ethyl]amino}ethoxy)phenyl]-1,3,4-thiadiazol-2-yl}-2-fluorobenzamide,
1-[2-(4-{5-[(3-chloro-2-fluorobenzoyl)(ethyl)amino]-1,3,4-thiadiazol-2-yl}-2,6-dimethylphenoxy)ethyl]piperidine-4-carboxylic acid,
N-[2-(4-{5-[ethyl(2-fluorobenzoyl)amino]-1,3,4-thiadiazol-2-yl}-2,6-dimethylphenoxy)ethyl]-N-methylglycine,
1-[2-(4-{5-[ethyl(2-fluorobenzoyl)amino]-1,3,4-thiadiazol-2-yl}-2,6-dimethylphenoxy)ethyl]piperidine-3-carboxylic acid,
1-[2-(4-{5-[butyl(2-fluorobenzoyl)amino]-1,3,4-thiadiazol-2-yl}-2,6-dimethylphenoxy)ethyl]-4-methylpiperidine-4-carboxylic acid,
cis-4-{[2-(4-{5-[butyl(2-fluorobenzoyl)amino]-1,3,4-thiadiazol-2-yl}-2,6-dimethylphenoxy)ethyl]amino}cyclohexanecarboxylic acid,
1-[2-(4-{5-[butyl(2,4-difluorobenzoyl)amino]-1,3,4-thiadiazol-2-yl}-2,6-dimethylphenoxy)ethyl]piperidine-4-carboxylic acid,
1-[2-(4-{5-[butyl(2-fluorobenzoyl)amino]-1,3,4-thiadiazol-2-yl}phenoxy)ethyl]-azetidine-3-carboxylic acid,
1-[2-(4-{5-[butyl(2-fluorobenzoyl)amino]-1,3,4-thiadiazol-2-yl}phenoxy)ethyl]-piperidine-4-carboxylic acid,
1-[2-(4-{5-[ethyl(2-fluorobenzoyl)amino]-1,3,4-thiadiazol-2-yl}-2,6-dimethylphenoxy)ethyl]azetidine-3-carboxylic acid,
1-[2-(4-{5-[butyl(2-methoxybenzoyl)amino]-1,3,4-thiadiazol-2-yl}-2,6-dimethylphenoxy)ethyl]piperidine-4-carboxylic acid,
1-[2-(4-{5-[butyl(phenylacetyl)amino]-1,3,4-thiadiazol-2-yl}-2,6-dimethylphenoxy)ethyl]piperidine-4-carboxylic acid,
1-[2-(4-{5-[butyl(2,3-dimethylbenzoyl)amino]-1,3,4-thiadiazol-2-yl}-2,6-dimethylphenoxy)ethyl]piperidine-4-carboxylic acid,
1-[2-(4-{5-[butyl(2-chloro-3-fluorobenzoyl)amino]-1,3,4-thiadiazol-2-yl}-2,6-dimethylphenoxy)ethyl]piperidine-4-carboxylic acid,
1-[2-(4-{5-[(2-chlorobenzoyl)(cyclopropylmethyl)amino]-1,3,4-thiadiazol-2-yl}-2,6-dimethylphenoxy)ethyl]piperidine-4-carboxylic acid,
N-[2-(4-{5-[(2-chlorobenzoyl)(cyclopropylmethyl)amino]-1,3,4-thiadiazol-2-yl}-2,6-dimethylphenoxy)ethyl]-N-methylglycine,
{1-[2-(4-{5-[(2-chlorobenzoyl)(cyclopropylmethyl)amino]-1,3,4-thiadiazol-2-yl}-2,6-dimethylphenoxy)ethyl]piperidin-4-yl}acetic acid,
N-butyl-2-chloro-N-{5-[3,5-dimethyl-4-(piperidin-4-ylmethoxy)phenyl]-1,3,4-thiadiazol-2-yl}benzamide,
1-[2-(4-{5-[butyl(2-chlorobenzoyl)amino]-1,3,4-thiadiazol-2-yl}-2,6-dimethylphenoxy)ethyl]pyrrolidine-3-carboxylic acid,
{1-[2-(4-{5-[butyl(2-chlorobenzoyl)amino]-1,3,4-thiadiazol-2-yl}-2,6-dimethylphenoxy)ethyl]piperidin-4-yl}acetic acid,
1-[2-(4-{5-[butyl(2-chlorobenzoyl)amino]-1,3,4-thiadiazol-2-yl}-2,6-dimethylphenoxy)ethyl]-4-methylpiperidine-4-carboxylic acid,
(1S,2R)-2-{[2-(4-{5-[butyl(2-chlorobenzoyl)amino]-1,3,4-thiadiazol-2-yl}-2,6-dimethylphenoxy)ethyl]amino}cyclopentanecarboxylic acid,
cis-4-{[2-(4-{5-[butyl(2-chlorobenzoyl)amino]-1,3,4-thiadiazol-2-yl}-2,6-dimethylphenoxy)ethyl]amino}cyclohexanecarboxylic acid,
1-[2-(4-{5-[(2-chlorobenzoyl)(cyclopropylmethyl)amino]-1,3,4-thiadiazol-2-yl}-2,6-dimethylphenoxy)ethyl]azetidine-3-carboxylic acid,
{1-[2-(4-{5-[butyl(2-chlorobenzoyl)amino]-1,3,4-thiadiazol-2-yl}-2,6-dimethylphenoxy)ethyl]pyrrolidin-3-yl}acetic acid,
N-[2-(4-{5-[butyl(2-chlorobenzoyl)amino]-1,3,4-thiadiazol-2-yl}-2,6-dimethylphenoxy)ethyl]-beta-alanine,
{1-[2-(4-{5-[butyl(2-chlorobenzoyl)amino]-1,3,4-thiadiazol-2-yl}-2,6-dimethylphenoxy)ethyl]piperidin-3-yl}acetic acid,
1-[2-(4-{5-[butyl(2-chlorobenzoyl)amino]-1,3,4-thiadiazol-2-yl}-2,6-dimethylphenoxy)ethyl]piperidine-2-carboxylic acid,
N-[2-(4-{5-[butyl(2-chlorobenzoyl)amino]-1,3,4-thiadiazol-2-yl}-2,6-dimethylphenoxy)ethyl]glycine,
1-[2-(4-{5-[butyl(2-chlorobenzoyl)amino]-1,3,4-thiadiazol-2-yl}-2,6-dimethylphenoxy)ethyl]piperidine-3-carboxylic acid,
1-{4-[5-(butyl{[(4-fluorophenyl)amino]carbonyl}amino)-1,3,4-thiadiazol-2-yl]benzyl}azetidine-3-carboxylic acid,
1-(4-{5-[(anilinocarbonyl)(butyl)amino]-1,3,4-thiadiazol-2-yl}benzyl)azetidine-3-carboxylic acid,
1-{4-[5-(butyl{[(2-chloropyridin-3-yl)amino]carbonyl}amino)-1,3,4-thiadiazol-2-yl]benzyl}azetidine-3-carboxylic acid,
1-(4-{5-[butyl(2-fluorobenzoyl)amino]-1,3,4-thiadiazol-2-yl}benzoyl)azetidine-3-carboxylic acid,
N-butyl-2-fluoro-N-[5-(4-{[(2-hydroxyethyl)amino]methyl}phenyl)-1,3,4-thiadiazol-2-yl]benzamide,
3-[(4-{5-[butyl(2-fluorobenzoyl)amino]-1,3,4-thiadiazol-2-yl}benzoyl)amino]-2-hydroxypropanoic acid,
1-(4-{5-[butyl(2-fluorobenzoyl)amino]-1,3,4-thiadiazol-2-yl}benzyl)azetidine-3-carboxamide,
3-[(4-{5-[butyl(phenylacetyl)amino]-1,3,4-thiadiazol-2-yl}benzyl)amino]-2-methylpropanoic acid,
(1S,2S)-2-[(4-{5-[butyl(phenylacetyl)amino]-1,3,4-thiadiazol-2-yl}benzyl)-amino]cyclohexanecarboxylic acid,
1-[4-(5-{butyl[(2-methoxyphenyl)acetyl]amino}-1,3,4-thiadiazol-2-yl)benzyl]-azetidine-3-carboxylic acid,
N-butyl-2-fluoro-N-(5-{4-[({2-[(methylsulfonyl)amino]ethyl}amino)methyl]phenyl}-1,3,4-thiadiazol-2-yl)benzamide,
N-(4-{5-[butyl(2-fluorobenzoyl)amino]-1,3,4-thiadiazol-2-yl}benzyl)-N-methylglycine, N-butyl-N-(5-{4-[(3-cyanoazetidin-1-yl)methyl]phenyl}-1,3,4-thiadiazol-2-yl)-2-fluorobenzamide,
1-[4-(5-{butyl[(2-methoxyphenyl)acetyl]amino}-1,3,4-thiadiazol-2-yl)-3-methylbenzyl]azetidine-3-carboxylic acid,
N-butyl-2-fluoro-N-[5-(4-{2-[(2-hydroxyethyl)(methyl)amino]ethoxyl-3,5-dimethylphenyl)-1,3,4-thiadiazol-2-yl]benzamide,
N-(5-{4-[2-(acetylamino)ethoxy]-3,5-dimethylphenyl}-1,3,4-thiadiazol-2-yl)-N-butyl-2-fluorobenzamide,
1-(4-{5-[butyl(2-fluorobenzoyl)amino]-1,3,4-thiadiazol-2-yl}benzyl)-1H-pyrazole-4-carboxylic acid,
1-(4-{5-[butyl(2-fluorobenzoyl)amino]-1,3,4-thiadiazol-2-yl}benzyl)-4-(ethoxycarbonyl)piperidine-4-carboxylic acid,
N-(4-{5-[butyl(2-fluorobenzoyl)amino]-1,3,4-thiadiazol-2-yl}benzyl)-N-methyl-beta-alanine,
N-butyl-2-fluoro-N-[5-(4-{[3-(1H-tetrazol-5-yl)azetidin-1-yl]methyl}phenyl)-1,3,4-thiadiazol-2-yl]benzamide
N-butyl-N-{5-[3,5-dimethyl-4-(2-{[2-(1H-1,2,4-triazol-1-yl)ethyl]amino}ethoxy)phenyl]-1,3,4-thiadiazol-2-yl}-2-fluorobenzamide,
N-butyl-2-fluoro-N-{5-[4-({[2-(1H-1,2,4-triazol-1-yl)ethyl]aminolmethyl)phenyl]-1,3,4-thiadiazol-2-yl}benzamide,
N-(4-{5-[butyl(2-chlorobenzoyl)amino]-1,3,4-thiadiazol-2-yl}-2,6-dimethylbenzyl)-N-methylglycine, and
3-[(4-{5-[butyl(phenylacetyl)amino]-1,3,4-thiadiazol-2-yl}benzyl)amino]-3-methylpropanoic acid

13. A compound according to any one of claims 1 to 12 for use in the treatment of a pathological condition or disease susceptible to amelioration by sphingosine-1-phosphate receptors (S1P1) agonists.

14. A compound according to claim 13, wherein the pathological condition or disease is selected from autoimmune diseases, chronic immune and inflammatory diseases, transplant rejection, malignant neoplastic diseases, angiogenic-related disorders, pain, neurological diseases, viral and infectious diseases.

15. A compound according to claim 13 wherein the pathological condition or disease is selected from multiple sclerosis, transplant rejection, systemic lupus erythematosus, asthma, psoriasis, rheumatoid arthritis, psoriatic arthritis and Crohn's disease,

16. A pharmaceutical composition comprising a compound as defined in any one of claims 1 to 12 in association with a pharmaceutically acceptable diluent or carrier.

17. Use of a compound as defined in any one of claims 1 to 12 in the manufacture of a medicament for the treatment of a pathological condition or disease as defined in claims 13 to 15.

18. A method for treating a subject afflicted with a pathological condition or disease as defined in claims 13 to 15, which comprises administering to said subject a therapeutically effective amount of a compound as defined in any one of claims 1 to 12.

19. A combination product comprising (i) a compound as defined in any one of claims 1 to 12; and (ii) another compound selected from:
a) Interferons comprising Interferon beta 1a such as Avonex from Biogen Idec, CinnoVex from CinnaGen and Rebif from Merck Serono, and Interferon beta 1b such as Betaferon from Schering and Betaseron from Berlex
b) Immunomodulators such as glatiramer acetate
c) Inhibitors of DNA synthesis and repair, such as Mitoxantrone
d) Anti-alpha 4 integrin antibodies, such as Natalizumab (Tysabri)
e) Alpha 4 integrin antagonists such as R-1295 , TBC-4746, CDP-323, ELND-002, Firategrast and TMC-2003
f) Dyhydrofolate reductase inhibitors, such as Methotrexate or CH-1504
g) Glucocorticoids such as prednisone or methylprednisolone
h) DHODH inhibitors such as teriflunomide
*i)*Fumaric acid esters, such as *BG-12*
*j)*Immunomodulators such as Laquinimod
k) Anti-CD20 monoclonal antibodies such as Rituximab, Ocrelizumab Ofatumumab or TRU-015
l) Anti-CD52 such as alemtuzumab
m) Anti-CD25 such as daclizumab
n) Anti-CD88, such as eculizumab or pexilizumab
o) Calcineurin inhibitors such as cyclosporine A or tacrolimus
p) IMPDH inhibitors, such as mycophenolate mophetyl
q) Cannabinoid receptor agonists such as Sativex
r) Chemokine CCR1 antagonists such as MLN-3897 or PS-031291
s) Chemokine CCR2 antagonists such as INCB-8696
t) Interferon alpha such as Sumiferon MP
u) NF-kappaB activation inhibitors such as FAE and MLN-0415
v) JAK inhibitors such as CP-690550 or INCB018424
w) Syk inhibitors, such as R-112
x) PKC inhibitors, such as NVP-AEB071
y) Phosphosdiesterase IV inhibitors such as GRC-4039
z) P38 Inhibitors such as ARRY-797
aa) MEK inhibitors, such as ARRY-142886 or ARRY-438162
bb) Adenosine aminohydrolase inhibitors such as Cladribine from Merck Serono
